# EUROPEAN PATENT APPLICATION

(11) **EP 1 998 177 A2**
(43) Date of publication of application: **03.12.2008**
(21) Application number: 08011895.3
(22) Date of filing: 06.01.2004
(51) Int. Cl.: G01N 33/574, G01N 33/50

(54) **Compositions and methods for diagnosing and treating colon cancers**

(30) Priority: 06.01.2003 US 438000 P
(62) Divisional of application: 04700319.9
(71) Applicant: Wyeth, Madison, NJ 07940 (US)
(72) Inventor: Martinez, Robert Vincent, Carlisle, MA 01741 (US); Brown, Eugene L., Newton Highlands, MA 02461 (US); Liu, Wei, Sudbury, MA 01776 (US)
(74) Representative: Denholm, Anna Marie

(57) **Abstract**

Compositions, equipment, and methods for prognosing, diagnosing, preventing, or treating colon cancer. Colon cancer genes that are differentially expressed in colon cancer tissues relative to disease-free tissues are identified. Examples of these genes are illustrated in Tables 1-5. Colon cancer genes of the present invention and their encoded products can be used as markers or prophylactic or therapeutic agents for the detection or treatment of colon cancer.

## Description

This application incorporates by reference all materials recorded in compact discs "Copy 1," "Copy 2," and "Copy 3." Each of the compact discs includes the sequence listing file entitled "AM100927 Sequence Listing.ST25.txt" (8,300 KB, created on January 6, 2004).

### TECHNICAL FIELD

The present invention relates generally to the diagnosis and treatment of cancer, and in particular colon cancer. The invention specifically relates to colon cancer genes that are differentially expressed in colon cancer tissues as compared to disease-free tissues. These genes can be used for prognosing, diagnosing, preventing, or treating colon cancer.

### BACKGROUND OF THE INVENTION

Cancer is a significant health problem throughout the world. Although advances have been made in detecting and treating cancer, no vaccine or other universally successful method for prevention or treatment is currently available. Current therapies, which are generally based on a combination of chemotherapy or surgery and radiation, continue to prove inadequate in many patients.

Colon cancer is the second most frequently diagnosed malignancy in the United States, as well as the second most common cause of cancer death. An estimated 135,400 new cases of colon cancer were diagnosed in 2001, with an estimated 56,700 deaths. The five-year survival rate for patients with colon cancer detected in an early localized stage is 92%; unfortunately, only 37% of colon cancer is diagnosed at this stage. The survival rate drops to 64% if the cancer is allowed to spread to adjacent organs or lymph nodes, and to 7% in patients with distant metastases.

The prognosis of colon cancer is directly related to the degree of penetration of the tumor through the bowel wall and the presence or absence of nodal involvement; consequently, early detection and treatment are especially important. Colon cancer typically originates in the colonic epithelium and is not extensively vascularized (and therefore not invasive) during the early stages of development. The transition to a highly-vascularized, invasive and ultimately metastatic cancer commonly takes ten years or longer. With early detection and diagnosis, colon cancer may be effectively treated by, for example, surgical removal of the cancerous or precancerous tissue. However, colon cancer is often detected only upon manifestation of clinical symptoms, such as pain and black tarry stool. Generally, such symptoms are present only when the disease is well established, and only after metastasis has occurred. Early detection of colon cancer is therefore important in order to significantly reduce its morbidity. Currently, the best means of preventing colon cancer is through early detection of pre-neoplastic lesions in the colon through various invasive and noninvasive screening techniques.

Most methods for colon cancer screening are invasive. Invasive diagnostic screening methods, such as endoscopic examination, allow for direct visual identification, removal, and biopsy of potentially-cancerous tissue. However, invasive cancer screening procedures are often expensive, inherently risky, and can result in severe medical complications. Invasive screening procedures also frequently result in significant patient discomfort. The discomfort associated with typical invasive screening methods reduces patient compliance with routine screening procedures. For example, flexible sigmoidoscopy is an invasive procedure for diagnosing colon cancer that enables detection of approximately 55% of all colon cancer and is estimated to have an 85% sensitivity with a near 100% specificity. However, the procedure has a complication rate of about 4.5 per 10,000 persons screened. Further, patient compliance with physicians' recommendations to undergo sigmoidoscopy is low, reportedly varying from 30 to 75%, due to discomfort and perceived embarrassment associated with this procedure.

Non-invasive methods of colon cancer screening involve assaying samples for the presence of materials that are indicative of cancer or pre-cancer. Established non-invasive methods for detection of colon cancer focus on extracellular indicia of the presence of cancer, such as the presence of fecal occult blood or elevated levels of carcinoembryonic antigen, both of which are suggestive of the presence of colon cancer. However, such extracellular indicia typically occur only after the cancer has become invasive, and therefore more difficult to treat. As a result, many non-invasive screening procedures are of limited value in the early diagnosis of cancer. For example, fecal occult blood testing (FOBT) is a non-invasive screening test for colon cancer that is highly variable in accuracy, ranging between 28% and 93%, depending upon the subject's hydration status, with a specificity of 96%. One study estimates, however, that 50 to 60% of all colorectal cancers will be missed if FOBT is the only method of screening used (Allison et al., Ann. Intern. Med., 112:328-333, 1990).

Recent developments in molecular biology provide methods of great potential for detecting the presence of a range of DNA mutations indicative of oncogenesis. Mutations and the loss of heterozygosity at the p53 tumor suppressor locus have been correlated with various types of cancer. The loss or other mutation of the APC and DCC tumor suppressor genes has also been associated with tumor development. It has been suggested that specific mutations might be a basis for molecular screening assays for the early stages of certain types of cancer. Accordingly, non-invasive screening assays that are highly sensitive and highly specific for detecting the presence of a range of DNA mutations indicative of cancer have been developed. For instance, the presence of such mutations can be detected in DNA found in stool samples during various stages of colon cancer.

Treatment regimens are determined by the type and stage of the cancer, and include surgery, radiation therapy or chemotherapy. Recurrence following surgery (the most common form of therapy) is a major problem and is often the ultimate cause of death. Current methods for prognosing, detecting and treating colon cancer have failed to provide satisfactory results for reducing the morbidity associated with the disease.

### SUMMARY OF THE INVENTION

The present invention relates to colon cancer genes that are differentially expressed in colon cancer tissues relative to disease-free colon tissues. The present invention provides compositions, equipment, and methods of using these genes for the prognosis, diagnosis, prevention, or treatment of colon cancer.

In one embodiment, the colon cancer genes are differentially expressed not only between colon cancer tissues and disease-free colon tissues, but also between colon cancer tissues and one or more other disease-free tissues. These other disease-free tissues include, but are limited to, cervix, kidney, left atrium, left ventricle, right atrium, right ventricle, lung, ovary, prostate, rectum, skin, or stomach. Differential expression can be either over-expression or under-expression.

In another embodiment, the colon cancer genes are over-expressed in colon cancer tissues relative to disease-free colon tissues. The average expression levels of these genes in colon cancer tissues can be, for example, at least 1.5, 2, 3, 4, 5, 10, 20, or more times of those in disease-free colon tissues. In many cases, the p-value of the differential expression analysis for each selected colon cancer gene is no more than 0.1, 0.05, 0.001, 0.0005, 0.0001, or less.

In yet another embodiment, the colon cancer genes are selected from Tables 1-5. In still another embodiment, the colon cancer genes encode kinases, phosphatases, G-protein coupled receptors, ion channels, proteases, metabolic enzymes, or transcription factors.

In one aspect, the present invention provides methods useful for diagnosing or monitoring colon cancer in a subject of interest. The methods include the steps of detecting the levels of one or more polypeptides encoded by at least one colon cancer gene in a biological sample of the subject, and comparing the detected levels to control levels. The biological sample can be, for example, a blood sample, a colon tissue sample, or a bodily waste sample. In one embodiment, the control levels are average levels of the one or more polypeptides in control samples of disease-free subjects. In another embodiment, the biological sample and the control samples are prepared using the same procedure. In still another embodiment, the one or more polypeptides are selected from SEQ ID NOS:64-126, or fragments thereof. In yet another embodiment, the levels of the one or more polypeptides are determined by using antibodies specific for the polypeptides. The subject of interest may or may not have colon cancer. In one embodiment, the subject has colon cancer and is subject to a therapeutic treatment of the cancer. In another embodiment, the subject is a human, a canine, or another mammal.

In another aspect, the present invention further provides methods useful for diagnosing or monitoring colon cancer in a subject of interest. The methods include the steps of detecting the expression profile of one or more colon cancer genes in a biological sample of the subject, and comparing the expression profile to a control expression profile The expression profile and the control expression profile can be determining by measuring the levels of the polypeptides or polynucleotides encoded by the one or more colon cancer genes. In one embodiment, the control expression profile is an average expression profile of the one or more colon cancer genes in controls samples of disease-free subjects.

In yet another aspect, the present invention provides additional methods useful for diagnosing or monitoring colon cancer in a subject of interest. The methods include the steps of detecting in a biological sample of the subject the level of T cells that are activated by one or more polypeptides encoded by at least one colon cancer gene, and comparing the detected level to a control level of activated T cells.

The present invention also features pharmaceutical compositions useful for treating or preventing colon cancer. In one embodiment, the pharmaceutical compositions of the present invention include a pharmaceutically acceptable carrier and at least one polypeptide or polynucleotide encoded by a colon cancer gene that is over-expressed in colon cancer tissues relative to disease-free colon tissues. The pharmaceutical compositions can also include a variant or an allele of the encoded polypeptide or polynucleotide. In one example, the pharmaceutical compositions are vaccine formulations capable of eliciting an immune response against a colon cancer cell or a component thereof The present invention also provides methods for administering an immunoeffective amount of a vaccine formulation into a subject in need thereof.

In another embodiment, the pharmaceutical compositions of the present invention include a pharmaceutically acceptable carrier and at least one active component selected from (i) agents capable of modulating the expression of a colon cancer gene which is over-expressed in colon cancer tissues relative to disease-free colon tissues, (ii) agents capable of binding to, or modulating the biological activity of, the polypeptide(s) encoded by the colon cancer gene, or (iii) T cells activated by the polypeptide(s) encoded by the colon cancer gene. Exemplary modulations include, but are not limited to, up-regulation, induction, stimulation, potentiation, inhibition, relief of inhibition, down-regulation, and suppression.

In one example, the active component is a polynucleotide comprising or encoding an RNA that is capable of inhibiting or decreasing expression of the colon cancer gene by RNA interference or an antisense mechanism. In another example, the active component is an antibody specific for the polypeptide(s) encoded by the colon cancer gene. In yet another example, the active component is an inhibitor of a protein encoded by the colon cancer gene. Proteins encoded by colon cancer genes can be, for example, kinases, phosphatases, G-protein coupled receptors, proteases, metabolic enzymes, ion channels, or transcription factors. These proteins and genes are potential targets for drug action and development.

In still yet another aspect, the present invention provides methods for screening anti-tumor agents based on their effects on the expression or function of colon cancer genes.

In another aspect, the present invention provides nucleic acid arrays useful for diagnosing colon cancer or screening for agents that can inhibit colon cancer. The nucleic acid arrays include one or more substrate supports which are stably associated with polynucleotide probes. A substantial portion of all polynucleotide probes that are stably associated with the substrate support(s) are capable of hybridizing under reduced stringent, stringent, or highly stringent conditions to RNA transcripts of colon cancer genes, or the complements thereof. In some cases, at least 10%, 20%, 30%, 40%, 50%, or more of all polynucleotide probes that are stably associated with the substrate support(s) can hybridize under reduced stringent, stringent, or highly stringent conditions to colon cancer genes. In one example, the nucleic acid array includes mismatch probes (e.g., perfect mismatch probes) for each perfect match probe. In another example, the nucleic acid array is a bead array.

In yet another aspect, the present invention provides polypeptide arrays useful for diagnosing colon cancer or screening for agents that can inhibit colon cancer. The polypeptide arrays include one or more substrate supports which are stably associated with numerous polypeptides. A substantial portion of all polypeptides that are stably associated with the substrate support(s) are polypeptides encoded by colon cancer genes, variants of the encoded polypeptides, antibodies specific for the encoded polypeptides or variants thereof, polypeptides comprising the encoded polypeptides or variants thereof, or any combination thereof. In some cases, the substantial portion of all polypeptides includes at least 10%, 20%, 30%, 40%, 50%, or more of all polypeptides that are stably associated with the substrate support(s).

In a further aspect, the present invention provides kits useful for diagnosing colon cancer. Each kit can include at least one of the following: (a) a polynucleotide probe capable of hybridizing under reduced stringent, stringent, or highly stringent conditions to a colon cancer gene (e.g., a sequence selected from SEQ ID NOS:1-63, or a complement thereof), or (ii) an antibody capable of specifically binding to a polypeptide encoded by a colon cancer gene (e.g., a sequence selected from SEQ ID NOS:64-126).

In yet another aspect, the present invention provides methods for inhibiting colon cancer in a subject. The methods comprise the steps of (a) stimulating and expanding T cells isolated from the subject with at least one of the following: (1) a polypeptide encoded by a colon cancer gene or a variant of the encoded polypeptide, (2) a polynucleotide encoded by a colon cancer gene or a variant of the encoded polynucleotide, and (3) an antigen presenting cell that expresses a polypeptide encoded by a colon cancer gene or its variant; and (b) administering to the subject an effective amount of the stimulated and expanded T cells. In one example, the stimulated and expanded cells are cloned prior to administration to the subject.

It is further intended that the inventions not be limited only to the specific structure, material or acts that are described in the preferred embodiments, but in addition, include any and all structures, materials or acts that are capable of performing the claimed function, along with any and all known or later-developed equivalent structures, materials or acts capable of performing the claimed function.

### BRIEF DESCRIPTION OF THE DRAWINGS

The inventions of this application are better understood in conjunction with the following drawings. The drawings are provided for illustration, not limitation.

**FIG. 1** depicts the hydrophobicity profile of the polypeptide consisting of an amino acid sequence recited in SEQ ID NO:64.

**FIG. 2** depicts the hydrophobicity profile of the polypeptide consisting of an amino acid sequence recited in SEQ ID NO:65.

**FIG. 3** depicts the hydrophobicity profile of the polypeptide consisting of an amino acid sequence recited in SEQ ID NO:66.

**FIG. 4** depicts the hydrophobicity profile of the polypeptide consisting of an amino acid sequence recited in SEQ ID NO:67.

**FIG. 5** depicts the hydrophobicity profile of the polypeptide consisting of an amino acid sequence recited in SEQ ID NO:68.

**FIG. 6** depicts the hydrophobicity profile of the polypeptide consisting of an amino acid sequence recited in SEQ ID NO:69.

**FIG. 7** depicts the hydrophobicity profile of the polypeptide consisting of an amino acid sequence recited in SEQ ID NO:70.

**FIG. 8** depicts the hydrophobicity profile of the polypeptide consisting of an amino acid sequence recited in SEQ ID NO:71.

**FIG. 9** depicts the hydrophobicity profile of the polypeptide consisting of an amino acid sequence recited in SEQ ID NO:72.

**FIG. 10** depicts the hydrophobicity profile of the polypeptide consisting of an amino acid sequence recited in SEQ ID NO:73.

**FIG. 11** depicts the hydrophobicity profile of the polypeptide consisting of an amino acid sequence recited in SEQ ID NO:74.

**FIG. 12** depicts the hydrophobicity profile of the polypeptide consisting of an amino acid sequence recited in SEQ ID NO:75.

**FIG. 13** depicts the hydrophobicity profile of the polypeptide consisting of an amino acid sequence recited in SEQ ID NO:76.

**FIG. 14** depicts the hydrophobicity profile of the polypeptide consisting of an amino acid sequence recited in SEQ ID NO:77.

**FIG. 15** depicts the hydrophobicity profile of the polypeptide consisting of an amino acid sequence recited in SEQ ID NO:78.

**FIG. 16** depicts the hydrophobicity profile of the polypeptide consisting of an amino acid sequence recited in SEQ ID NO:79.

**FIG. 17** depicts the hydrophobicity profile of the polypeptide consisting of an amino acid sequence recited in SEQ ID NO:80.

**FIG. 18** depicts the hydrophobicity profile of the polypeptide consisting of an amino acid sequence recited in SEQ ID NO:81.

**FIG. 19** depicts the hydrophobicity profile of the polypeptide consisting of an amino acid sequence recited in SEQ ID NO:82.

**FIG. 20** depicts the hydrophobicity profile of the polypeptide consisting of an amino acid sequence recited in SEQ ID NO:83.

**FIG. 21** depicts the hydrophobicity profile of the polypeptide consisting of an amino acid sequence recited in SEQ ID NO:84.

**FIG. 22** depicts the hydrophobicity profile of the polypeptide consisting of an amino acid sequence recited in SEQ ID NO:85.

**FIG. 23** depicts the hydrophobicity profile of the polypeptide consisting of an amino acid sequence recited in SEQ ID NO:86.

**FIG. 24** depicts the hydrophobicity profile of the polypeptide consisting of an amino acid sequence recited in SEQ ID NO:87.

**FIG. 25** depicts the hydrophobicity profile of the polypeptide consisting of an amino acid sequence recited in SEQ ID NO:88.

**FIG. 26** depicts the hydrophobicity profile of the polypeptide consisting of an amino acid sequence recited in SEQ ID NO:89.

**FIG. 27** depicts the hydrophobicity profile of the polypeptide consisting of an amino acid sequence recited in SEQ ID NO:90.

**FIG. 28** depicts the hydrophobicity profile of the polypeptide consisting of an amino acid sequence recited in SEQ ID NO:91.

**FIG. 29** depicts the hydrophobicity profile of the polypeptide consisting of an amino acid sequence recited in SEQ ID NO:92.

**FIG. 30** depicts the hydrophobicity profile of the polypeptide consisting of an amino acid sequence recited in SEQ ID NO:93.

**FIG. 31** depicts the hydrophobicity profile of the polypeptide consisting of an amino acid sequence recited in SEQ ID NO:94.

**FIG. 32** depicts the hydrophobicity profile of the polypeptide consisting of an amino acid sequence recited in SEQ ID NO:95.

**FIG. 33** depicts the hydrophobicity profile of the polypeptide consisting of an amino acid sequence recited in SEQ ID NO:96.

**FIG. 34** depicts the hydrophobicity profile of the polypeptide consisting of an amino acid sequence recited in SEQ ID NO:97.

**FIG. 35** depicts the hydrophobicity profile of the polypeptide consisting of an amino acid sequence recited in SEQ ID NO:98.

**FIG. 36** depicts the hydrophobicity profile of the polypeptide consisting of an amino acid sequence recited in SEQ ID NO:99.

**FIG. 37** depicts the hydrophobicity profile of the polypeptide consisting of an amino acid sequence recited in SEQ ID NO:100.

**FIG. 38** depicts the hydrophobicity profile of the polypeptide consisting of an amino acid sequence recited in SEQ ID NO:101.

**FIG. 39** depicts the hydrophobicity profile of the polypeptide consisting of an amino acid sequence recited in SEQ ID NO:102.

**FIG. 40** depicts the hydrophobicity profile of the polypeptide consisting of an amino acid sequence recited in SEQ ID NO:103.

**FIG. 41** depicts the hydrophobicity profile of the polypeptide consisting of an amino acid sequence recited in SEQ ID NO:104.

**FIG. 42** depicts the hydrophobicity profile of the polypeptide consisting of an amino acid sequence recited in SEQ ID NO:105.

**FIG. 43** depicts the hydrophobicity profile of the polypeptide consisting of an amino acid sequence recited in SEQ ID NO:106.

**FIG. 44** depicts the hydrophobicity profile of the polypeptide consisting of an amino acid sequence recited in SEQ ID NO:107.

**FIG. 45** depicts the hydrophobicity profile of the polypeptide consisting of an amino acid sequence recited in SEQ ID NO:108.

**FIG. 46** depicts the hydrophobicity profile of the polypeptide consisting of an amino acid sequence recited in SEQ ID NO:109.

**FIG. 47** depicts the hydrophobicity profile of the polypeptide consisting of an amino acid sequence recited in SEQ ID NO:110.

**FIG. 48** depicts the hydrophobicity profile of the polypeptide consisting of an amino acid sequence recited in SEQ ID NO:111.

**FIG. 49** depicts the hydrophobicity profile of the polypeptide consisting of an amino acid sequence recited in SEQ ID NO:112.

**FIG. 50** depicts the hydrophobicity profile of the polypeptide consisting of an amino acid sequence recited in SEQ ID NO:113.

**FIG. 51** depicts the hydrophobicity profile of the polypeptide consisting of an amino acid sequence recited in SEQ ID NO:114.

**FIG. 52** depicts the hydrophobicity profile of the polypeptide consisting of an amino acid sequence recited in SEQ ID NO:115.

**FIG. 53** depicts the hydrophobicity profile of the polypeptide consisting of an amino acid sequence recited in SEQ ID NO:116.

**FIG. 54** depicts the hydrophobicity profile of the polypeptide consisting of an amino acid sequence recited in SEQ ID NO:117.

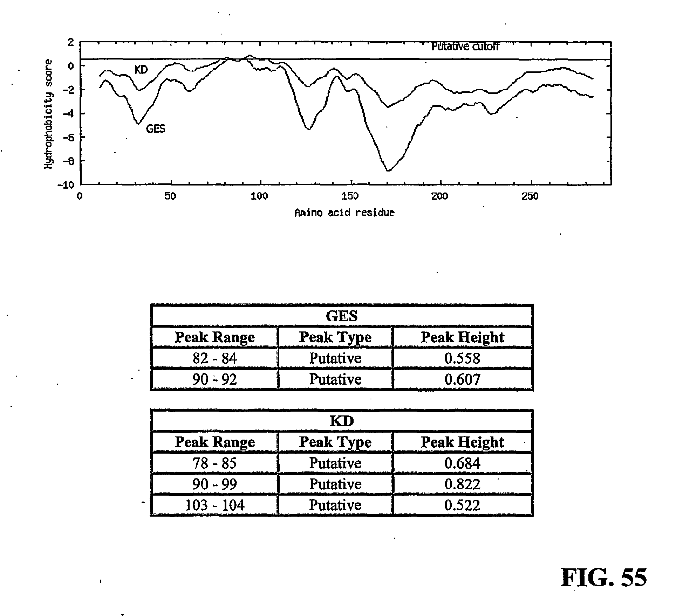

**.** **FIG. 55** depicts the hydrophobicity profile of the polypeptide consisting of an amino acid sequence recited in SEQ ID NO:118.

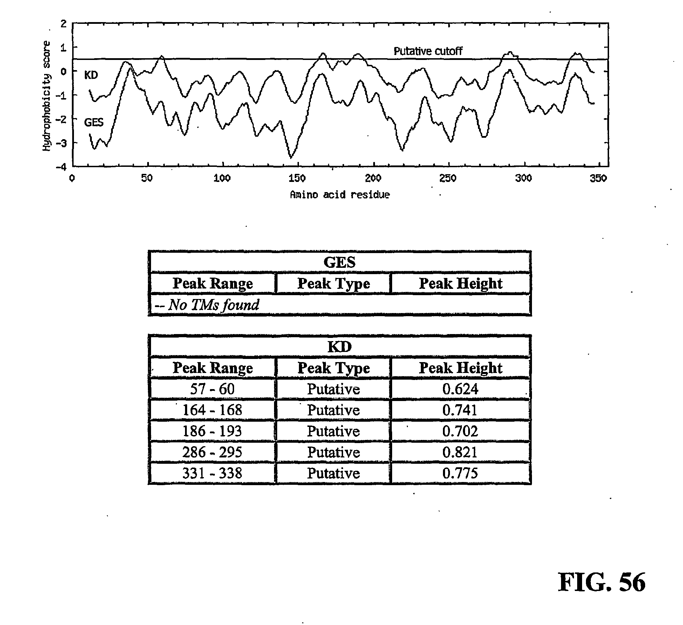

FIG. **56** depicts the hydrophobicity profile of the polypeptide consisting of an amino acid sequence recited in SEQ ID NO:119.

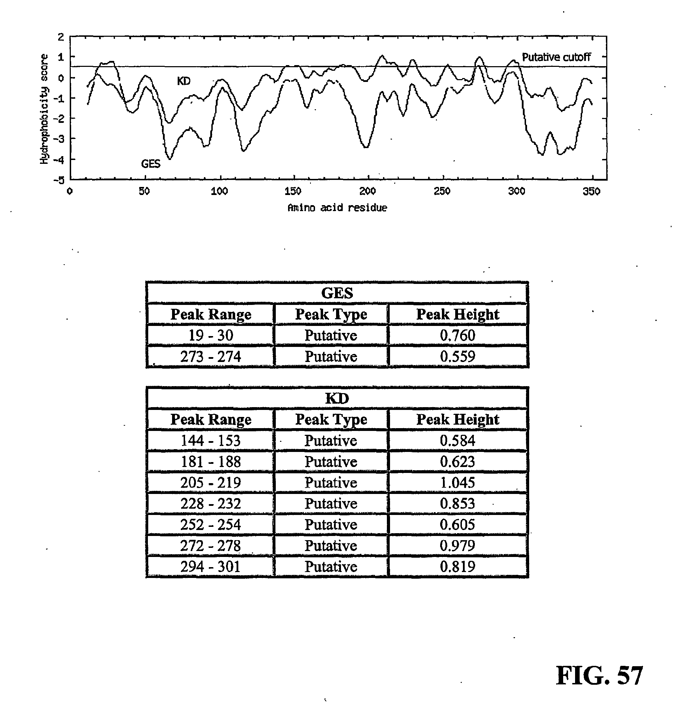

**FIG. 57** depicts the hydrophobicity profile of the polypeptide consisting of an amino acid sequence recited in SEQ ID NO: 120.

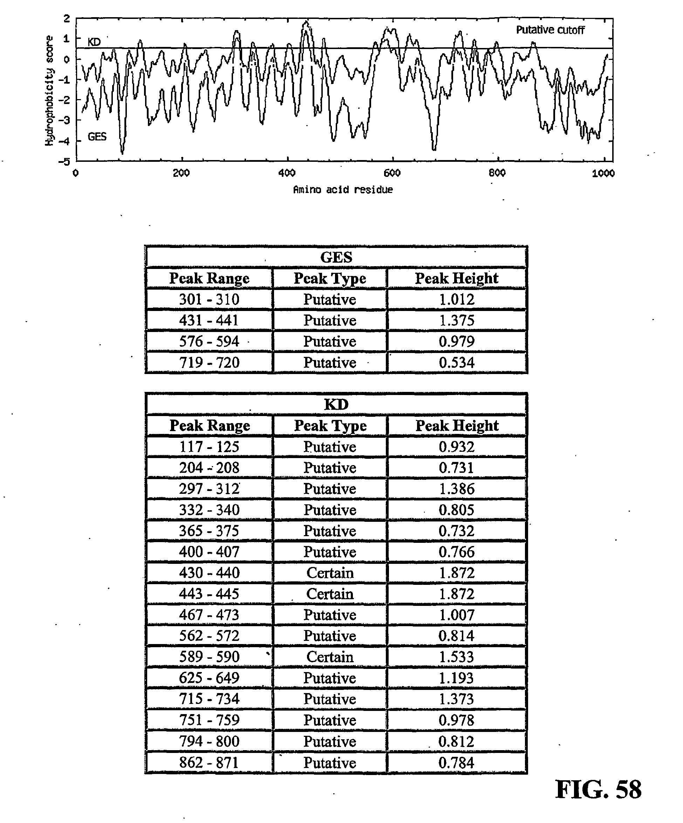

**FIG. 58** depicts the hydrophobicity profile of the polypeptide consisting of an amino acid sequence recited in SEQ ID NO:121.

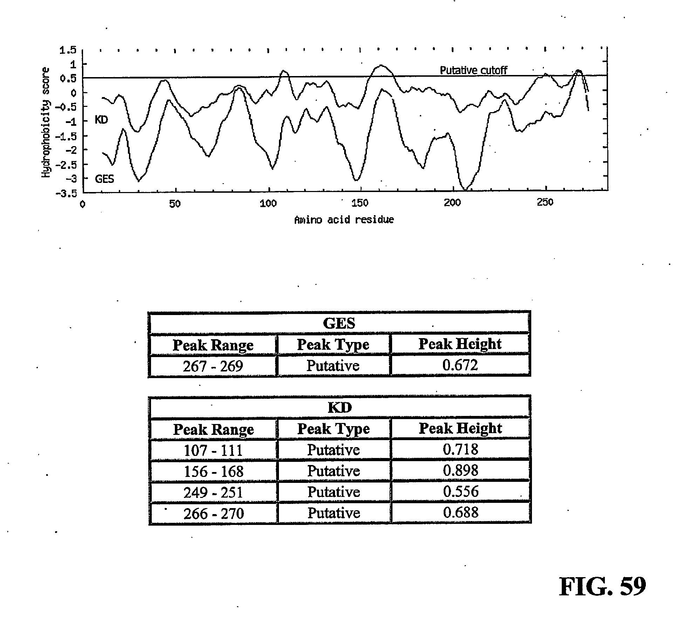

**FIG. 59** depicts the hydrophobicity profile of the polypeptide consisting of an amino acid sequence recited in SEQ ID NO:122.

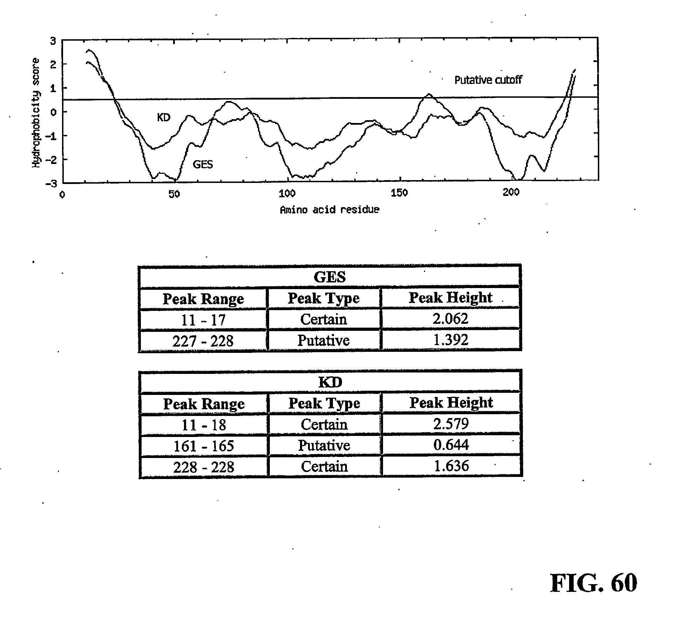

**FIG. 60** depicts the hydrophobicity profile of the polypeptide consisting of an amino acid sequence recited in SEQ ID NO:123.

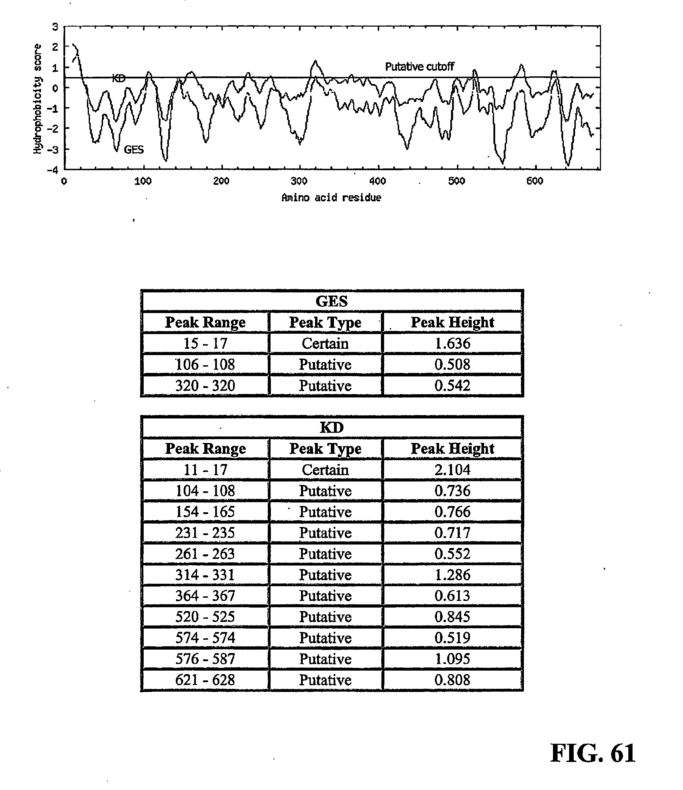

**FIG. 61** depicts the hydrophobicity profile of the polypeptide consisting of an amino acid sequence recited in SEQ ID NO:124.

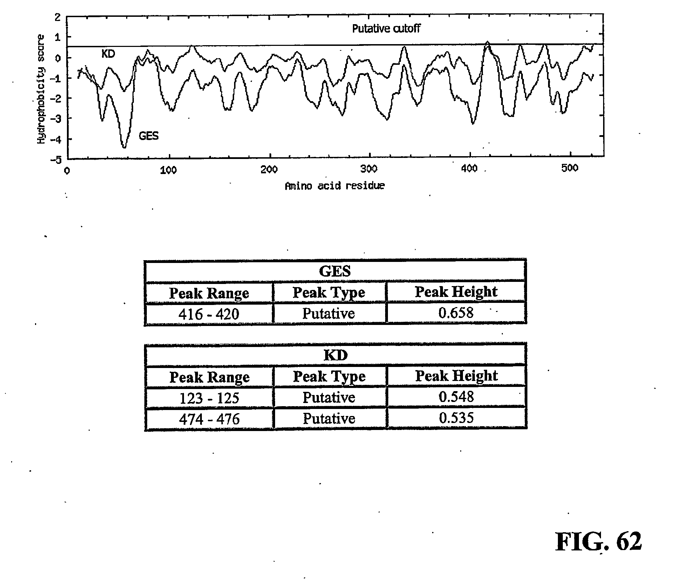

**FIG. 62** depicts the hydrophobicity profile of the polypeptide consisting of an amino acid sequence recited in SEQ ID NO:125.

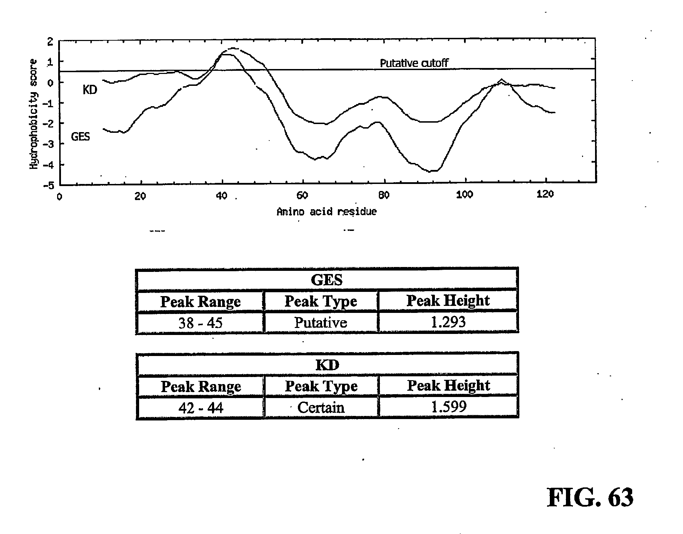

**FIG. 63** depicts the hydrophobicity profile of the polypeptide consisting of an amino acid sequence recited in SEQ ID NO:126.

### DETAILED DESCRIPTION OF THE INVENTION

Various aspects of the invention are described in further detail in the following subsections. The use of subsections is not meant to limit the invention; subsections may apply to any aspect of the invention. In this application, the use of "or" means "and/or" unless stated otherwise.

### Colon Cancer Genes (CCGs)

The present invention provides compositions, equipment, and methods of using colon cancer genes (CCGs) for the prognosis, diagnosis, prevention, or treatment of colon cancer. The present invention also provides methods for the identification of novel therapeutic agents for colon cancer, and animal models for studying the pathogenesis of the disease. A colon cancer gene is a gene that is differentially expressed in colon cancer cells as compared to disease-free colon cells.

In one embodiment, the colon cancer genes are differentially expressed not only between colon cancer tissues and disease-free colon tissues, but also between colon cancer tissues and one or more other disease-free tissues. These other disease tissues include, for example, cervix, kidney, left atrium, left ventricle, right atrium, right ventricle, lung, ovary, prostate, rectum, skin, and stomach tissues. In many examples, the p-value of the differentiation expression analysis for each selected colon cancer gene is no more than 0.1, 0.05, 0.001, 0.0005, 0.0001, or less.

In another embodiment, the colon cancer genes are over-expressed in colon cancer tissues relative to one or more disease-free tissues. In certain cases, the average expression level of each colon cancer gene in colon cancer cells is at least 0.5, 1, 2, 3, 4, 5, 10, 20, or more times higher than that in disease-free colon cells.

In yet another embodiment, 63 colon cancer genes are provided in Tables 1-5. These genes are over-expressed in colon cancer cells by at least 2-fold as compared to disease-free colon cells. These CCGs can be classified into the following five groups according to their functional categories:
Group I. 8 Kinases;
Group II. 12 Non-kinase enzymes or their inhibitors;
Group III. 3 Receptors;
Group IV. 3 Ion channels; and
Group V. 37 Other known genes

### Group I genes

As shown in Table 1, genes in Group I include 8 protein kinases.

**Table 1. Group I genes: Kinases**

| Gene symbol | Locus link. | Nucleic acid seq. | Amino acid seq. |
|---|---|---|---|
| ZAK | 51776 | SEQ ID NO:1 | SEQ ID NO:64 |
| STK15 | 8465 | SEQ ID NO:2 | SEQ ID NO:65 |
| MAD2L1 | 4085 | SEQ ID NO:3 | SEQ ID NO:66 |
| CDC2 | 983 | SEQ ID NO:4 | SEQ ID NO:67 |
| C20orf97 | 57761 | SEQ ID NO:5 | SEQ ID NO:68 |
| TTK | 7272 | SEQ ID NO:6 | SEQ ID NO:69 |
| CKS2 | 1164 | SEQ ID NO:7 | SEQ ID NO:70 |
| MET | 4233 | SEQ ID NO:8 | SEQ ID NO:71 |

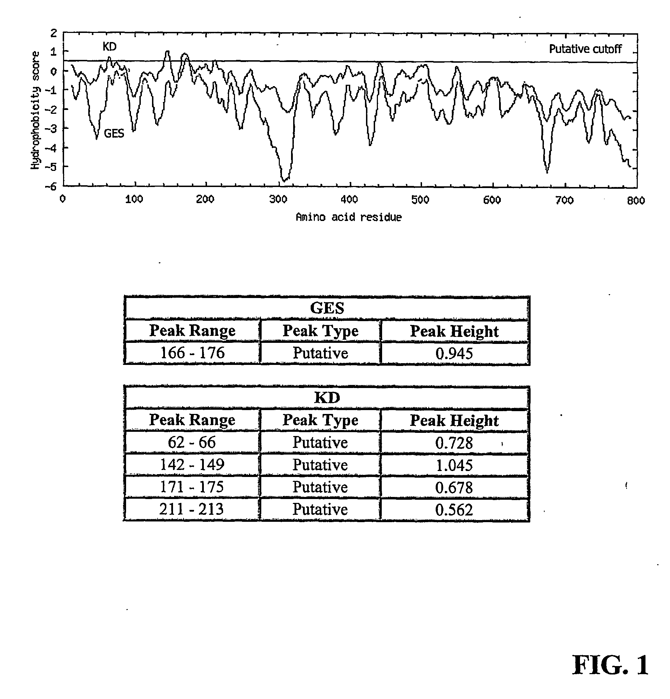

ZAK (sterile alpha motif and leucine zipper containing kinase AZK) gene encodes a mixed lineage kinase with a leucine zipper and a sterile alpha motif. ZAK proteins may form homodimers or oligomers in mammalian cells. The expression of ZAK in mammalian cells may lead to the activation of the JNK/SAPK pathway as well as the activation of transcription factor, NF-kappaB. Over-expression of the ZAK gene has been reported to induce the apoptosis of a hepatoma cell line. The hydrophobicity profile of ZAK is shown in FIG. 1.

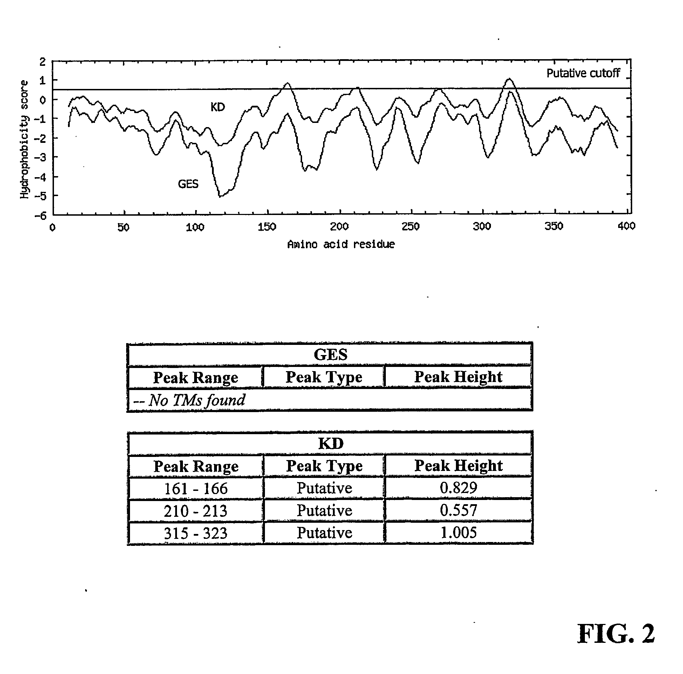

STK15 (ser/thr kinase 15) gene has been reported to be amplified or over-expressed in three human breast cancer cell lines. STK15 encodes a centrosome-associated kinase and is involved in the induction of centrosome duplication-distribution abnormalities and aneuploidy in mammalian cells. STK15 over-expression leads to centrosome amplification, chromosomal instability, and aneuploidy seen in many cancer cell types. The hydrophobicity profile of STK15 is shown in FIG. 2.

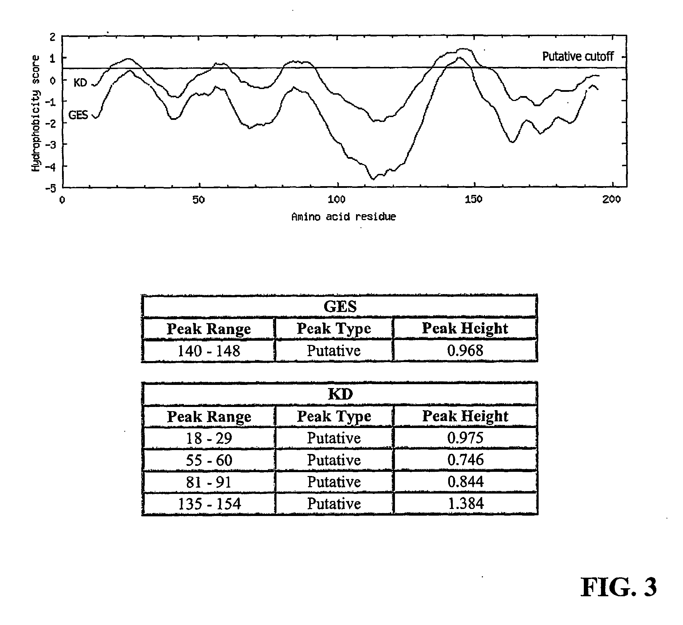

MAD2L1 (mitotic arrest deficient-like 1 (yeast)) may be involved in the execution of the mitotic checkpoint. Mitotic checkpoint control mechanisms check the cells preparedness to undergo division. Through these mechanisms cell cycle progression is blocked before the irreversible events associated with anaphase if either the mitotic spindle apparatus is not properly assembled or the kinetochore is not properly attached to the spindle. Dysfunction of MAD2 may lead to malignancy or degeneration of cells. The human breast tumor cell line T47D has reduced MAD2 expression, and it failed to arrest in mitosis after nocodazole treatment. It was proposed that loss of MAD2 function might also lead to aberrant chromosome segregation in mammalian cells. Deletion of one MAD2 allele results in a defective mitotic checkpoint in both human cancer cells and murine primary embryonic fibroblasts. Checkpoint-defective cells show premature sister chromatid separation in the presence of spindle inhibitors and an elevated rate of chromosome missegregation events in the absence of these agents. Furthermore, MAD2 +/- mice develop lung tumors at high rates after long latencies, implicating defects in the mitotic checkpoint in tumorigenesis. The hydrophobicity profile of MAD2 is shown in FIG. 3.

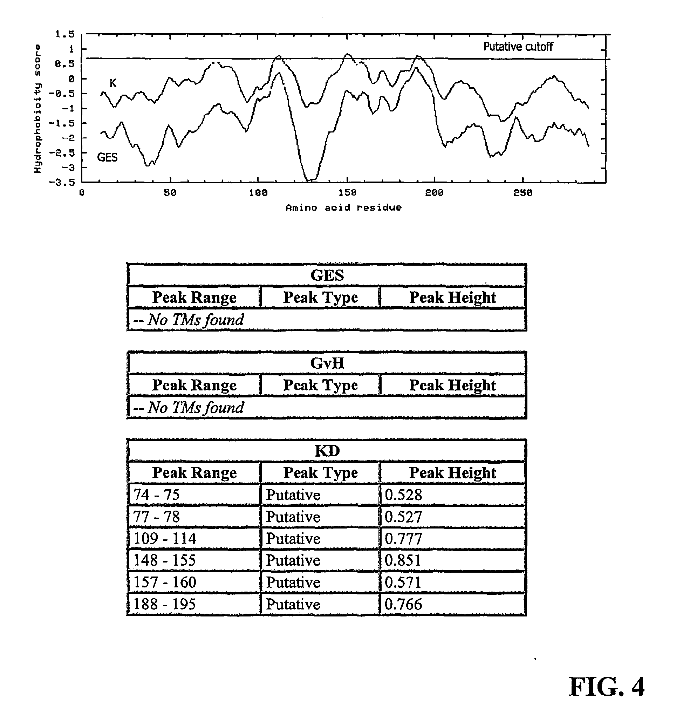

CDC2 (cell division cycle 2, G1 to S and G2 to M) gene encodes a member of the ser/thr protein kinase family. CDC2 protein is the catalytic subunit of a highly conserved protein kinase complex known as M-phase promoting factor (MPF), which is essential for G1/S and G2/M phase transitions of eukaryotic cell cycle. Mitotic cyclins stably associate with this protein and function as regulatory subunits. The kinase activity of CDC2 is controlled by cyclin accumulation and destruction through the cell cycle. The phosphorylation and dephosphorylation of CDC2 may also play regulatory roles in cell cycle control. The hydrophobicity profile of CDC2 is shown in FIG. 4.

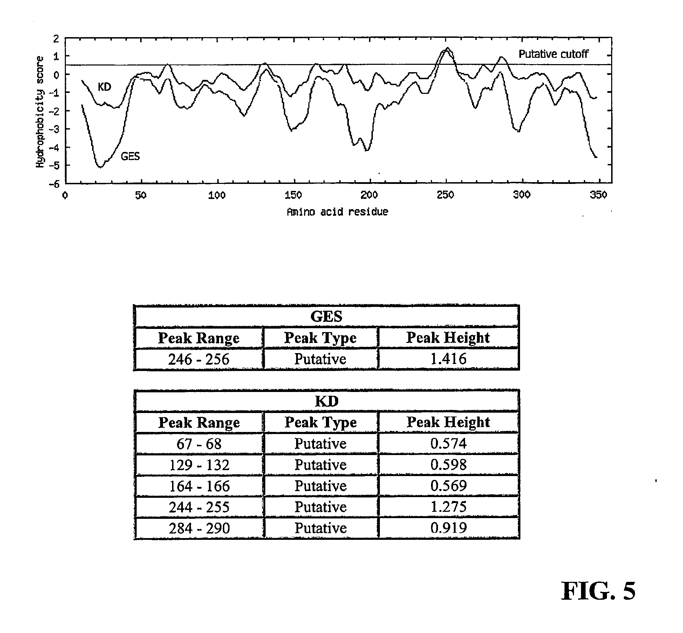

C20orf97 (chromosome 20 open reading frame 97) is a protein-kinase*-domains-containing protein similar to human C8FW, a phosphoprotein regulated by mitogenic pathways. The hydrophobicity profile of C20orf97 is shown in FIG. 5.

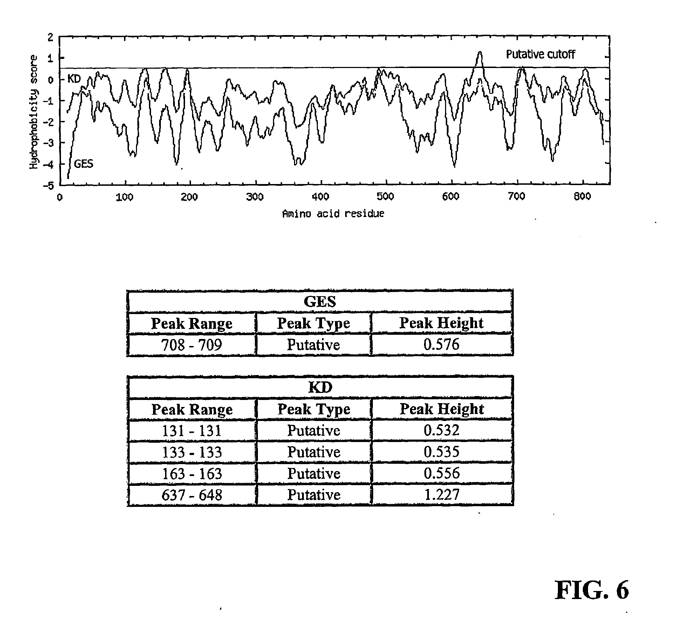

TTK protein kinase (Msp1 p homolog) gene encodes a dual specificity ser/thr and tyrosine kinase. The mouse Mpsl ortholog, Esk, regulates centrosome duplication. Endogenous Esk and over-expressed GFP-Esk are localized to centrosomes and kinetochores in mouse cells. Over-expression of GFP-Esk caused reduplication of centrosomes during S-phase arrest. In contrast, a kinase-deficient mutant blocked centrosome duplication altogether. The hydrophobicity profile of TTK is shown in FIG. 6.

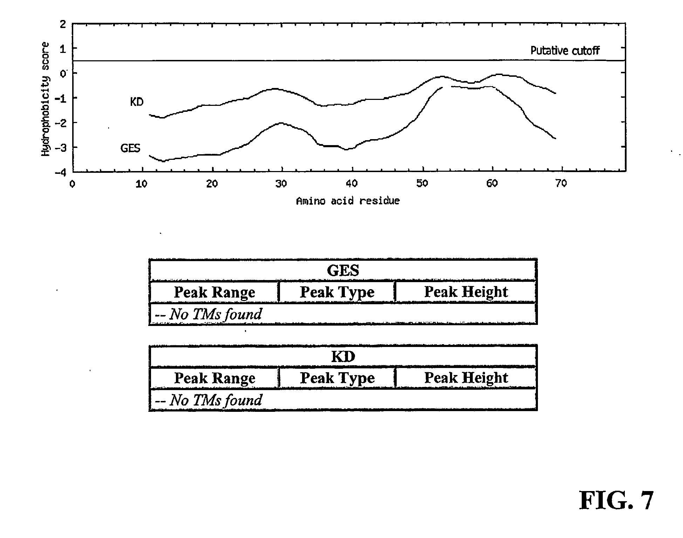

CKS2 (CDC28 protein kinase 2) protein binds to the catalytic subunit of the cyclin dependent kinases and is essential for their biological function. The CKS2 mRNA is found to be expressed in different patterns through the cell cycle in HeLa cells, which reflects specialized role for the encoded protein. The hydrophobicity profile of CKS2 is shown in FIG. 7.

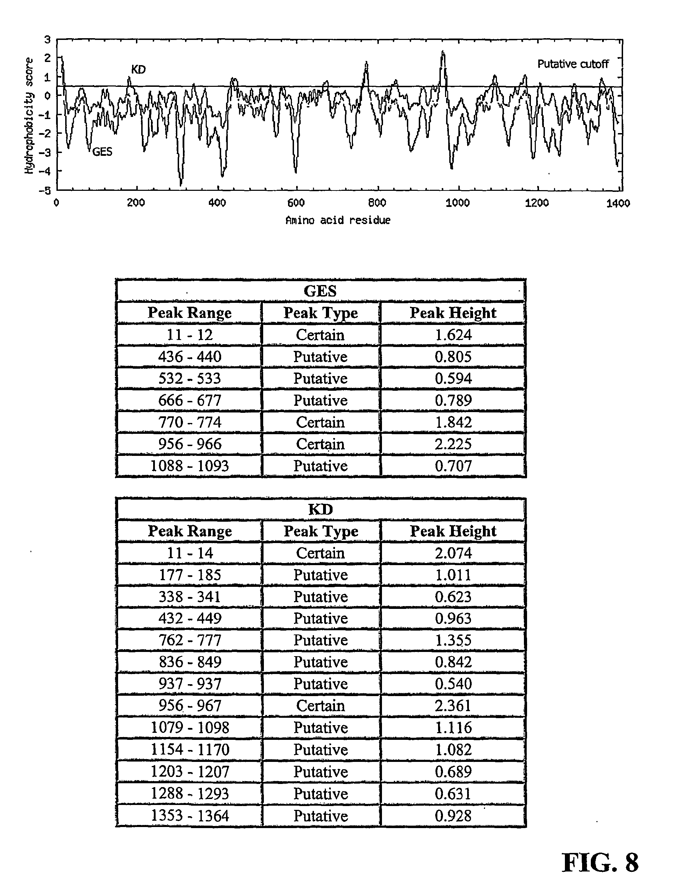

MET (met proto-oncogene) product is the hepatocyte growth factor receptor and has tyrosine-kinase activity. The primary single chain MET precursor is post-translationally cleaved to produce the alpha and beta subunits, which are disulfide linked to form the mature receptor. Various mutations in the MET gene are associated with papillary renal carcinoma. The hydrophobicity profile of MET is shown in FIG. 8.

### Group II genes

As shown in Table 2, genes in Group II include 12 non-kinase enzymes or their inhibitors. Among them, four genes relate to matrix metalloproteinases (MMP), seven geens relate to metabolic enzymes or their inhibitors, and one gene relates to tyrosine phosphatase.

**Table 2. Group II genes: Non-kinase enzymes**

| Gene symbol | Locus link. | Nucleic acid seq. | Amino acid seq. |
|---|---|---|---|
| MMP1 | 120353 | SEQ ID NO:9 | SEQ ID NO:72 |
| MMP3 | 185250 | SEQ ID NO:10 | SEQ ID NO:73 |
| MMP11 | 185261 | SEQ ID NO:11 | SEQ ID NO:74 |
| MMP12 | 601046 | SEQ ID NO:12 | SEQ ID NO:75 |
| NES1 | 602673 | SEQ ID NO:13 | SEQ ID NO:76 |
| CAST | 831 | SEQ ID NO:14 | SEQ ID NO:77 |
| CST1 | 123855 | SEQ ID NO:15 | SEQ ID NO:78 |
| SQLE | 6713 | SEQ ID NO:16 | SEQ ID NO:79 |
| LOXL2 | 4017 | SEQ ID NO:17 | SEQ ID NO:80 |
| PUS1 | 80324 | SEQ ID NO:18 | SEQ ID NO:81 |
| TOMM34 | 10953 | SEQ ID NO:19 | SEQ ID NO:82 |
| CDC25B | 994 | SEQ ID NO:20 | SEQ ID NO:83 |

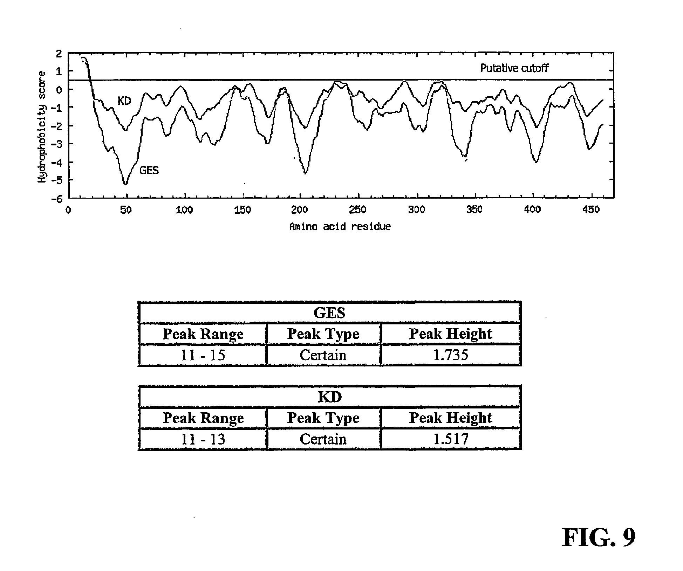

MMP1 (matrix metalloproteinase 1), also referred to as collagenase, is the only enzyme able to initiate breakdown of the interstitial collagen types I, II, and III. The fact that the collagens are abundant in the body means that MMP1 plays a key role in the remodeling that occurs constantly in both normal and diseased conditions. The identity of human skin and synovial cell collagenase and the ubiquity of this enzyme and of its substrates, collagens I, II, and III, imply that the common mechanism controlling collagenolysis throughout the body may be operative in both normal and disease states. The hydrophobicity profile of MMP1 is shown in FIG. 9.

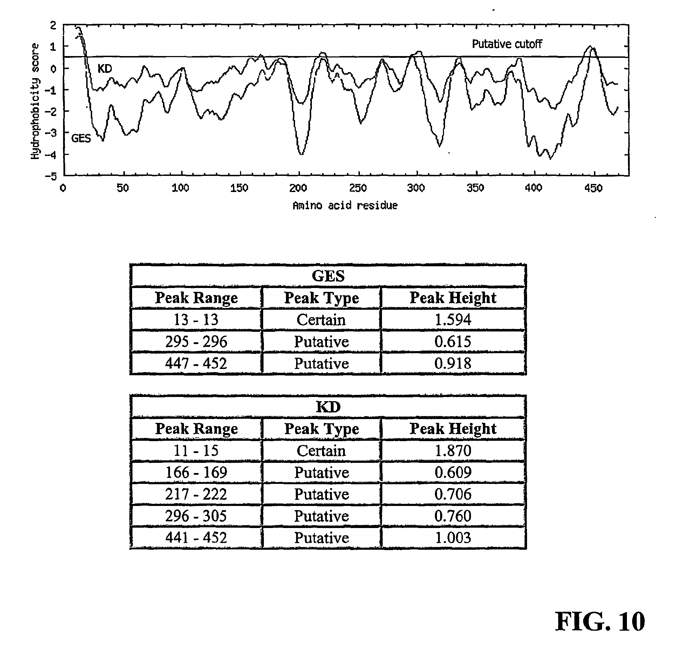

MMP3 (matrix metalloproteinase 3), also referred to as stromelysin 1, progelatinase or transin, is a proteoglycanase closely related to collagenase (MMP1) with a wide range of substrate specificities. It is a secreted metalloprotease produced predominantly by connective tissue cells. Together with other metalloproteases, MMP3 can synergistically degrade the major components of the extracellular matrix. MMP3 is capable of degrading proteoglycan, fibronectin, laminin, and type IV collagen, but not interstitial type I collagen. R4Be3 also promotes spontaneous premalignant changes and malignant conversion in mammary glands of transgenic mice. These changes were blocked by coexpression of a TIMP1 transgene. The premalignant and malignant lesions had stereotyped genomic changes unlike those seen in other murine mammary cancer models. These data indicated that MMP3 influences tumor initiation and alters neoplastic-risk. The hydrophobicity profile of MMP3 is shown in FIG. 10.

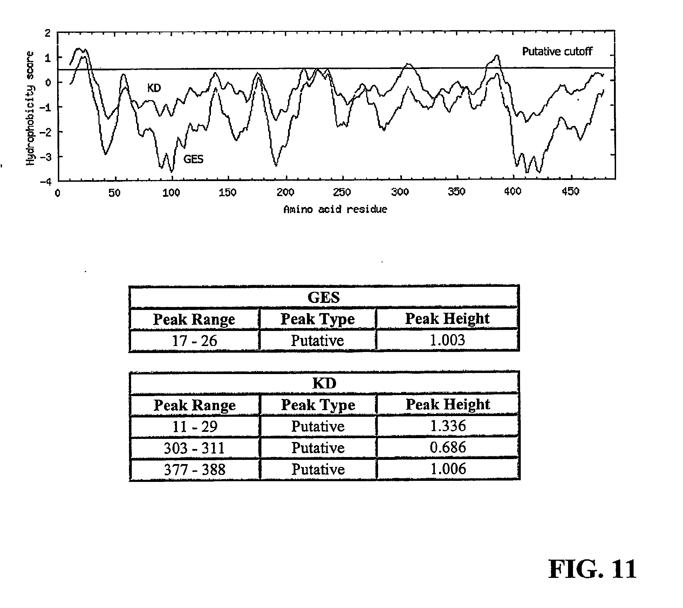

MMP11 (matrix metalloproteinase 11), also referred to as stromelysin 3, is a member of the MMP gene family. MMP11 is over-expressed in the stromal cells of invasive breast carcinomas but not in the stromal cells surrounding benign breast fibroadenomas. The hydrophobicity profile of MMP11 is shown in FIG. 11.

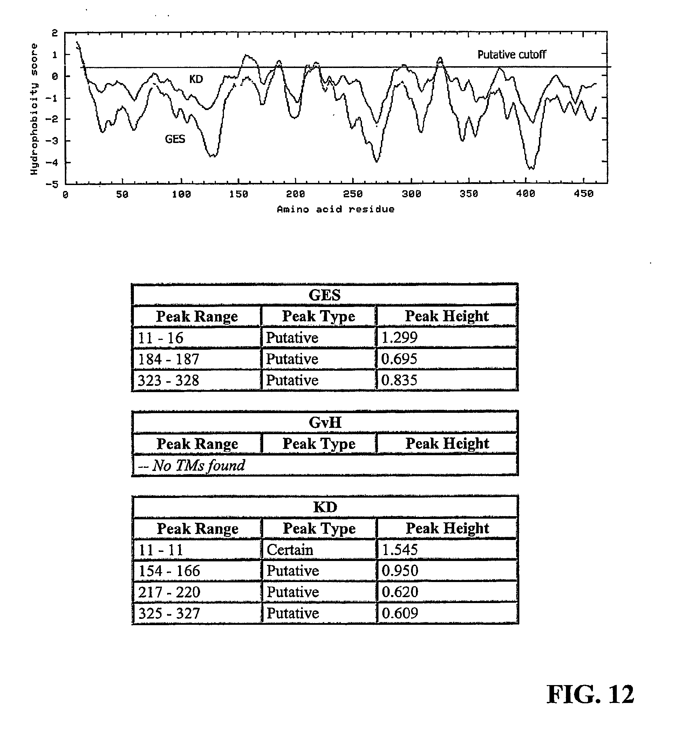

MMP12 (matrix metalloproteinase 12), also referred to as human macrophage metalloelastase (HME), has been shown to convert plasminogen into angiostatin, an essential inhibitor of tumor angiogenesis. MMP12 plays a role in the inhibition of tumor progression in patients with colorectal carcinoma. MMP12 over-expression is correlated closely with a better prognosis. The hydrophobicity profile of MMP12 is shown in FIG. 12.

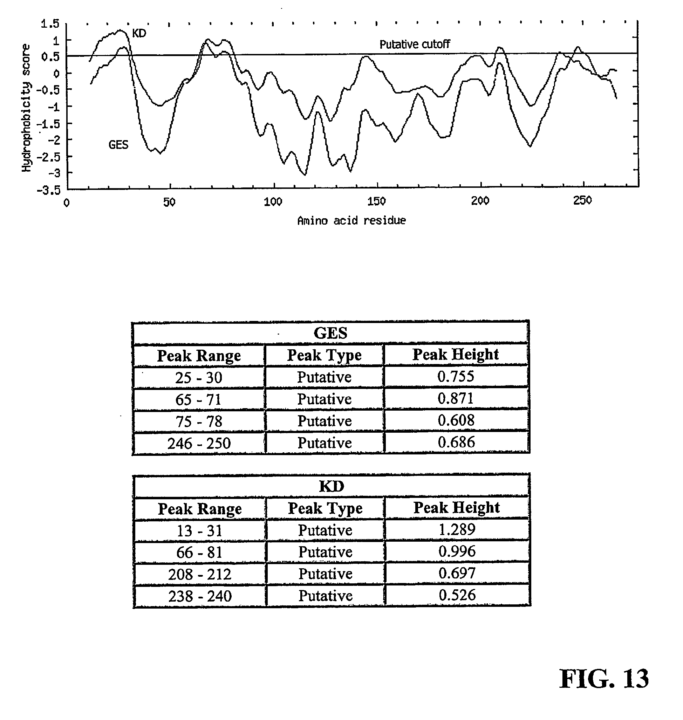

NES1 (normal epithelial-cell specific-1), also referred to as Kallikreln 10, is a trypsin-like serine protease. The NES1 gene is expressed in normal mammary epithelial cells, but its expression is dramatically decreased in breast cancer cell lines. Hypermethylation may be responsible for the tumor-specific loss of NES1 gene expression. Results suggest that hypermethylation of the NES1 gene may serve as a potential marker for breast cancer. The hydrophobicity profile of NES1 is shown in FIG. 13.

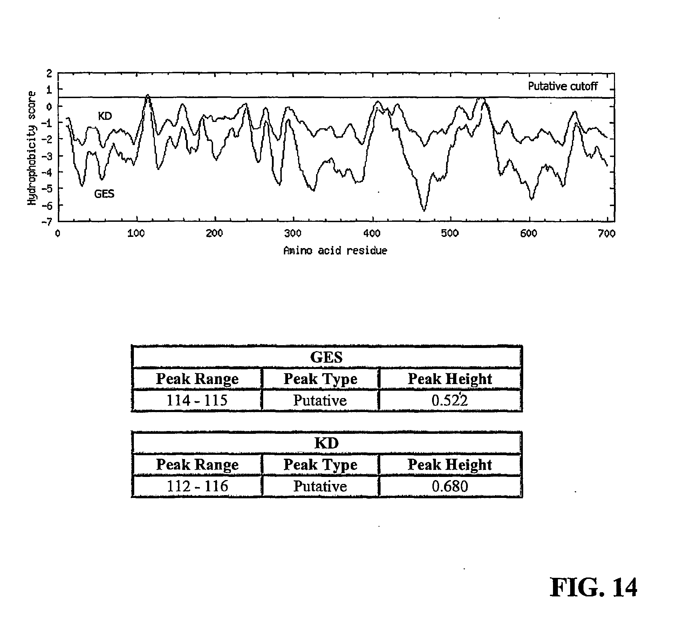

CAST (calpastatin) is a specific inhibitor of calpain. Calpastatin consists of a unique N-terminal domain (domain L) and four repetitive protease inhibitor domains (domains 1-4). It has been suggested that calpains may contribute to the regulation of wild-type p53 protein levels *in vivo* and calpastatin enhances p53 stability by inhibiting calpain activity. The hydrophobicity profile of CAST is shown in FIG. 14.

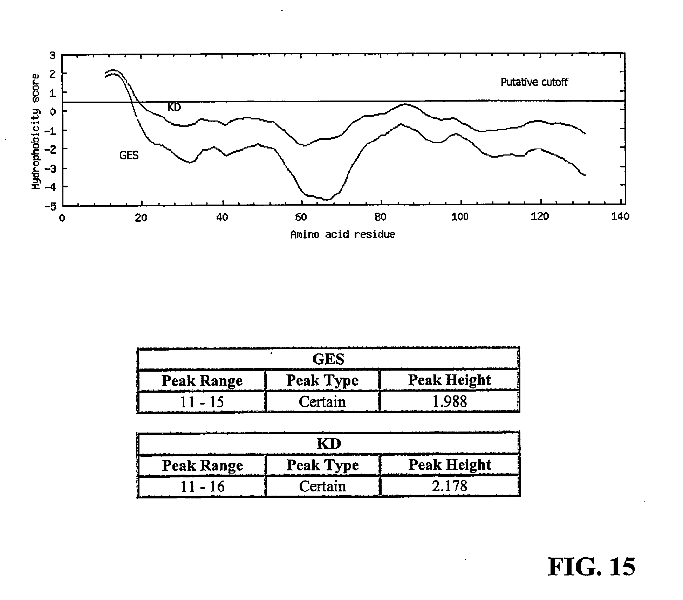

CST1 (cystatin SN) is a cysteine-proteinase inhibitor. Salivary cystatins, known as cystatins S, SA and SN, are mainly found in saliva, tears, and seminal plasma. No reports have been made on an association with this gene and cancer. The hydrophobicity profile of CST1 is shown in FIG. 15.

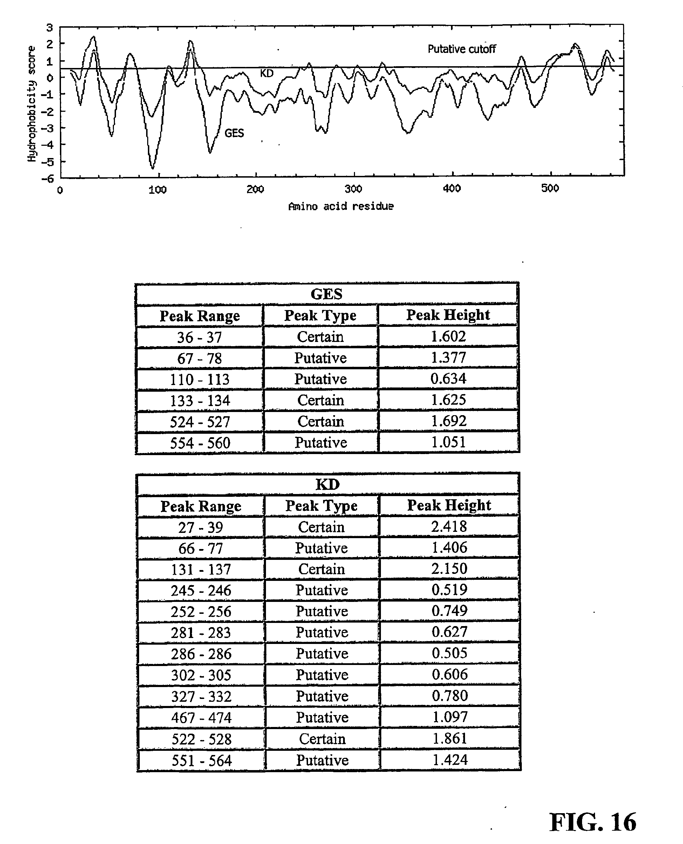

SQLE (squalene epoxidase) catalyzes the first oxygenation step in sterol (like cholesterol) biosynthesis and is thought to be one of the rate-limiting enzymes in this pathway. The hydrophobicity profile of SQLE is shown in FIG. 16.

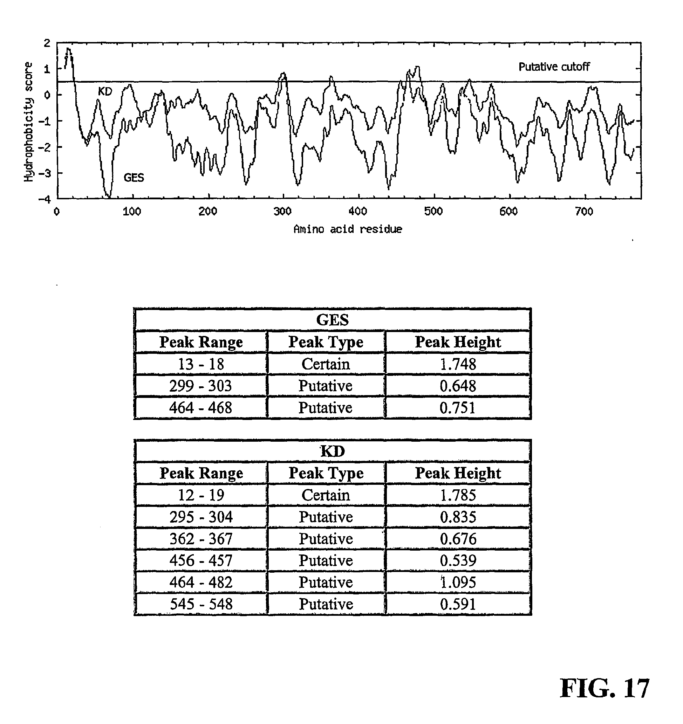

LOXL2 (lysyl oxidase-like 2) is involved in the main pathway of collagen and elastin cross-linking. It has a role in the maturation of fibrillar matrix proteins in fibrosing processes and dictates their stability against metal loproteases. Lysyl oxidase has been seen differentially expressed in renal cell carcinoma relative to normal tissue. The hydrophobicity profile of LOXL2 is shown in FIG. 17.

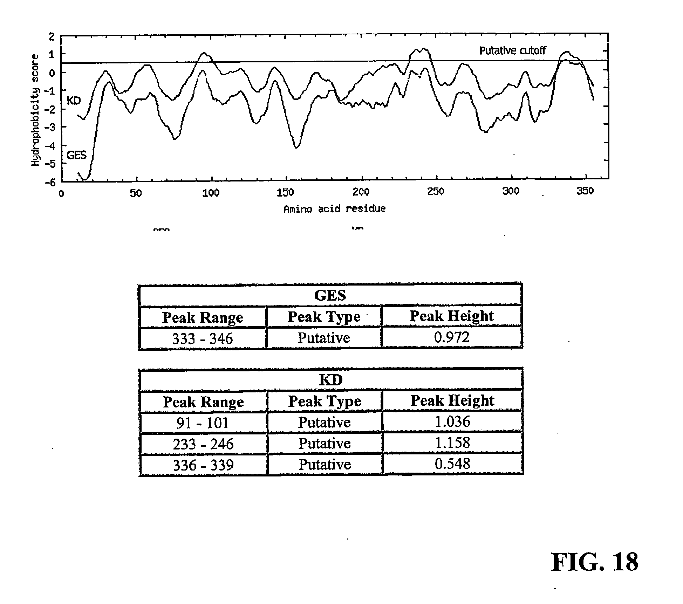

PUS1 (pseudouridylate synthase 1) is involved in the synthesis of pseudouridine. Numerous pseudouridine residues are present in rRNAs and tRNAs from archaea, bacteria, and eukarya and in UsnRNAs from eukarya. There are no reports linking PUS1 expression to cancer. The hydrophobicity profile of PUS1 is shown in FIG. 18.

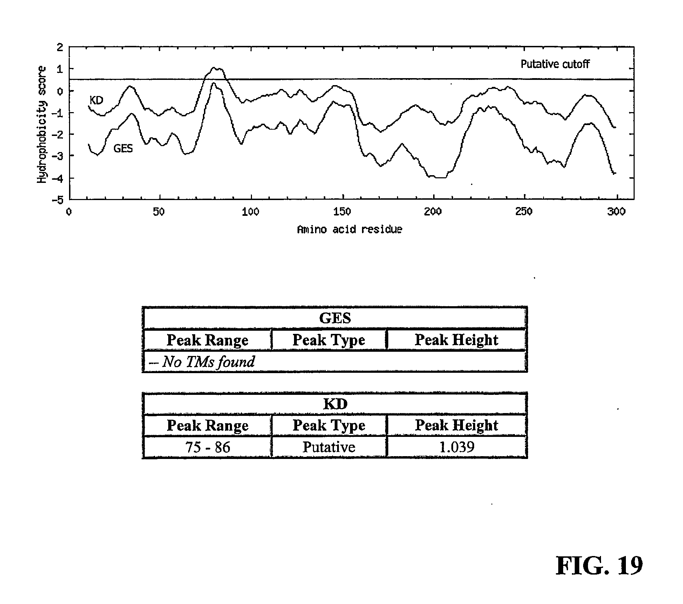

TOMM34 encodes the 34-kDa translocase of the outer mitochondrial membrane. TOMM34 is a subunit of the translocase of the outer mitochondrial membrane and a component of the mitochondrial protein import complex. The hydrophobicity profile of TOMM34 is shown in FIG. 19.

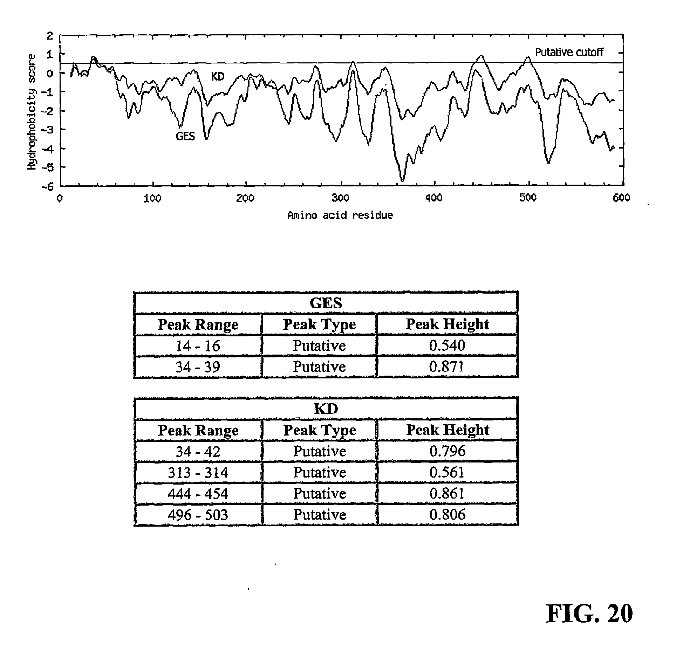

CDC25B (cell division cycle 25B) is a member of the CDC25 family of phosphatases. CDC25B activates the cyclin dependent kinase CDC2 by removing two phosphate groups and is required for entry into mitosis. CDC25B shuttles between the nucleus and the cytoplasm due to nuclear localization and nuclear export signals. The protein is nuclear in the M and G1 phases of the cell cycle and moves to the cytoplasm during S and G2. CDC25B has oncogenic properties, although its role in tumor formation has not been determined. The hydrophobicity profile of CDC25B is shown in FIG. 20.

### Group III genes

As shown in Table 3, Group III includes 3 receptor genes:

**Table 3. Group III genes: Receptors**

| Gene symbol | Locus link. | Nucleic acid seq. | Amino acid seq. |
|---|---|---|---|
| GPR49 | 8549 | SEQ ID NO:21 | SEQ ID NO:84 |
| GABBR1 | 603540 | SEQ ID NO:22 | SEQ ID NO:85 |
| OR2L6P | 81465 | SEQ ID NO:23 | SEQ ID NO:86 |

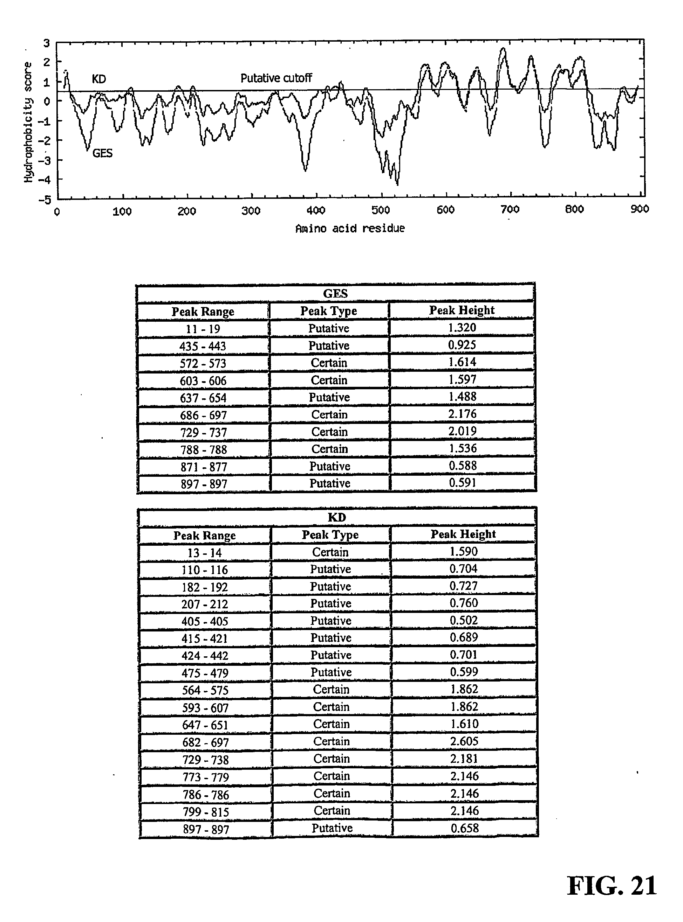

GPR49 (G protein-coupled receptor 49) is an orphan-G protein-coupled receptor with an unknown ligand. Expression of GPR49 gene has been reported in brain, skeletal muscle, placenta, and spinal cord. The hydrophobicity profile of GPR49 is shown in FIG. 21.

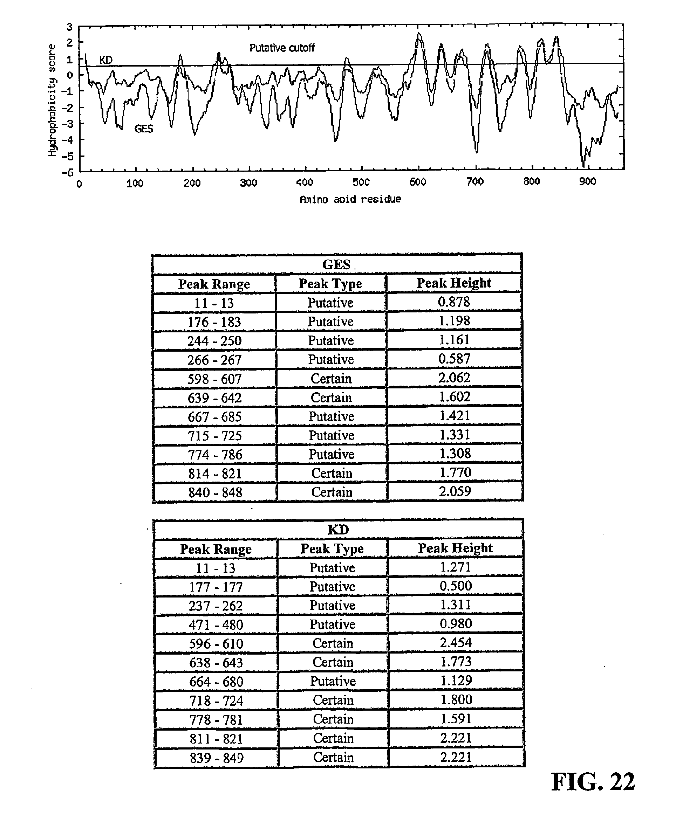

GABBR1 (Gamma-aminobutyric acid (GABA) B receptor 1) is a member of the GABA receptor family. GABA receptors are involved in the GABAergic neurotransmission of the mammalian central nervous system. The metabotropic GABA(B) receptors are coupled to G proteins and modulate synaptic transmission through intracellular effector systems. GABA(B) receptors function by inhibiting presynaptic transmitter release or by increasing the potassium conductance responsible for long-lasting inhibitory postsynaptic potentials. Findings indicate that GABA inhibits gastric carcinogenesis via the GABA(B) receptor and that this effect may be related to its effect in decreasing the proliferation of antral mucosa. The hydrophobicity profile of GABBR1 is shown in FIG. 22.

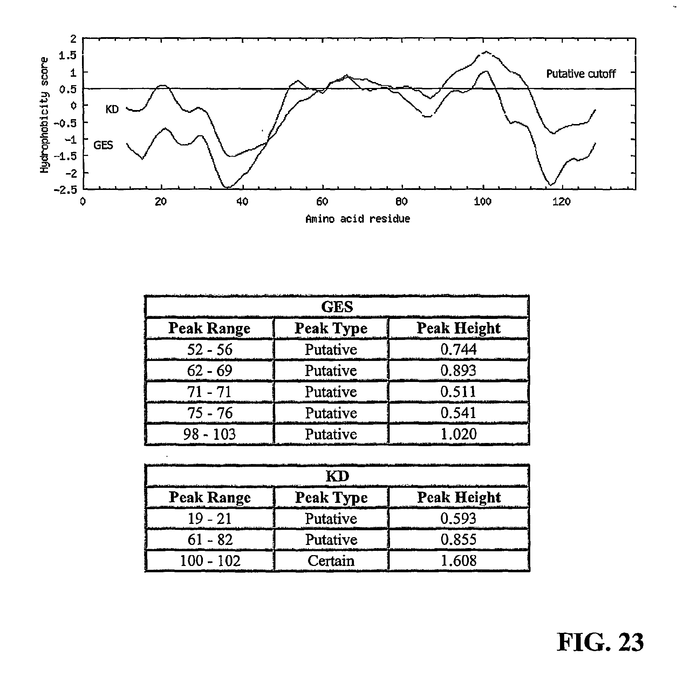

OR2L6P (Olfactory receptor, family 2, subfamily L, member 6 pseudogene) is a member of the olfactory receptor family. Olfactory receptors are believed to be encoded by an extremely large subfamily of G-protein-coupled receptors. These receptors share a 7-transmembrane domain structure with many neurotransmitter and hormone receptors. Olfactory receptors are responsible for the recognition and G-protein-mediated transduction of odorant signals. The hydrophobicity profile of OR2L6P is shown in FIG. 23.

### Group IVgenes

As shown in Table 4, Group IV includes 3 ion channel genes:

**Table 4. Group IV genes: Ion channel**

| Gene symbol | Locus link. | Nucleic acid seq. | Amino acid seq. |
|---|---|---|---|
| SLC12A2 | 6558 | SEQ ID NO:24 | SEQ ID NO:87 |
| SLC21A12 | 28231 | SEQ ID NO:25 | SEQ ID NO:88 |
| SLC7A5 | 600182 | SEQ ID NO:26 | SEQ ID NO:89 |

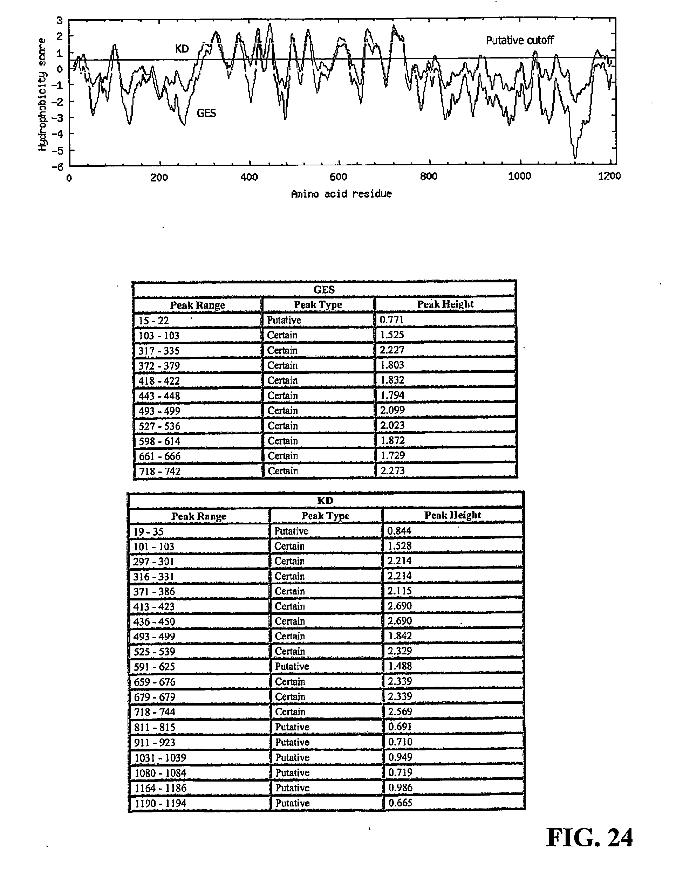

SLC12A2 (solute carrier family 12, member 2) encodes a Na⁺/K⁺/CI⁻ transporter. The Na⁺-K⁺-CI⁻ cotransporters are a family of integral membrane proteins that mediate the coupled transport of Na⁺, K⁺, and CI⁻ across the plasma membrane. The vectorial transport of chloride across epithelia is a prominent mechanism in the maintenance of water and electrolyte homeostasis. Chloride transport is involved in reabsorption of NaCl in the thick ascending limb of the loop of Henle in mammalian kidney and in secretion of NaCl in a diverse array of secretory epithelia, including the intestine, trachea, and parotid and the avian and elasmobranch salt glands. In all of these tissues, the chloride entry into the epithelia cell is mediated by a Na⁺-K⁺-CI⁻ cotransporter protein, which couples the electroneutral movement of sodium, potassium, and chloride ions. In order to carry out net salt transport, the Na⁺-K⁺-CI⁻ cotransporter functions in concert with three other membrane proteins: chloride channel, potassium channels and sodium pump. The importance of the proper functioning of these ion transport mechanisms in chloride secretory epithelia is exemplified by the disease states of cystic fibrosis and secretory diarrhea, where there are defects in the regulation of ion transport. The hydrophobicity profile of SLC12A2 is shown in FIG. 24.

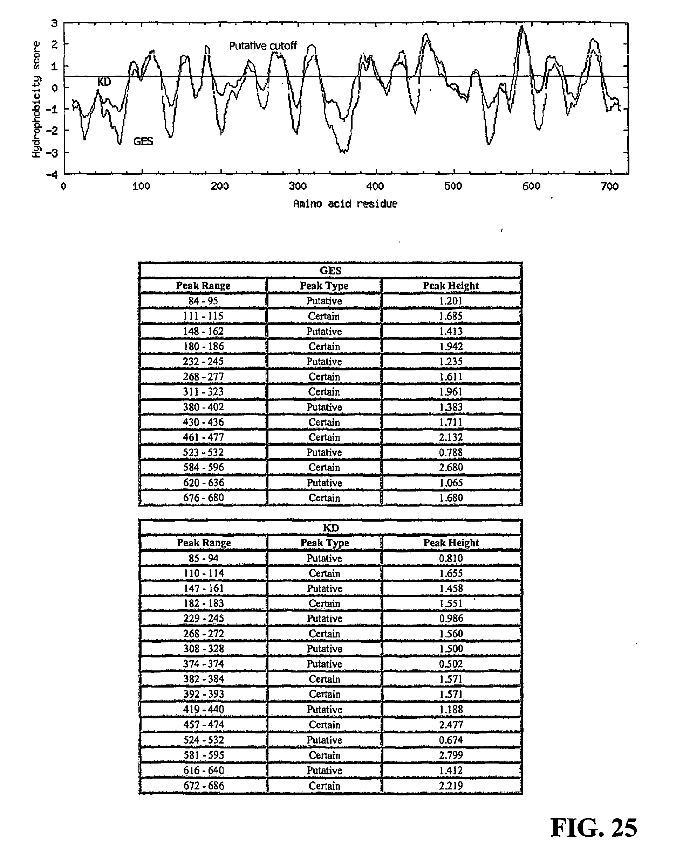

SLC21A12 (solute carrier family 21, member 12) gene encodes an organic anion transporter. SLC21A12 gene has not been linked to colon cancer. The hydrophobicity profile of SLC12A12 is shown in FIG. 25.

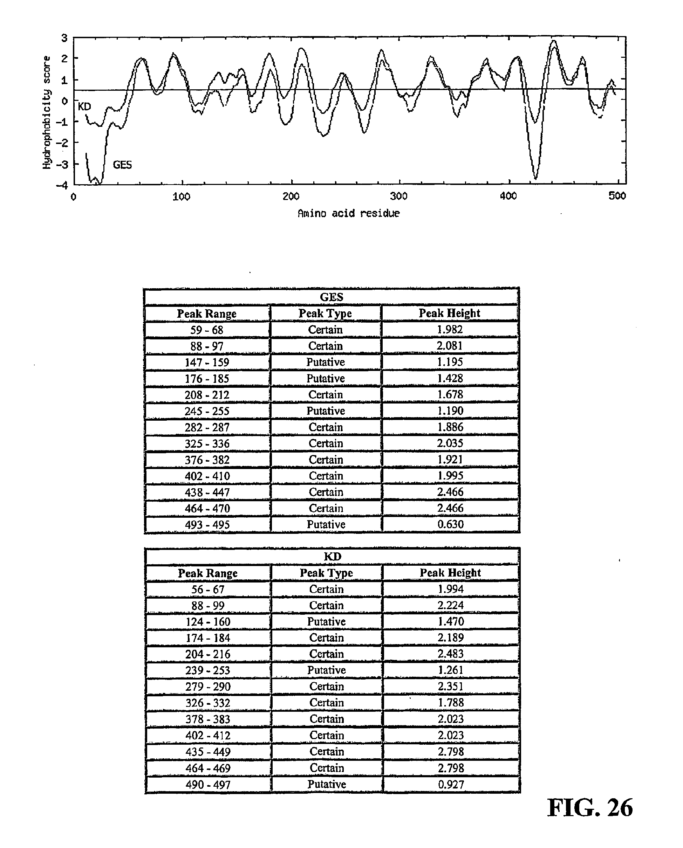

SLC7A5 (solute carrier family 7, member 5) encodes a cationic amino acid transporter (y+ system). It is also referred to as membrane protein E16 (MPE16) or L-type ammo acid transporter 1 (LAT1). SLC7A5 transports large neutral amino acids across cell membrane. The hydrophobicity profile of SLC7A5 is shown in FIG. 26.

### Group V genes

As shown in Table 5, Group V includes 37 genes having a variety of functions.

**Table 5. Group V genes**

| Gene symbol | Locus link | Nucleic acid seq. | Amino acid seq. |
|---|---|---|---|
| ASCL2 | 430 | SEQ ID NO:27 | SEQ ID NO:90. |
| DEFA6 | 1671 | SEQ ID NO:28 | SEQ ID NO:91 |
| FABP6 | 2172 | SEQ ID NO:29 | SEQ ID NO:92 |
| SCYA20 | 6364 | SEQ ID NO:30 | SEQ ID NO:93 |
| HHLA1 | 10086 | SEQ ID NO:31 | SEQ ID NO:94 |
| REG1A | 5967 | SEQ ID NO:32 | SEQ ID NO:95 |
| REG1B | 5968 | SEQ ID NO:33 | SEQ ID NO:96 |
| LCN2 | 3934 | SEQ ID NO:34 | SEQ ID NO:97 |
| GTF3A | 2971 | SEQ ID NO:35 | SEQ ID NO:98 |
| CENPF | 1063 | SEQ ID NO:36 | SEQ ID NO:99 |
| CSEIL | 1434 | SEQ ID NO:37 | SEQ ID NO:100 |
| C20orf1 | 22974 | SEQ ID NO:38 | SEQ ID NO:101 |
| E1AF | 2118 | SEQ ID NO:39 | SEQ ID NO:102 |
| MYBL2 | 4605 | SEQ ID NO:40 | SEQ ID NO:103 |
| PCNA | 5111 | SEQ ID NO:41 | SEQ ID NO:104 |
| EDN1 | 1906 | SEQ ID NO:42 | SEQ ID NO: 105 |
| KNSL2 | 3833 | SEQ ID NO:43 | SEQ ID NO:106 |
| SALL4 | 57167 | SEQ ID NO:44 | SEQ ID NO:107 |
| CLDN2 | 9075 | SEQ ID NO:45 | SEQ ID NO:108 |
| JPO1 | 83879 | SEQ ID NO:46 | SEQ ID NO:109 |
| CKAP2 | 26586 | SEQ ID NO:47 | SEQ ID NO:110 |
| D2152056E | 8568 | SEQ ID NO:48 | SEQ ID NO:111 |
| BM039 | 55839 | SEQ ID NO:49 | SEQ ID NO:112 |
| HIG2 | 29923 | SEQ ID NO:50 | SEQ ID NO:113 |
| ANLN | 54443 | SEQ ID NO:51 | SEQ ID NO:114 |
| ICBP90 | 29128 | SEQ ID NO:52 | SEQ ID NO:115 |
| AXIN2 | 8313 | SEQ ID NO:53 | SEQ ID NO:116 |
| HSPE1 | 3336 | SEQ ID NO:54 | SEQ ID NO:117 |
| NPM1 | 4869 | SEQ ID NO:55 | SEQ ID NO:118 |
| RFC3 | 5983 | SEQ ID NO:56 | SEQ ID NO:119 |
| HSPBP1 | 23640 | SEQ ID NO:57 | SEQ ID NO:120 |
| HCAP-G | 64151 | SEQ ID NO:58 | SEQ ID NO:121 |
| OS4 | 10106 | SEQ ID NO:59 | SEQ ID NO:122 |
| EFNA3 | 1944 | SEQ ID NO:60 | SEQ ID NO:123 |
| TGFBI | 7045 | SEQ ID NO:61 | SEQ ID NO:124 |
| GRB7 | 2886 | SEQ ID NO:62 | SEQ ID NO:125 |
| CDKN2A | 1029 | SEQ ID NO:63 | SEQ ID NO:126 |

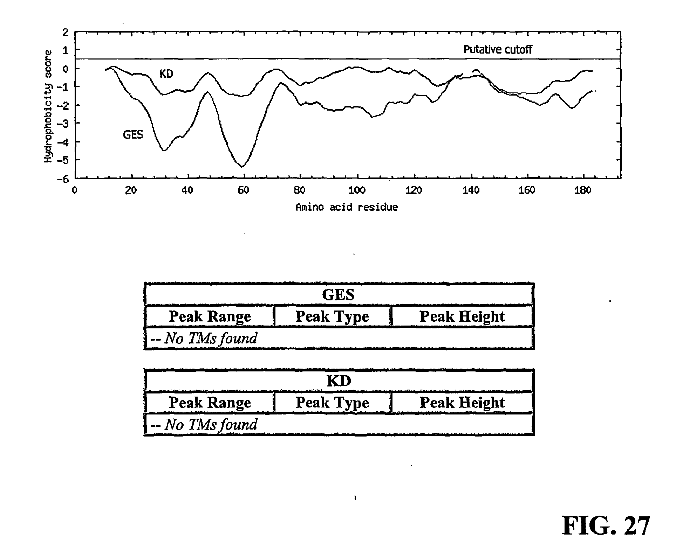

ASCL2 (achaete-scute complex) is a member of the basic helix-loop helix (BHLH) family of transcription factors. It activates transcription by binding to the E box (5'-CANNTG-3'). Dimerization with other BHLH proteins is required for efficient DNA binding. ASCL2 is involved in the determination of the neuronal precursors in the peripheral nervous system and the central nervous system. The hydrophobicity profile of ASCL2 is shown in FIG. 27.

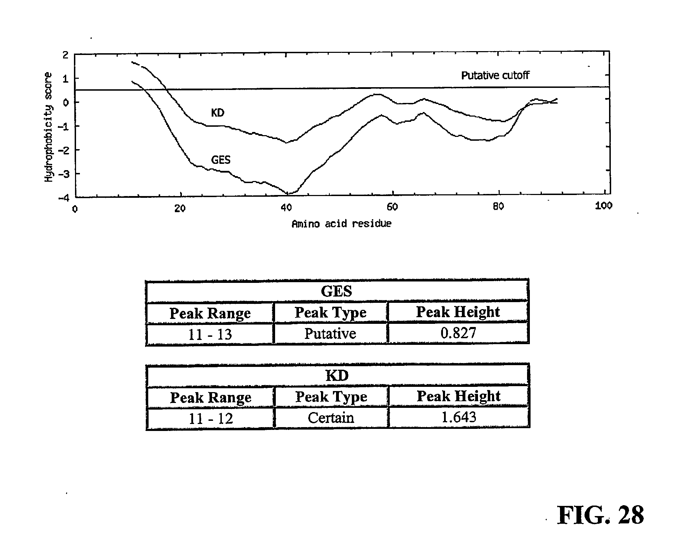

DEFA6 (defensin, alpha, 6) is a member of the defensin family. Defensins are microbicidal and cytotoxic peptides thought to be involved in host defense. Defensins are abundant in the granules of neutrophils and also found in the epithelia of mucosal surfaces, such as those of the intestine, respiratory tract, urinary tract, and vagina. Members of the defensin family are highly similar in protein sequence and distinguished by a conserved cysteine motif. Several alpha defensin genes appear to be clustered on chromosome 8. The protein encoded by this gene, defensin, alpha 6, is highly expressed in the secretory granules of Paneth cells of the small intestine, and likely plays a role in host defense of human bowel. The hydrophobicity profile of DEFA6 is shown in FIG. 28.

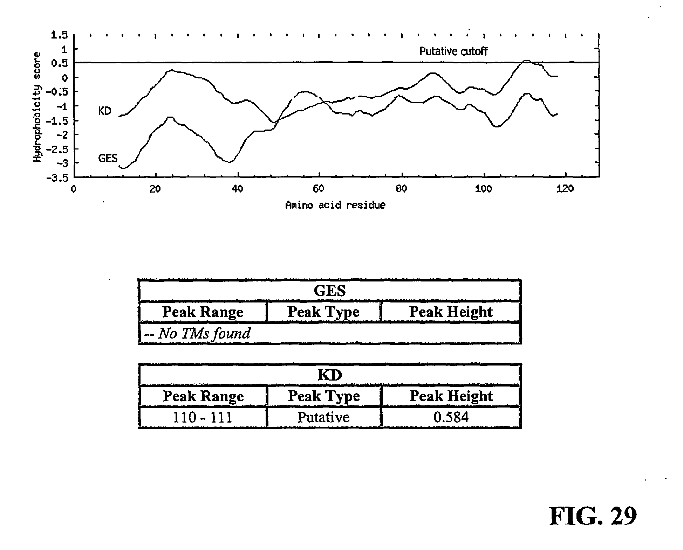

FABP6 (fatty acid binding protein 6) gene encodes a fatty acid binding protein. Fatty acid binding proteins are a family of small, highly conserved, cytoplasmic proteins that bind long-chain fatty acids and other hydrophobic ligands. FABP6 and FABP1 (the liver fatty acid binding protein) are also able to bind bile acids. It is thought that FABPs roles include fatty acid uptake, transport, and metabolism. The hydrophobicity profile of FABP6 is shown in FIG. 29.

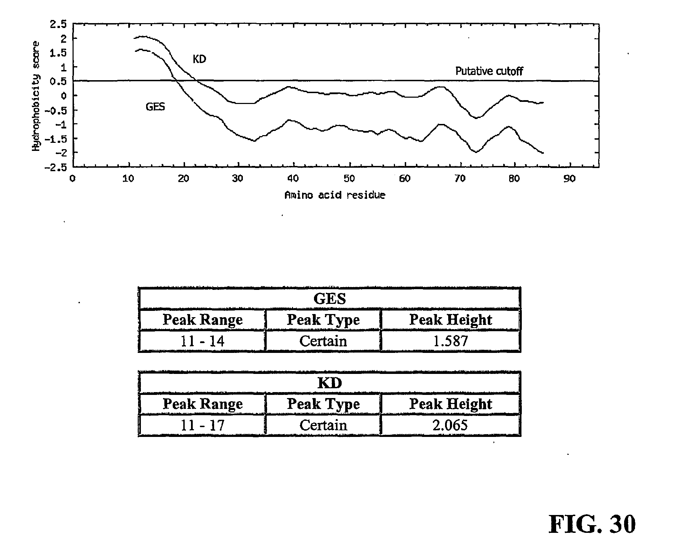

SCYA20 (small inducible cytokine subfamily A) is a chemotactic factor for lymphocytes, but not a chemotactic factor for monocytes. Chemokines are basic, heparin-binding proteins of 8 to 12 kd that have 4 cysteines conserved among all family members. SCYA20 is expressed preferentially in lymphocytes and monocytes, and its expression is markedly upregulated by mediators of inflammation such as TNF and LPS. The hydrophobicity profile of SCYA20 is shown in FIG. 30.

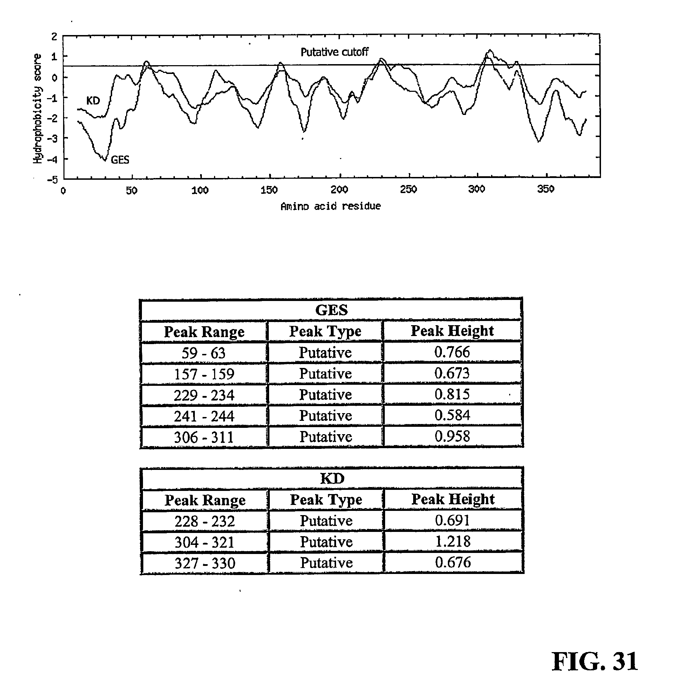

HHLA1 encodes HERV-H LTR-associating 1, a member of the HERV-H family. HHLA1 has been shown to play a role in the expression of a variety of adjacent genes. The hydrophobicity profile of HHLA1 is shown in FIG. 31.

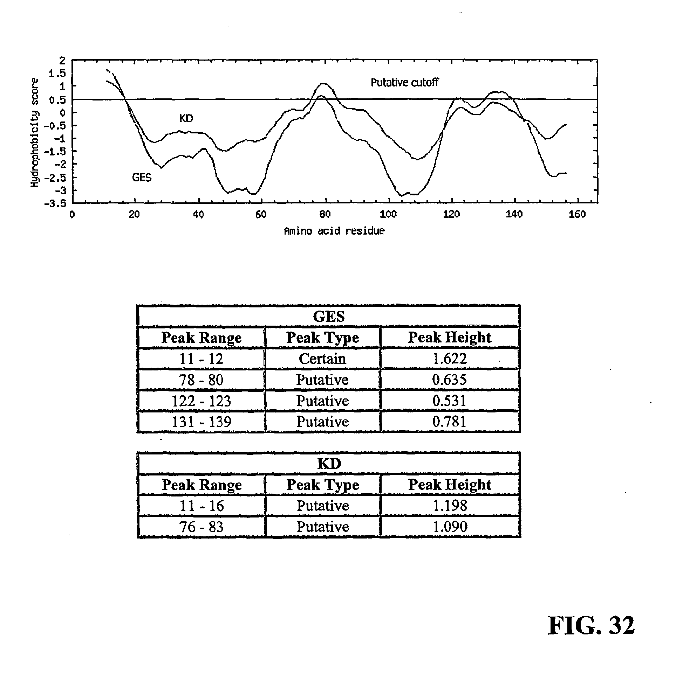

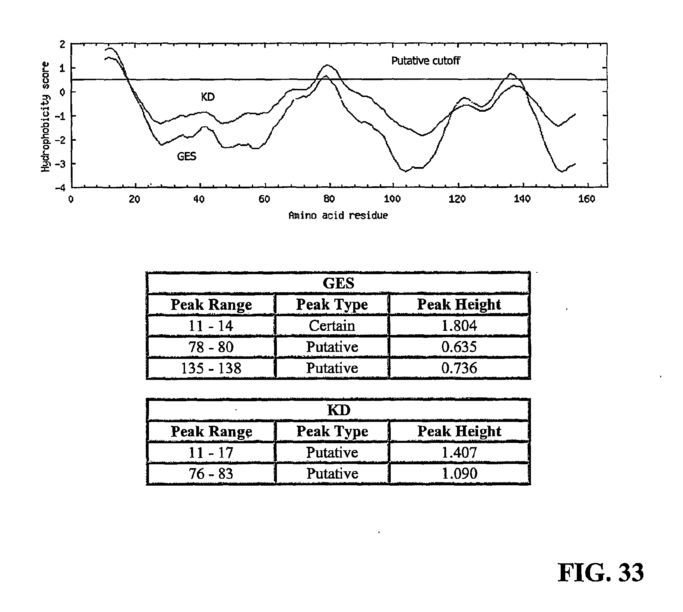

REG1A (regenerating islet-derived. 1 alpha) and REG1B (regenerating islet-derived 1 beta) genes belong to the type I subclass of the REG family of genes, each of which encodes a 166 amino acid protein. Mice transgenic for REG gene (REG-Tg mice) developed diabetes by apoptosis of beta-cells, as well as various malignant tumors. In addition to the decrease in beta-cells, compensatory islet regeneration and proliferation of ductal epithelial cells were observed in REG-Tg mice. Because REG1 protein was secreted primarily into pancreatic ducts from acinar cells, it may primarily stimulate the proliferation of ductal epithelial cells, and not beta-cells, and their differentiation into islets. The hydrophobicity profile of REG1A and REG1B are shown in FIGs. 32 and 33, respectively.

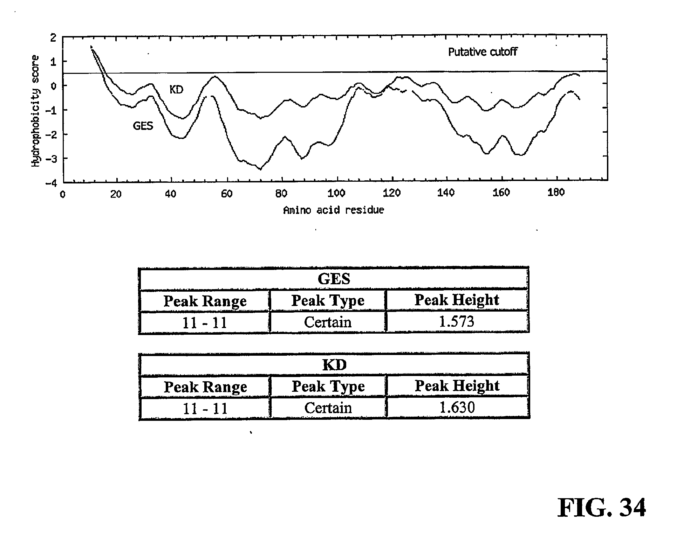

LCN2 encodes Lipocalin 2. Lipocalin 2, also known as NGAL, is a protein associated with neutrophil gelatinase. The 25kD LCN2 protein is believed to bind small lipophilic substances such as bacteria-derived lipopolysaccharide (LPS) and formylpeptides and may function as a modulator of inflammation. It was reported that the LCN2 gene undergoing maximal transcriptional induction in IL3-dependent murine FL5.12 pro-B cells after cytokine withdrawal. Conditioned medium from IL3-deprived cells contained Lipocalin 2 and induced apoptosis in naive cells, even when IL3 was present. Lipocalin 2 also induced apoptosis in a wide variety of leukocytes but not other cell types. The hydrophobicity profile of Lipocalin 2 is shown in FIG. 34.

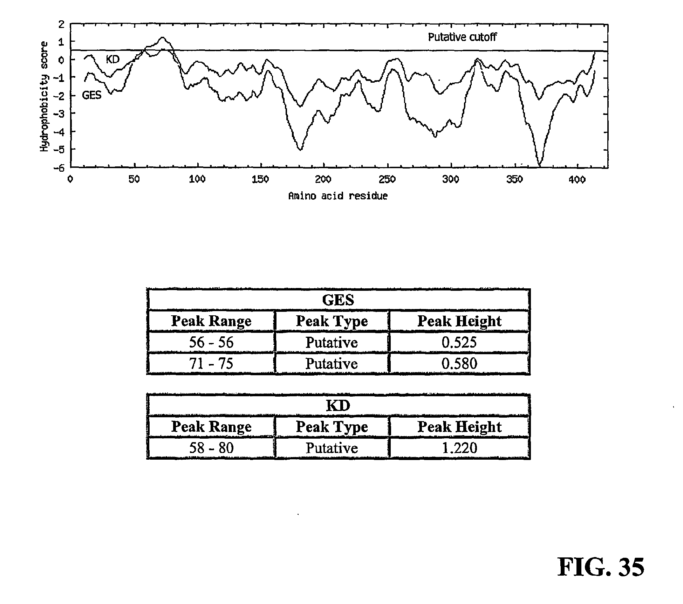

GTF3A (general transcription factor IIIA) is required for RNA polymerase III-mediated transcription of the 5S RNA genes and binds to both DNA and RNA. The hydrophobicity profile of GTF3A is shown in FIG. 35.

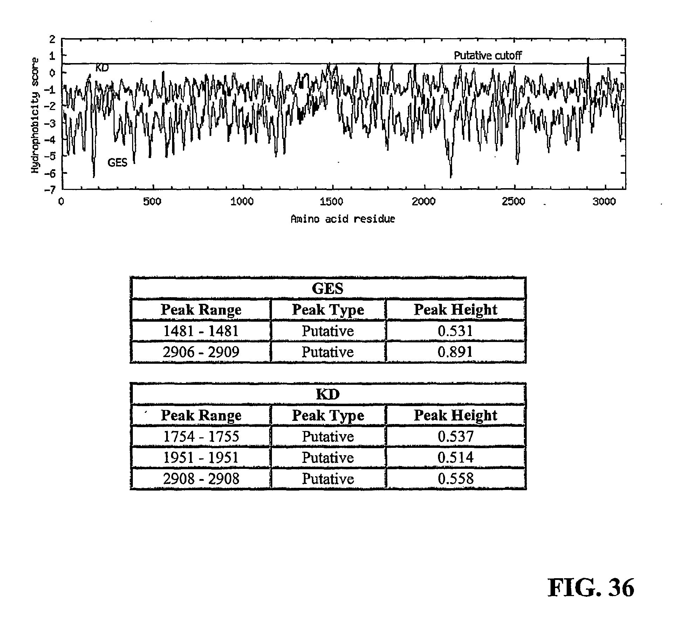

CENPF (centromere protein F, 350/400kD) CENPF is a protein that associates with the centromere-kinetochore complex. The protein is a component of the nuclear matrix during the G2 phase of interphase. In late G2 the protein associates with the kinetochore and maintains this association through early anaphase. It localizes to the spindle midzone and the intracellular bridge in late anaphase and telophase, respectively, and is thought to be subsequently degraded. The localization of this protein suggests that it may play a role in chromosome segregation during mitosis. It is thought to form either a homodimer or heterodimer. Autoantibodies against this protein have been found in patients with cancer or graft versus host disease. The hydrophobicity profile of CENPF is shown in FIG. 36.

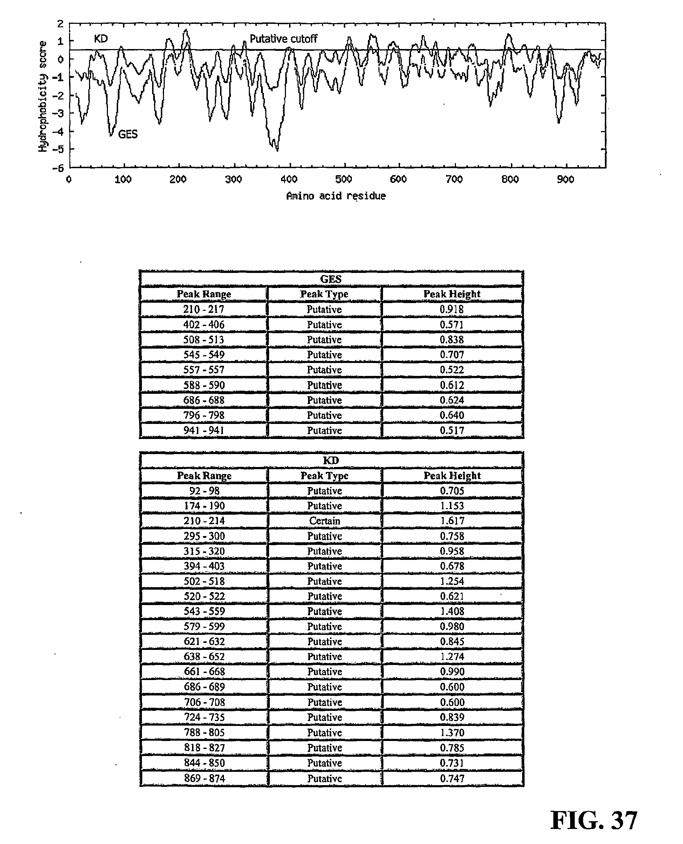

CSE1L (chromosome segregation 1 (yeast homology)-like protein) is a cellular apoptosis susceptibility (CAS) protein. CSE1L exports importin alpha from nucleus to cytoplasm. CAS1L maps to 20q13. This region harbors amplifications that correlate with aggressive breast cancer. Elevated copy numbers of CAS are seen in a leukemia cell line, three of four colon cell lines, and three of seven breast cancer cell lines. The hydrophobicity profile of CSE1L is shown in FIG. 37.

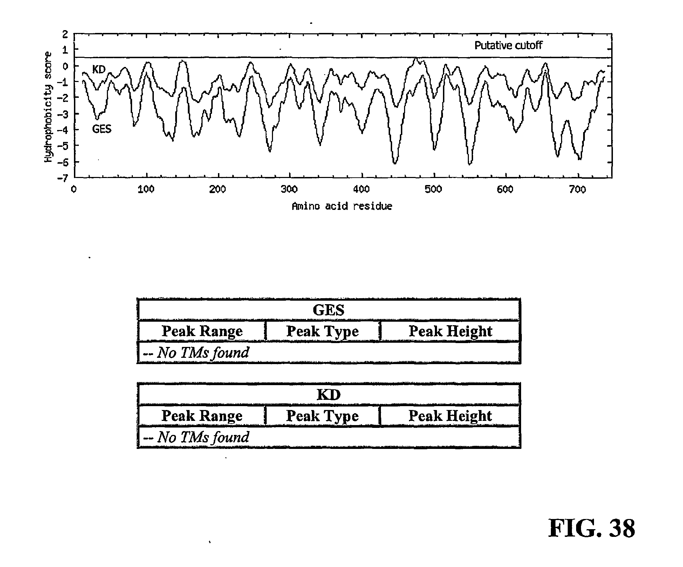

C20orf1 (chromosome 20 open reading frame 1) is a proliferation-associated nuclear protein. C20orf1 associates with the spindle pole and mitotic spindle during mitosis. The full-length C20orf1 cDNA encodes a 747 amino acid protein with a putative ATP/GTP binding site motif. RT PCR analysis demonstrated strong expression of C20orf1 in lung carcinoma cell lines. The hydrophobicity profile of C20orf1 is shown in FIG. 38.

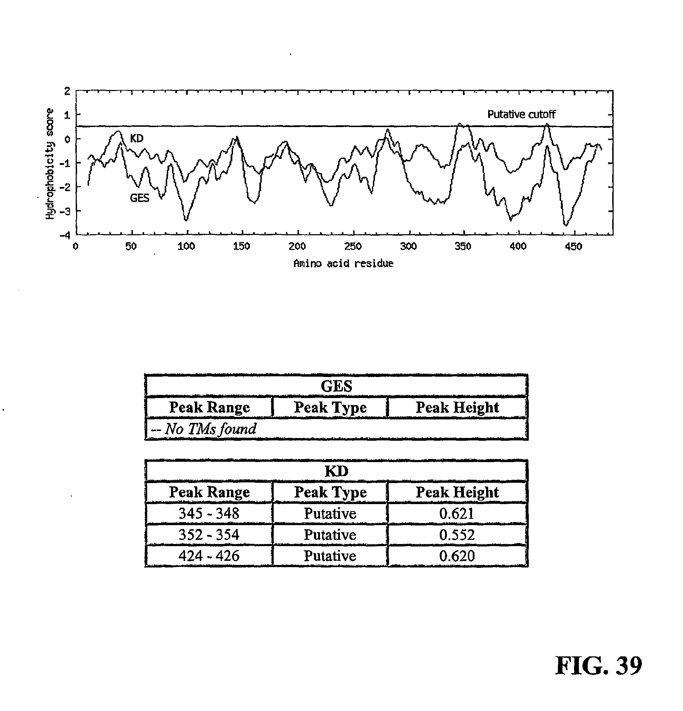

ElAF (Ets variant gene 4) is a 462 amino acid protein that shows sequence similarity within the ETS domain, a region required for sequence-specific DNA binding by members of the ets oncogene family. E1AF is about 94% identical to the mouse PEA3 protein (polyomavirus enhancer activator-3). Northern blot analysis detected a 2.5kb mRNA in HeLa cells whose levels increased during the early phase of adenovirus infection. Subsequently, studies show that E1AF can activate the promoters of various MMPs, whose expression is associated with tumor cell invasion and metastasis, by 10 to 20 fold. The hydrophobicity profile of E1AF is shown in FIG. 39.

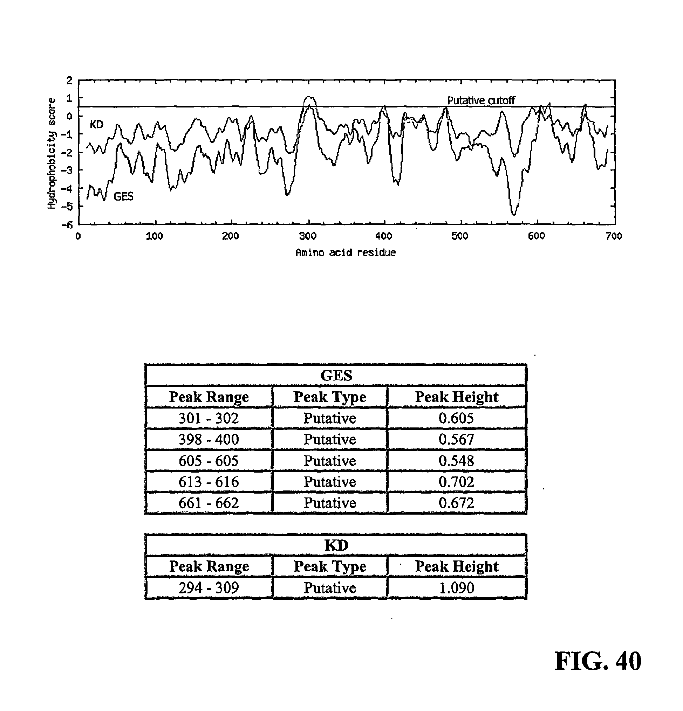

MYBL2 (v-myb avian myeloblastosis viral oncogene homolog-like 2) is a transcription factor and a member of the oncoprotein myb family. MYBL2 may have a role in cell cycle progression. MYBL2 transcripts are detectable in a wide variety of dividing cell types. MYBL2 activates CDC2 and cyclin D1 gene expression in proliferating fibroblasts, and antisense oligonucleotides specific to MYBL2 inhibit proliferation of human hematopoietic cell lines. MYBL2 expression is also regulated at the G1/S phase transition, and its transcription relies on E2F activity in a cell cycle-dependent manner. Thus, unlike MYB and MYBL1, whose transcriptional activity is mainly restricted to hematopoietic, spermatogenic, and neural progenitor cells, MYBL2 appears to possess a broader function during cell proliferation. The hydrophobicity profile of MYBL2 is shown in FIG. 40.

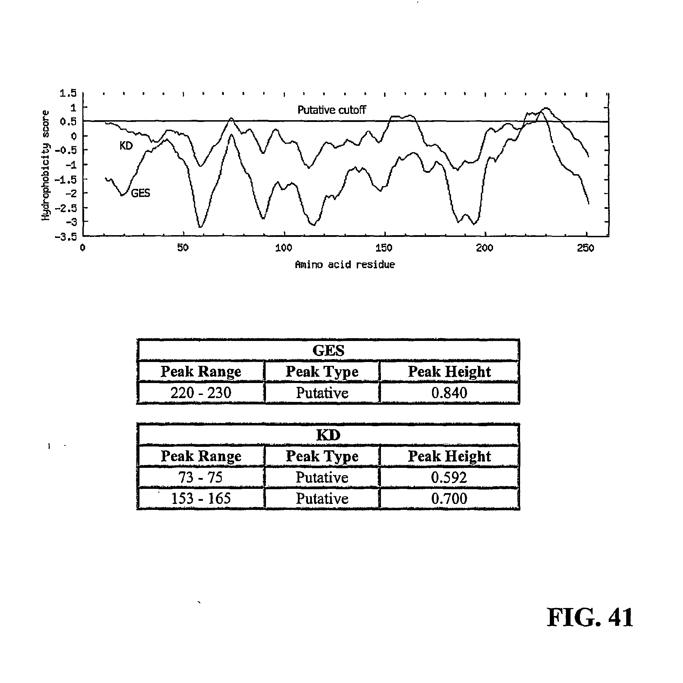

PCNA (proliferating cell nuclear antigen) is a processivity factor for DNA polymerases delta and epsilon. The hydrophobicity profile of PCNA is shown in FIG. 41.

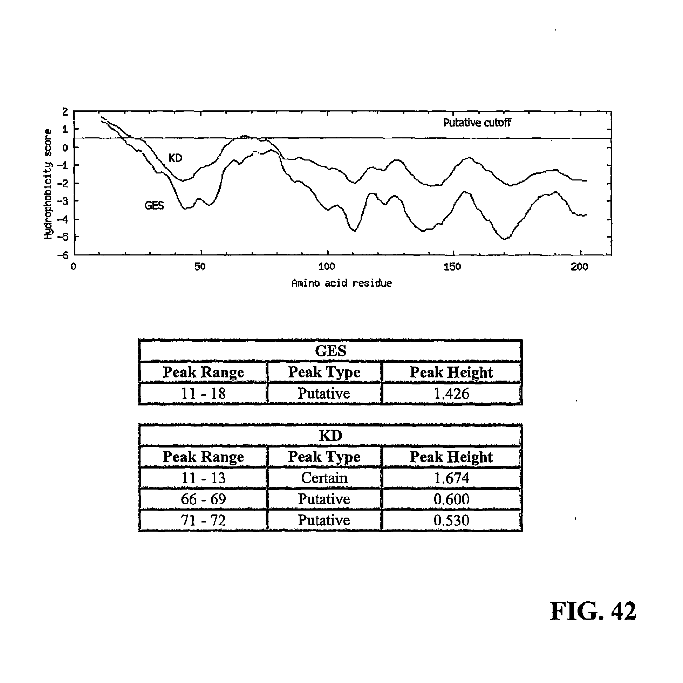

EDN1 (Endothelin 1) is a potent, 21-amino acid vasoconstrictor peptide produced by vascular endothelial cells. In addition to its vasoconstrictor action, endothelin has effects on the central nervous system and on neuronal excitability. Mice homozygous for a knockout of the EDN11 gene die of respiratory failure at birth and show morphologic abnormalities of the pharyngeal-arch-derived craniofacial tissues and organs. Heterozygous mice produce lower levels of EDN1 than wild-type mice and develop elevated blood pressure. EDN1 is present at high concentrations in ovarian cancer ascites and is over-expressed in primary and metastatic ovarian carcinoma. In these cells, EDN1 acts as an autocrine mitogenic and angiogenic factor selectively through the EDN1 receptor. Findings demonstrate that EDN1 promotes ovarian carcinoma cell invasion, acting through the EDN1 receptor by up-regulating secretion and activation of multiple tumor proteinases (MMP2, 3, 7, 9, and 13). Therefore, EDN1 may represent a key component of more aggressive ligand-induced invasiveness of ovarian carcinoma. The hydrophobicity profile of EDN1 is shown in FIG. 42.

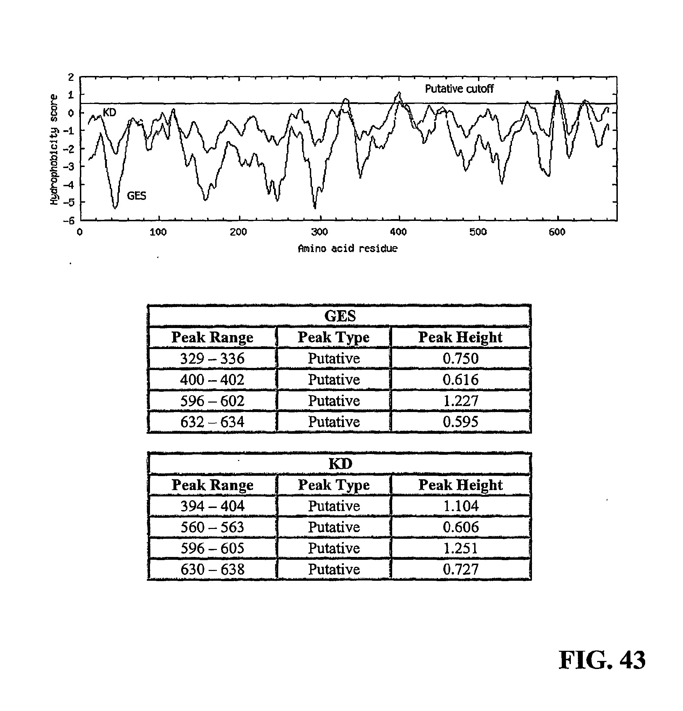

KNSL2 (Kinesin-like 2 protein) mRNA is expressed in several human cell lines. The C-terminal 350 amino acids of the predicted KNSL2 protein share extensive homology with the ATP-binding and motor domains of kinesin heavy chain and the kinesin-related proteins CENPE and MKLP1. Although the mechanochemical domain of kinesin and kinesin-like proteins is generally located within the N-terminal region, KNSL2 contains a C-terminal mechanochemical domain. This 'reversed' structural organization is also found in the *S. cerevisiae* KAR3 and *Drosophila* Ncd kinesin-like proteins. Molecular motors move directionally to either the plus or the minus ends of microtubules or actin filaments. For example, kinesin moves towards the plus end, whereas the Drosophila Ned motor moves towards the minus end. The hydrophobicity profile of KNSL2 is shown in FIG. 43.

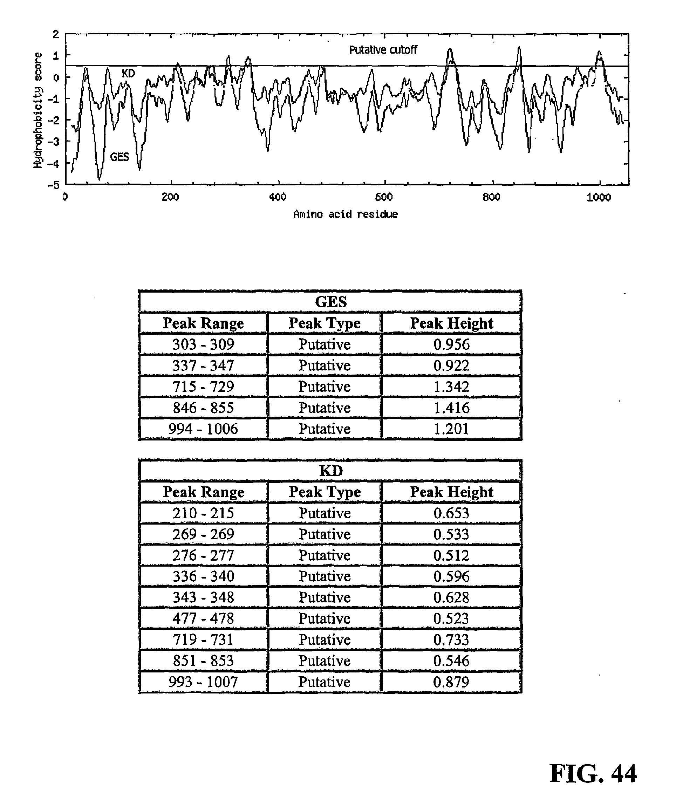

SALL4 (sal-like 4 (*Drosophila*)) encodes a protein that is similar to SALL1. SALL1 is a mammalian homolog of the *Drosophila* region-specific homeotic gene spalt (sal); heterozygous mutations in SALL1 in humans lead to Townes-Brocks syndrome. The mouse homolog of SALL1 (Sall1) has been isolated and mice deficient in Sall1 die in the perinatal period and kidney agenesis or severe dysgenesis are present. Salll is expressed in the metanephric mesenchyme surrounding ureteric bud. Homozygous deletion of Sall1 results in an incomplete ureteric bud outgrowth, a failure of tubule formation in the mesenchyme and an apoptosis of the mesenchyme. This phenotype is likely to be primarily caused by the absence of the inductive signal from the ureter, as the Salll-deficient mesenchyme is competent with respect to epithelial differentiation. Sa111 is therefore essential for ureteric bud invasion, the initial key step for metanephros development. The hydrophobicity profile of SALL4 is shown in FIG. 44.

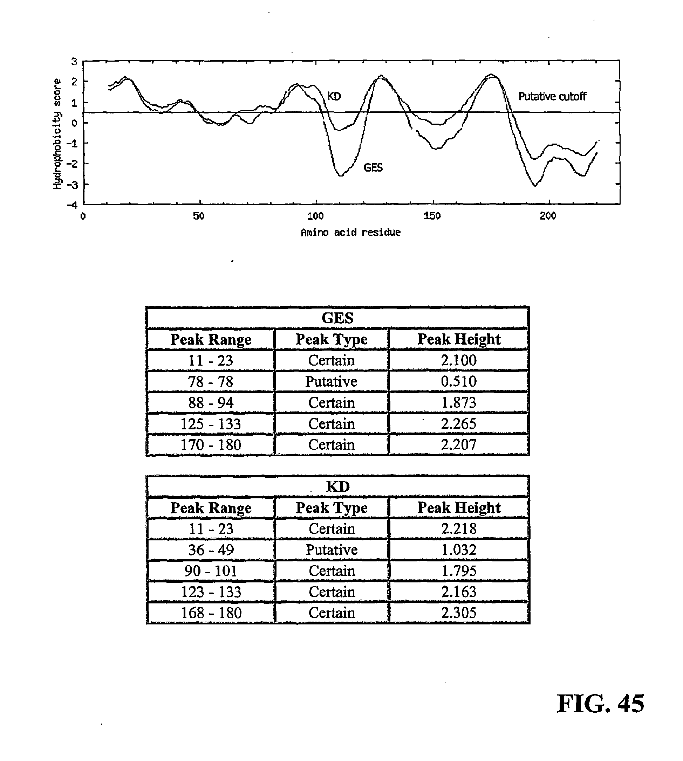

CLDN2 (Claudin 2) is a member of the claudin family. The claudins are transmembrane proteins that are part of the tight junction complex and likely form structural components of a paracellular pore. Claudin-2 mRNA and protein are found throughout the proximal tubule and in the contiguous early segment of the thin descending limb of long-looped nephrons. The level of expression reveals an axial increase from proximal to distal segments. In confocal images, the subcellular localization of claudin-2 protein coincide with that of the tight junction protein ZO-1. Claudin-2 is a component of the paracellular pathway of the most proximal segments of the nephron and may be responsible for their uniquely leaky permeability properties. The hydrophobicity profile of CLDN2 is shown in FIG. 45.

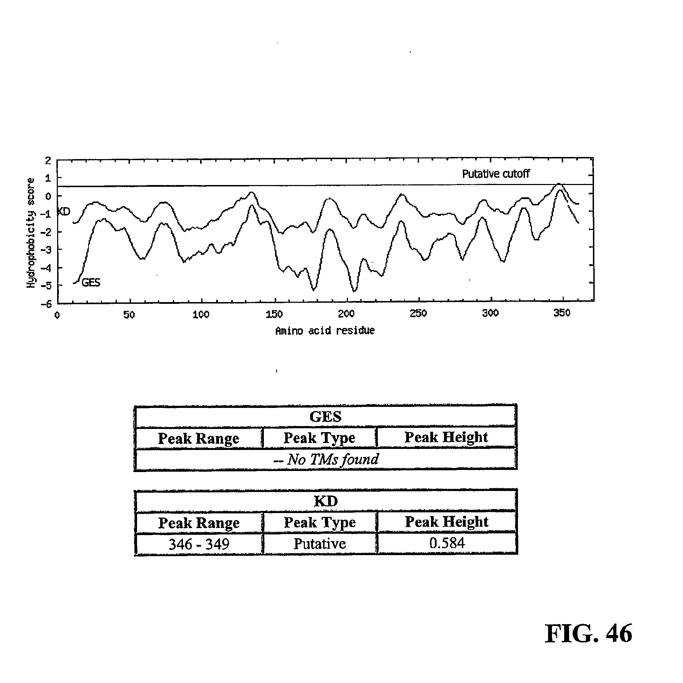

JPO1 (c-Myc target) gene encodes a 47 kDa nuclear protein. JPO1 mRNA expression is readily detectable in the thymus, small intestine and colon, whereas expression is relatively low in spleen, bone marrow, and peripheral leukocytes. Stable Rat1a fibroblasts over-expressing JPO1 were tested and compared to transformed Ratla-Myc cells. Although JPO1 has a diminished transforming activity as compared with c-Myc, JPO1 complements a transformation-defective Myc Box II mutant in the Ratla transformation assay. This complementation provides evidence for a genetic link between c-Myc and JPO1. Similar to c-Myc, JPO1 over-expression enhances the clonogenicity of CB33 human lymphoblastoid cells in methylcellulose assays. These observations suggest that JPO1 participates in c-Myc mediated transformation, supporting an emerging concept that c-Myc target genes constitute nodal points in a network of pathways that lead from c-Myc to various Myc-related phenotypes and ultimately to tumorigenesis. The hydrophobicity profile of JPO1 is shown in FIG. 46.

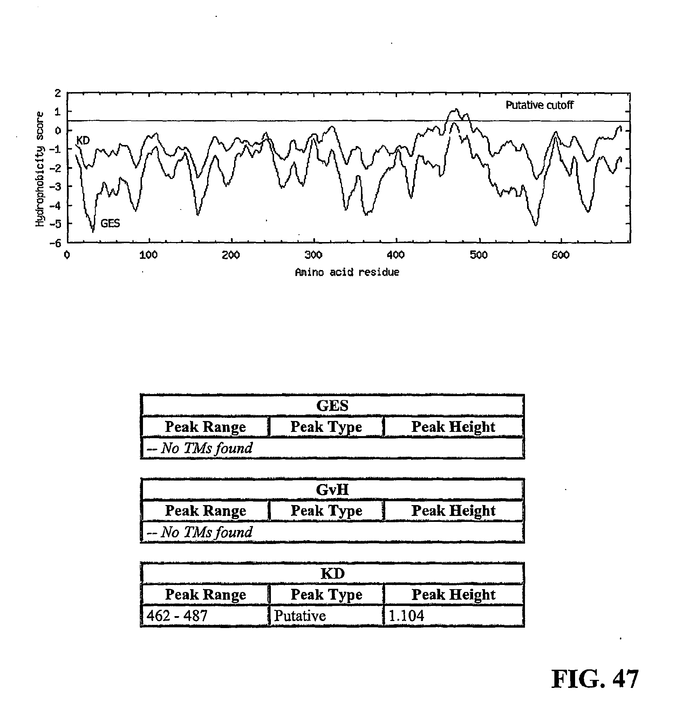

CKAP2 is a cytoskeleton associated protein. The hydrophobicity profile of CKAP2 is shown in FIG. 47.

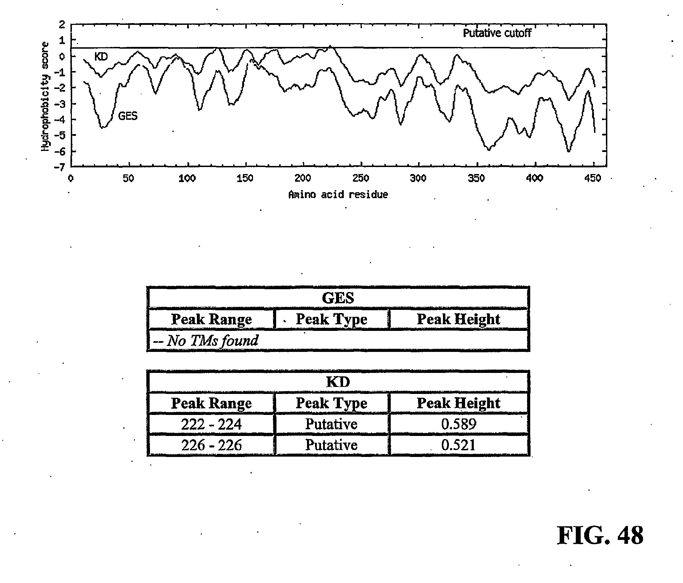

D21S2056E (DNA segment on chromosome 21 (unique) 2056 expressed sequence) encodes a nuclear protein with a ubiquitous tissue expression. The hydrophobicity profile of D2152056E is shown in FIG. 48.

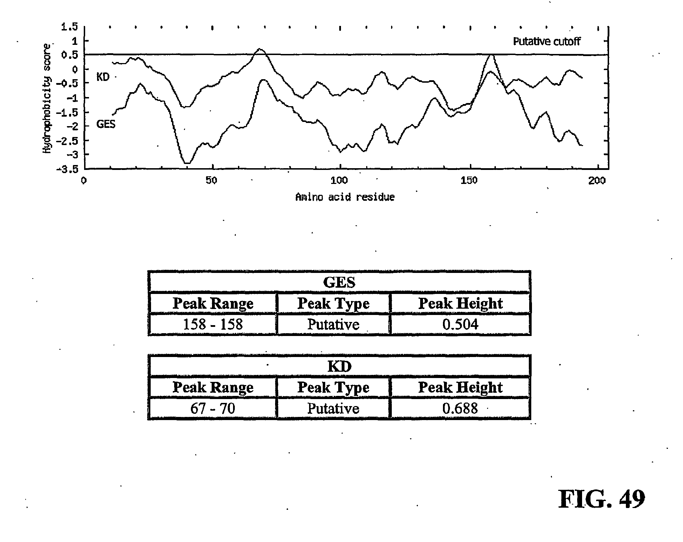

BM039 encodes an uncharacterized bone marrow protein BM039. The hydrophobicity profile of BM309 is shown in FIG. 49.

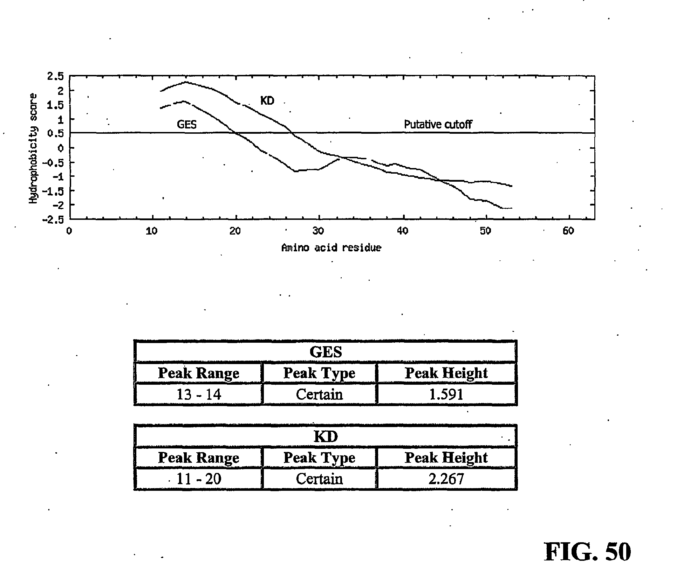

HIG2 encodes hypoxia-inducible protein 2. Evidence is accumulating that the adverse tumor microenvironment both modifies the malignant progression of tumor cells and contributes to chemotherapy and radiation resistance. One hypothesis is that some of the effects on malignant progression are mediated through the transcriptional regulation of genes responsive to the stresses of the microenvironment, such as low oxygen or low glucose conditions. To determine epigenetic changes in gene expression that were consistent with that hypothesis, RDA was used to identify hypoxia-induced cDNAs from cultured human cervical epithelial cells. Twelve induced genes were identified: two novel genes (HIG1 and HIG2), three genes known to be hypoxia-inducible (tissue factor, GAPDH, thioredoxin), and seven genes not previously identified as hypoxia-inducible (HNRNP(a1), ribosomal L7, annexin V, lipocortin2, Ku(70), PRPP synthase, and acetoacetyl-CoA thiolase). In cultured cells, HIG1 and HIG2 expression is induced by hypoxia and by glucose deprivation, but their expression is not induced by serum deprivation, UV, or ionizing radiation. The putative HIG1 and HIG2 open reading frames are expressed in cells, as confirmed by epitope tagging. In addition, tumor xenografts derived from human cervical cancer cells display increased expression of HIG1 and HIG2 when they are deprived of oxygen. Taken together, these data suggest a coordinated transcriptional response of eukaryotic cells to microenvironmental stresses found in the solid tumor. The hydrophobicity profile of HIG2 is shown in FIG. 50.

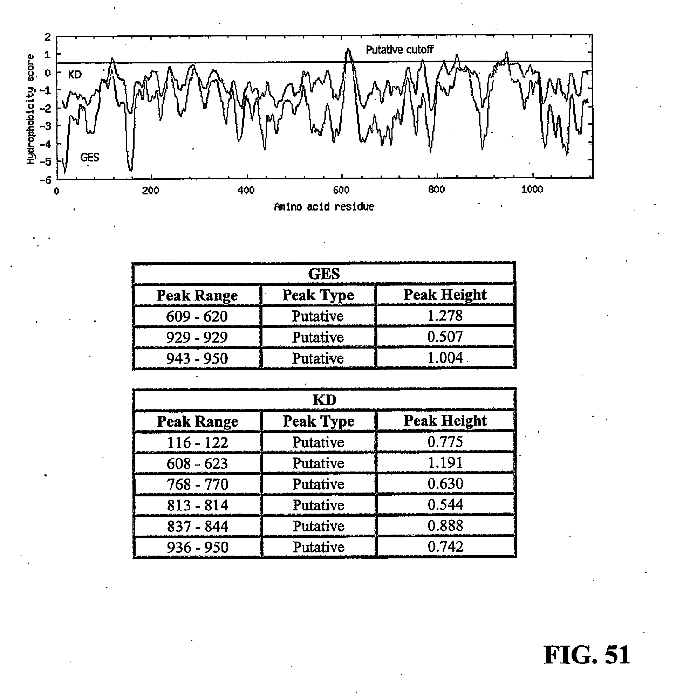

ANLN (anillin (Drosophila Scraps homolog) or actin binding protein) interacts with cleavage furrow proteins, such as septins, and may play a role in cytokinesis. Like Drosophila anillin, the human protein localizes to the nucleus during interphase, the cortex following nuclear envelope breakdown, and the cleavage furrow during cytokinesis. Anillin also localizes to ectopic cleavage furrows generated between two spindles in fused PtK(1) cells. Microinjection of anti-anillin antibodies slows cleavage, leading to furrow regression and the generation of multinucleate cells. GFP fusions that contain the COOH-terminal 197 amino acids of anillin, which includes a pleckstrin homology (PH) domain, form ectopic cortical foci during interphase. The septin Hcdc10 localizes to these ectopic foci, whereas myosin II and actin do not, suggesting that anillin interacts with the septins at the cortex. Robust cleavage furrow localization requires both the COOH-terminal domain of anullin and additional NH(2)-terminal sequences corresponding to an actin binding domain defined by *in vitro* cosedimentation assays. Endogenous anillin and Hcdc10 colocalize to punctate foci associated with actin cables throughout mitosis, and the accumulation of both proteins at the cell equator requires filamentous actin. These results indicate that anillin is a conserved cleavage furrow component important for cytokinesis. Interactions with at least two other furrow proteins, actin and the septins, likely contribute to anillin function. The hydrophobicity profile of ANLN is shown in FIG. 51.

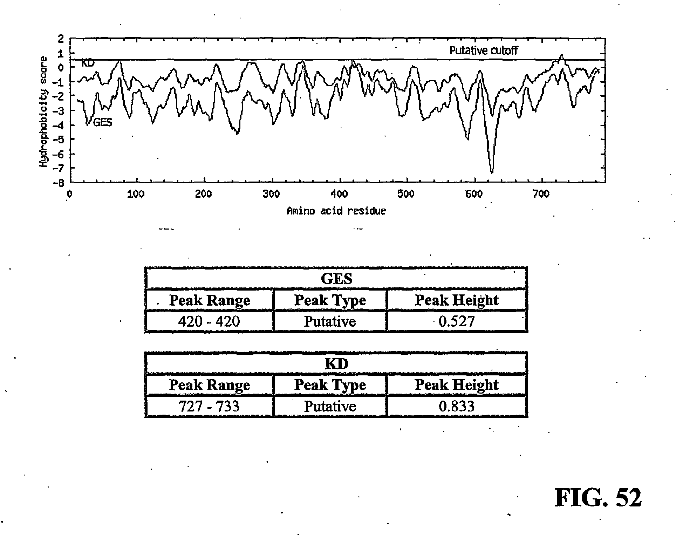

ICBP90 encodes a transcription factor, a DNA-binding protein that may regulate expression of topoisomerase 2 alpha. ICBP90 may also be useful as a new proliferation marker for cancer tissues. The hydrophobicity profile of ICBP90 is shown in FIG. 52.

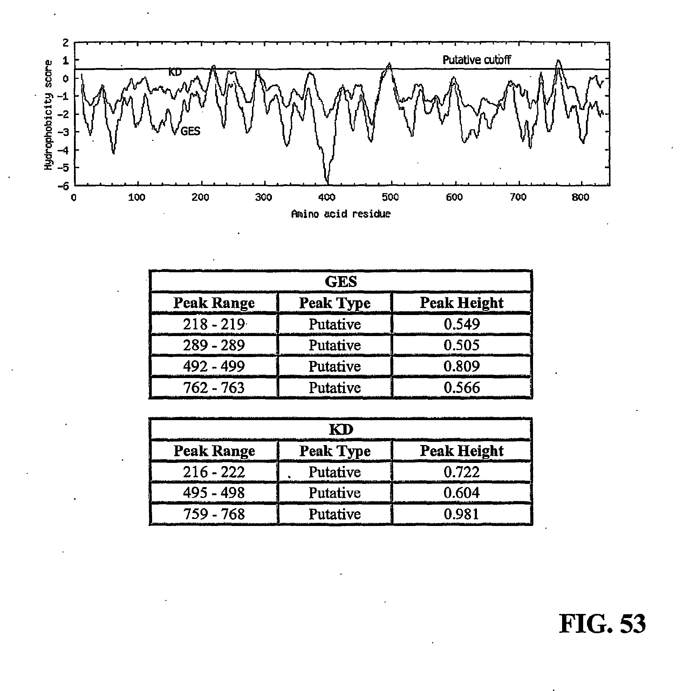

AXIN2 (conductin, axin-related protein) presumably plays a role in the regulation of the stability of beta-catenin in the Wnt signaling pathway. In mouse, conductin organizes a multiprotein complex of adenomatous polyposis of the colon, beta-catenin, glycogen synthase kinase 3-beta, and conductin, which leads to the degradation of beta-catenin. The deregulation of beta-catenin is an important event in the genesis of a number of malignancies. The AXIN2 gene has been mapped to 17q23-q24, a region that shows frequent loss of heterozygosity in breast cancer, neuroblastoma, and other tumors. The hydrophobicity profile of AXIN2 is shown in FIG. 53.

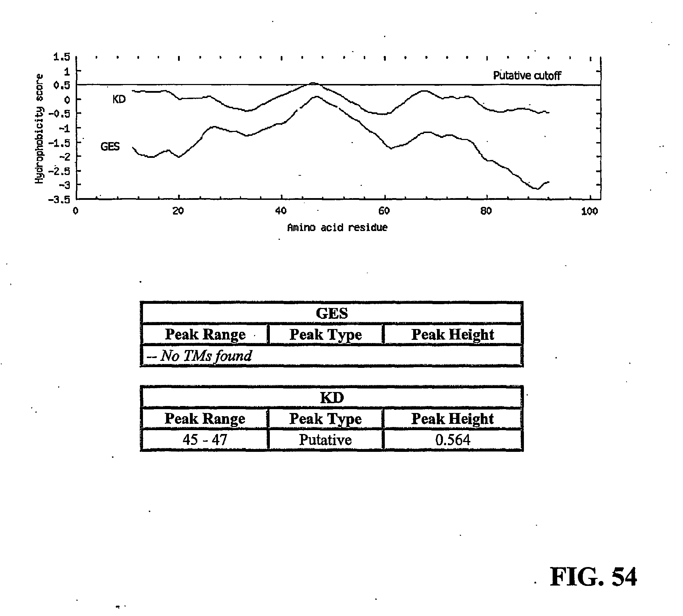

HSPE7 encodes heat shock 10kD protein 1, which is also referred to as GroES or chaperonin 10. Chaperonins are ubiquitous, indispensable proteins that facilitate protein folding in an ATP dependent manner to enhance the yield of properly folded substrate protein under conditions where spontaneous folding does not occur. Chaperonins are typified by the *E. coli* heat-shock proteins GroEL (cpn60) and GroES (cpn10). GroES is a heptameric ring of identical 10.4kD subunits that binds to each end of GroEL to form a symmetric, functional heterodimer. The hydrophobicity profile of HSPE7 is shown in FIG. 54.

NPM1 (nucleophosmin, nucleolar phosphoprotein B23 or numatrin) is a nucleolar phosphoprotein that is more abundant in tumor cells than in normal resting cells. Stimulation of the growth of normal cells, e.g., mitogen activation of B lymphocytes, is accompanied by an increase in NPM1 protein level. Although the function of NPM1 has not been defined precisely, ample evidence suggests that it is involved in the assembly of ribosomal proteins into ribosomes. Electron microscopic study indicates that NPM1 is concentrated in the granular region of the nucleolus, where ribosome assembly occurs. The cDNA has a coding sequence equivalent to a protein of 294 amino acids. When protein levels were compared with Western blot immunoassays, Novikoff hepatoma showed 20 times more NPM1 than normal, and arid hypertrophic rat liver showed about 5 times more NPM1 than unstimulated normal liver. The hydrophobicity profile of NPM1 is shown in FIG. 55.

RFC3 (replication factor C, activator 1) is a multimeric primer-recognition protein consisting of 5 distinct subunits of 145, 40, 38, 37, and 36.5 kD. Human RFC3 is essential for the *in vitro* replication of simian virus 40 (SV40) DNA. RFC3, in the presence of ATP, assembles PCNA and DNA polymerase-delta or polymerase-epsilon on primed DNA templates. The complex of primed DNA-RFC-PCNA-DNA polymerase, when supplemented with dNTPs, results in the efficient elongation of DNA in the presence of human single-stranded DNA binding protein. Studies with the complete 5-subunit holoenzyme indicated that the large subunit binds to DNA and the 40-kD subunit binds ATP. The other subunits may play discrete roles in the elongation process catalyzed by polymerase. The subunit genes are numbered in sequence of decreasing molecular weight: RFC1, RFC2, RFC3, RFC4, and RFC5. The RFC3 gene maps to 13q12.3-q13. The hydrophobicity profile of RFC3 is shown in FIG. 56.

HSPBP7 (Hsp70-Interacting protein) binds HSP70 and HSC70 (HSPAB) and inhibits chaperone activity by preventing ATP binding. The hydrophobicity profile of HSPBP7 is shown in FIG. 57.

HCAP-G (chromosome condensation protein G) is a component of 13S condensin, which is a five subunit protein complex that plays a central role in mitotic chromosome condensation. The condensin complex has an ATP-dependent positive supercoiling activity *in vitro.* The human 13S complex has exactly the same composition as its *Xenopus* counterpart, being composed of two structural maintenance of chromosomes (human chromosome-associated polypeptide (hCAP)-C and hCAP E) subunits and three non-structural maintenance of chromosomes (hCAP-D2/CNAP1, hCAP-G, and hCAP-H/BRRN) subunits. Human condensin purified from asynchronous HeLa cell cultures fails to reconfigure DNA structure *in vitro.* When phosphorylated by purified CDC2-cyclin B, however, it gains the ability to introduce positive supercoils into DNA in the presence of ATP and topoisomerase I. Strikingly, human condensin can induce chromosome condensation when added back into a *Xenopus* egg extract that has been immunodepleted of endogenous condensin. Thus, the structure and function of the condensin complex are highly conserved between *Xenopus* and humans, underscoring its importance in mitotic chromosome dynamics in eukaryotic cells. The hydrophobicity profile of HCAP-G is shown in FIG. 58.

OS4 (conserved gene amplified in osteosarcoma) gene consists of 4833 base pairs (bp) encoding an open reading frame (ORF) of 283 amino acids. The ORF predicts a water-soluble acidic (pl 5.50) polypeptide with a molecular weight of 32kD. Database searches reveal highly significant similarity between OS4 and eight proteins predicted from genomic sequences of *C. elegans, S. pombe,* and *S. cerevisiae.* Thus, OS4 defines a novel evolutionarily conserved gene superfamily. Northern and database analyses revealed OS4 transcripts in numerous human tissues demonstrating its ubiquitous expression. Over-expression of OS4 is seen in three cancer cell lines with amplification of this gene. OS4 is amplified in primary sarcomas with known amplification of the closely linked marker CDK4. These results demonstrate that the highly conserved OS4 gene is frequently included in the 12q13-q15 amplicon and may contribute to the development of a subset of sarcomas. The hydrophobicity profile of OS4 is shown in FIG. 59.

EFNA3 (ephrin A3) is a member of ephrins family. Ephrins bind to members of the EPH group of receptor tyrosine kinases. The various ephrins are characterized by sequence similarities and the fact that they are attached to the cell membrane by glycosylphosphatidylinositol (GPI) anchors or by a single transmembrane domain. The hydrophobicity profile of EFNA3 is shown in FIG. 60.

TGFBI encodes a 68kD, beta-induced transforming growth factor. The hydrophobicity profile of TGFBI is shown in FIG. 61.

GRB7 (growth factor receptor-bound protein 7) and its splice variant GRB7V were cloned from an invasive human esophageal carcinoma. Although both GRB7 isoforms share homology with the Mig10 cell migration gene of *C. elegans,* the GRB7V isoform lack 88 basepairs in the C terminus. The resultant frameshift led to substitution of an SH2 domain with a short hydrophobic sequence. The wild-type GRB7 protein, but not the GRB7V isoform, is rapidly tyrosyl phosphorylated in response to EGF stimulation in esophageal carcinoma cells. Analysis of human esophageal tumor tissues and regional lymph nodes with metastases revealed that GRB7V was expressed in 40% of GRB7-positive esophageal carcinomas. GRB7V expression was enhanced after metastatic spread to lymph nodes as compared to the original tumor tissues. Transfection of an antisense GRB7 RNA expression construct lowered endogenous GRB7 protein levels and suppressed the invasive phenotype exhibited by esophageal carcinoma cells. These findings suggested that GRB7 isoforms are involved in cell invasion and metastatic progression of human esophageal carcinomas. The hydrophobicity profile of GRB7 is shown in FIG. 62.

CDKN2A (cyclin-dependent kinase inhibitor 2A) inhibits CDK-cyclin complexes and is involved in G1 phase checkpoint arrest. The hydrophobicity profile of CDKN2A is shown in FIG. 63.

### CCGs and CCG Products as Therapeutic Targets

CCGs, such as those listed in Tables 1-5, are expressed at abnormally levels in colon cancer tissues. These genes may be a component in the disease mechanism and therefore, can be used as markers for diagnosing and monitoring colon cancer. Furthermore, CCGs, as well as their encoded polynucleotides (CCPNs) or polypeptides (CCPPs), can be used as therapeutic targets for the treatment and prevention of colon cancer. For instance, CCGs can encode kinases, phosphatases, G-protein coupled receptors, ion channels, proteases, metabolic enzymes, or transcription factors. These proteins and genes are known targets for drug action and development.

As an example, protein kinases regulate many cellular functions, such as cell proliferation/differentiation, and signal transduction, by adding phosphate groups to proteins. Uncontrolled signaling has been implicated in a variety of disease conditions including inflammation, cancer, arteriosclerosis; and psoriasis. Reversible protein phosphorylation is the main strategy for controlling the activities of eukaryotic cells. It is estimated that more than 1,000 or the 10,000 proteins active in a typical mammalian cell are phosphorylated. The high energy phosphate, which drives activation, is generally transferred from adenosine triphosphate molecules (ATP) to a particular protein by protein kinases and removed from that protein by protein phosphatases. Phosphorylation occurs in response to extracellular signals (hormones, neurotransmitters, growth and differentiation factors, etc), cell cycle checkpoints, and environmental or nutritional stresses.

The importance of kinases in the etiology of diseases has been well established. Kinase proteins are a major target for drug action and development. A January 2002 survey of ongoing clinical trials in the USA revealed more than 100 clinical trials involving the modulation of kinases. Trials are ongoing in a wide variety of therapeutic indications including asthma, Parkinson's, inflammation, psoriasis, rheumatoid arthritis, spinal cord injuries, muscle conditions, osteoporosis, graft versus host disease, cardiovascular disorders, autoimmune disorders, retinal detachment, stroke, epilepsy, ischemia/reperfusion, breast cancer, ovarian cancer, glioblastoma, non-Hodgkin's lymphoma, colorectal cancer, non-small cell lung cancer, brain cancer, Kaposi's sarcoma, pancreatic cancer, liver cancer, and other tumors. Numerous kinds of modulators of kinase activity are currently in clinical trials including antisense molecules, antibodies, small molecules, and even gene therapy. The present invention advances the state of the art by providing new links of kinase proteins to the etiology of colon cancer.

Many therapeutic strategies are aimed at protein kinases since they are critical components in signal transduction pathways. Approaches for regulating kinase gene expression include specific antisense oligonucleotides for inhibiting post-transcriptional processing of the messenger RNA, naturally occurring products and their chemical derivatives to inhibit kinase activity and monoclonal antibodies to inhibit receptor linked kinases. In some cases, kinase inhibitors also allow other therapeutic agents additional time to become effective and act synergistically with current treatments.

The role of phosphorylation in transcriptional control, apoptosis, protein degradation, nuclear import and export, cytoskeletal regulation, and checkpoint signaling has been an important subject in pharmaceutical research. The accumulating knowledge about signaling networks and the proteins involved will be put to practical use in the development of potent and specific pharmacological modulators of phosphorylation-dependent signaling that can be used for therapeutic purposes. The rational structure-based design and development of highly specific kinase modulators is becoming routine and drugs that intercede in signaling pathways are becoming a major class of drug.

The kinases comprise the largest known protein group, a superfamily of enzymes with widely varied functions and specificities. They are usually named after their substrate, their regulatory molecules, or some aspect of a mutant phenotype. With regard to substrates, the protein kinases may be roughly divided into two groups; those that phosphorylate tyrosine residues (protein tyrosine kinases, PTK) and those that phosphorylate serine or threonine residues (ser/thr kinases, STK).

An important subfamily of the STK family is cyclic-AMP dependent protein kinases (PKA). Cyclic-AMP is an intracellular mediator of hormone action in all prokaryotic and animal cells that have been studied. Such hormone-induced cellular responses include thyroid hormone secretion, cortisol secretion, progesterone secretion, glycogen breakdown, bone resorption, and regulation of heart rate and force of heart muscle contraction. PKA is found in many animal cells and is thought to account for the effects of cyclic-AMP in most of these cells. Altered PKA expression is implicated in a variety of disorders and diseases including cancer, thyroid disorders, diabetes, atherosclerosis, and cardiovascular disease.

The mitogen-activated protein kinases (MAP) are also members of the STK family. MAP kinases also regulate intracellular signaling pathways. They mediate signal transduction from the cell surface to the nucleus via phosphorylation cascades. Several subgroups have been identified, and each manifests different substrate specificities and responds to distinct extracellular stimuli. MAP kinase signaling pathways are present in mammalian cells as well as in yeast. The extracellular stimuli that activate mammalian pathways include epidermal growth factor (EGF), ultraviolet light, hyperosmolar medium, heat shock, endotoxic lipopolysaccharide (LPS), and pro-inflammatory cytokines such as tumor necrosis factor (TNF) and interleukin-1 (IL-1).

EGF receptor is found in over half of breast tumors unresponsive to hormone. EGF is found in many tumors, and EGF may be required for tumor cell growth. Antibody to EGF blocked the growth of tumor xenografts in mice. An antisense oligonucleotide for amphiregulin inhibited growth of a pancreatic cancer cell line.

Cell proliferation and differentiation in normal cells are under the regulation and control of multiple MAP kinase cascades. Aberrant and deregulated functioning of MAP kinases can initiate and support carcinogenesis. Insulin and IGF-1 also activate a mitogenic MAP kinase pathway that may be important in acquired insulin resistance occurring in type 2 diabetes.

Many cancers become refractory to chemotherapy by developing a survival strategy involving the constitutive activation of the phosphatidylinositol 3-kinase-protein kinase B/Akt signaling cascade. This survival signaling pathway thus becomes an important target for the development of specific inhibitors that would block its function. PI-3 kinase/Akt signaling is equally important in diabetes. The pathway activated by RTKs subsequently regulates glycogen synthase 3 (GSK3) and glucose uptake. Since Akt has decreased activity in type 2 diabetes, it provides a therapeutic target.

Although some protein kinases have, to date, no known system of physiological regulation, many are activated or inactivated by autophosphorylation or phosphorylation by upstream protein kinases. The regulation of protein kinases also occurs transcriptionally, post-transcriptionally, and post-translationally. The mechanism of post-transcriptional regulation is alternative splicing of precursor mRNA. Protein kinase C βI and βII are two isoforms of a single PKCβ gene derived from differences in the splicing of the exon encoding the C-terminal 50-52 amino acids. Splicing can be regulated by a kinase cascade in response to peptide hormones such as insulin and IGF-1. PKCβI and βII have different specificities for phosphorylating members of the MAP kinase family, for glycogen synthase 3β, for nuclear transcription factors such as TLS/Fus, and for other nuclear kinases. By inhibiting the post-transcriptional alternative splicing of PKCβII mRNA, PKCβII-dependent processes are inhibited.

Protein kinase C isoforms have been implicated in cellular changes observed in the vascular complications of diabetes. Hyperglycemia is associated with increased levels of PKCα and β isoforms in renal glomeruli of diabetic rats. Oral administration of a PKCβ inhibitor prevented the increased mRNA expression of TGF-β1 and extracellular matrix component genes. Administration of the specific PKCβ inhibitor (LY333531) also normalized levels of cytokines, caldesmon and hemodynamics of retinal and renal blood flow. Over-expression of the PKCβ isoform in the myocardium resulted in cardiac hypertrophy and failure. The use of LY333531 to prevent adverse effects of cardiac PKCβ over-expression in diabetic subjects is under investigation. The compound is also in Phase I/II clinical trials for diabetic retinopathy and diabetic macular edema indicating that it may be pharmacodynamically active.

PRK (proliferation-related kinase) is a serum/cytokine inducible STK that is involved in regulation of the cell cycle and cell proliferation in human megakaroytic cells. PRK is related to the polo (derived from human polo gene) family of STKs implicated in cell division. PRK is downregulated in lung tumor tissue and may be a proto-oncogene whose deregulated expression in normal tissue leads to oncogenic transformation. Altered MAP kinase expression is implicated in a variety of disease conditions including cancer, inflammation, immune disorders, and disorders affecting growth and development.

Protein kinase inhibitors provide much of our knowledge *about in vivo* regulation and coordination of kinase functions. A pseudosubstrate sequence within PKC acts to inhibit the kinase in the absence of its lipid activator.. A PKC inhibitor such as chelerythrine acts on the catalytic domain to block substrate interaction, while calphostin acts on the regulatory domain to mimic the pseudosubstrate sequence and block ATPase activity, or by inhibiting cofactor binding. The ability to inhibit specific PKC isozymes is limited.

Tamoxifen, a protein kinase C inhibitor with anti-estrogen activity, is currently a standard treatment for hormone-dependent breast cancer. The use of this compound may increase the risk of developing cancer in other tissues such as the endometrium. Raloxifene, a related compound, has been shown to protect against osteoporosis. The tissue specificity of inhibitors must be considered when identifying therapeutic targets.

The cyclin-dependent protein kinases (CDKs) are another group of STKs that control the progression of cells through the cell cycle. Cyclins are small regulatory proteins that act by binding to and activating CDKs that then trigger various phases of the cell cycle by phosphorylating and activating selected proteins involved in the mitotic process. CDKs are unique in that they require multiple inputs to become activated. In addition to the binding of cyclin, CDK activation requires the phosphorylation of a specific threonine residue and the dephosphorylation of a specific tyrosine residue.

Cellular inhibitors of CDKs also play a major role in cell cycle progression. Alterations in the expression, function, and structure of cyclin and CDK are encountered in the cancer phenotype. Therefore CDKs may be important targets for new cancer therapeutic agents.

Often chemotherapy resistant cells tend to escape apoptosis. Under certain circumstances, inappropriate CDK activation may even promote apoptosis by encouraging the progression of the cell cycle under unfavorable conditions, e.g., attempting mitosis while DNA damage is largely unrepaired.

Purines and purine analogs act as CDK inhibitors. Flavopiridol (L86-2,275) is a flavonoid that causes 50% growth inhibition of tumor cells at 60 nM (57). It also inhibits EGFR and protein kinase A. Flavopiridel induces apoptosis and inhibits lymphoid, myeloid, colon, and prostate cancer cells grown *in vivo* as tumor xenografts in nude mice.

Staurosporine and its derivative, UCN-O1, in addition to inhibiting protein kinase C, inhibit cyclin B/CDK (IC₅₀ 3 to 6 nM). Staurosporine is toxic, but its derivative 7-hydroxystaurosporine (UCN1) has anti-tumor properties and is in clinical trials. UCN-01 affects the phosphorylation of CDKs and alters the cell cycle checkpoint functioning. These compounds illustrate that multiple intracellular targets may be affected as the concentration of an inhibitor is increased within cells.

Protein tyrosine kinases, PTKs, specifically phosphorylate tyrosine residues on their target proteins and may be divided into transmembrane, receptor PTKs and non-transmembrane, non-receptor PTKs. Transmembrane protein-tyrosine kinases are receptors for many growth factors. Binding of growth factor to the receptor activates the transfer of a phosphate group from ATP to selected tyrosine side chains of the receptor and other specific proteins. Growth factors (GF) associated with receptor protein-tyrosine kinases (RTK) include epidermal GF, platelet-derived GF, fibroblast GF, hepatocyte GF, insulin and insulin-like GFs, nerve GF, vascular endothelial GF, and macrophage colony stimulating factor.

Inhibitors of RTKs may inhibit the growth and proliferation of such cancers, since RTKs stimulate tumor cell proliferation. Inhibitors of RTKs are also useful in preventing tumor angiogenesis and can eliminate support from the host tissue by targeting RTKs located on vascular cells (e.g., blood vessel endothelial cells and stromal fibroblasts (FGF receptor)).

Increasing knowledge of the structure and activation mechanism of RTKs and the signaling pathways controlled by tyrosine kinases provided the possibility for the development of target-specific drugs and new anti-cancer therapies. Approaches towards the prevention or interception of deregulated RTK signaling include the development of selective components that target either the extracellular ligand-binding domain or the intracellular tyrosine kinase or substrate binding region.

One successful strategy to selectively kill tumor cells is the use of monoclonal antibodies (mAbs) that are directed against the extracellular domain of RTKs which are involved in cancer and are expressed at the surface of tumor cells. In the past years, recombinant antibody technology has made enormous progress in the design, selection and production of new engineered antibodies, and it is possible to generate humanized antibodies, human-mouse chimeric or biospecific antibodies for targeted cancer therapy. Mechanistically, anti-RTK mAbs might work by blocking the ligand-receptor interaction and therefore inhibiting ligand-induced RTK signaling. In addition, by binding of to certain epitopes on the cancer cells, the anti-RTK mAbs induce immune-mediated responses such as opsonization and complement-mediated lysis and trigger antibody-dependent cellular cytotoxicity by macrophages or natural killer cells. In recent years, it became evident that mAbs control tumor growth by altering the intracellular signaling pattern inside the targeted tumor cell, leading to growth inhibition or apoptosis. In contrast, biospecific antibodies can bridge selected surface molecules on a target cell with receptors on an effector cell triggering cytotoxic responses against the target cell. Despite the toxicity that has been seen in clinical trials of bispecific antibodies, advances in antibody engineering, characterization of tumor antigens and immunology might help to produce rationally designed bispecific antibodies for anti-cancer therapy.

Another promising approach to inhibit aberrant RTK signaling are small molecule drugs that selectively interfere with the intrinsic tyrosine kinase activity and thereby block receptor autophosphorylation and activation of downstream signal transducers. The tyrphostins, which belong to the quinazolines, are one important group of such inhibitors that compete with ATP for the ATP binding site at the receptor's tyrosine kinase domain and some members have been shown to specifically inhibit the EGFR. Potent and selective inhibitors of receptors involved in neovascularization have been developed and are now undergoing clinical evaluation. Using the advantages of structure-based drug design, crystallographic structure information, combinatorial chemistry and high-throughput screening, new structural classes of tyrosine kinase inhibitors with increased potency and selectivity, higher *in vitro* and *in* vivo efficacy and decreased toxicity have emerged.

Recombinant immunotoxins provide another possibility of target-selective drug design. They are composed of a bacterial or plant toxin either fused or chemically conjugated to a specific ligand such as the variable domains of the heavy and light chains of mAbs or to a growth factor. Immunotoxins either contain the bacterial toxins Pseudomouas exotoxin A or diphtheria toxin or the plant toxins ricin A or clavin. These recombinant molecules can selectively kill their target cells when internalized after binding to specific cell surface receptors.

The use of antisense oligonucleotides represents another strategy to inhibit the activation of RTKs. Antisense oligonucleotides are short pieces of synthetic DNA or RNA that are designed to interact with the mRNA to block the transcription and thus the expression of specific-target proteins. These compounds interact with the mRNA by Watson-Crick base-pairing and are therefore highly specific for the target protein. Several preclinical and clinical studies suggest that antisense therapy might be therapeutically useful for the treatment of solid tumors.

Non-receptor PTKs lack transmembrane regions and, instead, form complexes with the intracellular regions of cell surface receptors. Such receptors that function through non-receptor PTKs include those for cytokines, hormones (growth hormone and prolactin) and antigen-specific receptors on T and B lymphocytes.

Many of these PTKs were first identified as the products of mutant oncogenes in cancer cells where their activation was no longer subject to normal cellular controls. In fact, about one third of the known oncogenes encode PTKs, and it is well-known that cellular transformation (oncogenesis) is often accompanied by increased tyrosine phosphorylation activity.

The potential of protein kinases and their relevant signaling as selective anti-cancer targets for therapeutic intervention has been recognized. As a consequence, a variety of successful target-specific drugs such as mAbs and protein kinase inhibitors have been developed and are currently evaluated in clinical trials.

Many tyrosine kinase inhibitors are derived from natural products including flavopiridol, genistem, erbstatin, lavendustin A, staurosporine, and UCN-O1. Inhibitors directed at the ATP binding site are also available. Signals from RTKs can also be inhibited at other target sites such as: nuclear tyrosine kinases, membrane anchors (inhibition of famesylation) and transcription factors.

Targeting the signaling potential of growth promoting tyrosine kinases such as EGFR, HER2, PDGFR, *src,* and *abl* may block tumor growth, while blocking IGF-I and TRK will interfere with tumor cell survival. Inhibiting these kinases may lead to tumor shrinkage and apoptosis. FkII/KDR and *src* are kinases necessary for neovascularization (angiogenesis) of tumors and inhibition of these may slow tumor growth thereby decreasing metastases.

Inhibitors of RTKs may stabilize the tumor in terms of cell proliferation, normal cell loss via apoptosis, and prevent cell migration, invasion and metastases. These drugs are likely to increase the time required for tumor progression, and may inhibit or attenuate the aggressiveness of the disease but may not initially result in measurable tumor regression.

Likewise, phosphatases, G-protein coupled receptors, ion channels, proteases, metabolic enzymes, and transcription factors are known targets for drug discovery.

### CCGs and CCG Products as Markers for Colon Cancer

CCGs, CCPNs, and CCPPs can be used as markers for colon cancer. Exemplary CCGs include those listed in Tables 1-5, or homologs thereof. Examples of CCPNs include SEQ ID NOS:1-63, or the complements or fragments thereof. Examples of CCPPs include SEQ ID NOS:64-126, or the fragments thereof. In many embodiments, CCPNs or CCPPs employed in the present invention include at least 5, 6, 7, 8, 9, 10, 15, 20, 25, 30, 35, 40, 45, 50, 100, or more consequence residues.

The expression profile of CCGs can also indicate the presence of a risk of colon cancer. These markers are further useful for correlating differences in levels of expression with a poor or favorable prognosis of colon cancer. In one example, probes for CCGs can be conveniently attached on one or more substrates (*e.g.,* biochips) for use in kits. In addition, the CCGs can be used to assess the efficacy of a treatment or therapy of colon cancer, or as targets for treatment or therapeutic agents. CCGs can also be used to generate vaccines for colon cancer, to produce antibodies specific to cancer cells, and to construct gene therapy vectors that can inhibit tumor growth. Without limitation as to mechanism, the invention is based in part on the principle that modulation of the expression of CCGs of the invention may ameliorate colon cancer when they are expressed at levels similar or substantially similar to diseased-free (*i.e.,* normal) tissues.

In one aspect, the expression levels of CCGs employed in the present invention are correlated with the presence of colon cancer. In certain embodiments, the present invention can be performed by detecting the presence of CCPNs or CCPPs using any suitable method known in the art. In another aspect, the expression levels of the CCGs are determined in a biological sample of a particular subject for which either diagnosis or prognosis information is desired. The expression profile of one or more CCGs can be used as a "fingerprint" to represent the disease state of a cell. In some examples, relative levels of expression are indicative of the severity of colon cancer and as such, can be used for diagnostic and prognostic analyses. Moreover, by comparing relative expression profiles of CCGs from tissue samples taken at different points in time, e.g., pre- and post-therapy or at different time points within a course of therapy or during colon cancer development, information regarding which gene is important for each of these stages can be obtained. In one example, comparison of expression profiles of CCGs at different stages of the tumor progression provides a method for long-term prognosis, including survival. In another example, a particular treatment regime can be evaluated based on CCG expression profiles, including whether a particular drug will act to improve the long-term prognosis in a particular patient.

The discovery of the differential expression patterns for individual or panels of CCGs allows for screening for test compounds that modulate a particular expression pattern. For example, screening can be done for compounds that will convert an expression profile for a poor prognosis to one for a better prognosis. In certain embodiments, this can be done by making biochips comprising sets of the significant CCGs, which can then be used in these screens. These methods can also be done on the protein level. Protein expression levels of the CCGs can be evaluated for diagnostic and prognostic purposes, or used to screen test compounds. For example, in relation to these embodiments, significant CCGs can comprise CCGs which are determined to have modulated activity or expression in response to a therapy regime. Alternatively, the modulation of the activity or expression of a CCG can be correlated with the diagnosis or prognosis of colon cancer. In addition, the CCGs can be administered for therapeutic purposes, including the administration of antisense nucleic acids or proteins (including CCPPs, antibodies to CCPPs and other modulators of CCPPs).

For example, the CCG STK 15 has increased expression in colon cancer tissue samples relative to control tissue samples. The presence of increased mRNA for this gene (or any other CCGs set forth in Tables 1-5), or increased levels of the protein products of this gene (or any other CCGs set forth in Tables 1-5) serve as markers for colon cancer. Accordingly, amelioration of colon cancer can be achieved by modulating up-regulated colon cancer markers, such as STK-15, to normal levels (*e.g.,* levels similar or substantially similar to tissue substantially free of colon cancer). In one instance, the up-regulated colon cancer marker is modulated to be similar to a control sample which is taken from a subject or tissue that is substantially free of colon cancer. Indeed, it is well established that the targets of many cancer therapeutics are kinases.

In another embodiment, a product of CCG, either in the form of a polynucleotide or a polypeptide, can be used as a therapeutic compound of the invention. In yet other embodiments, a modulator of CCG expression or the activity of a CCG product can be used as a therapeutic compound of the invention. The modulation may also be used in combination with one or more other therapeutic compositions of the invention. Formulation of such compounds into pharmaceutical compositions is described below. Administration of such a pharmaceutical composition may suppress bioactivity of CCG products and therefore can be used to ameliorate colon cancer.

### Sources of CCG Products

Polynucleotides and polypeptides encoded by CCGs *(i.e.,* CCPNs and CCPPs, respectively) can be isolated from any suitable tissue or cell of a subject of interest. In one embodiment, the cell or tissue is from colon or rectum. Other cell or tissue samples, including bodily fluids such as blood or feces, can serve as sources for isolation of CCG products. In addition, CCPNs or CCPPs can be prepared by using, without limitation, nucleic acid amplification, recombinant vectors encoding CCGs, chemical synthesis, or other methods as appreciated by those skilled in the art.

### Isolated Polynucleotides

One aspect of the invention pertains to isolated polynucleotides. Another aspect of the invention pertains to isolated polynucleotide fragments sufficient for use as hybridization probes to identify a CCPN in a sample; as well as nucleotide fragments for use as PCR primers of the amplification or mutation of the nucleic acid molecules which encodes CCPPs.

The probes/primers employed in the invention can be in any desired length. For instance, the probes/primers can include at least 7, 8, 9, 10, 15, 20, 30, 40, 50, 100, or more consecutive residues. Each probe/primer can hybridize under reduced stringent, stringent, or highly stringent conditions to an RNA transcript of a CCG, or the complement thereof. In one embodiment, each probe/primer can hybridize to an RNA transcript (or its complement) of a CCG but not RNA transcripts (or their complements) of other CCGs.

As used herein, the "stringency" of a hybridization reaction refers to the difficulty with which any two nucleic acid molecules will hybridize to one another. Examples of stringency conditions are shown in Table 6 below. Highly stringent conditions are those that are at least as stringent as, for example, conditions A-F; stringent conditions are at least as stringent as, for example, conditions G-L; and reduced stringency conditions are at least as stringent as, for example, conditions M-R.

**Table 6. Stringency Conditions**

| Stringency Condition | Poly-nucleotide Hybird | Hybrid Length (bp)¹ | Hybridization Temperature and Buffer^{H} | Wash Temp. and Buffer^{H} |
|---|---|---|---|---|
| A | DNA:DNA | >50 | 65°C; 1xSSC -or- 42°C; 1xSSC, 50% formamide | 65°C; 0.3xSSC |
| B | DNA:DNA | <50 | T_{B}*; 1xSSC | T_{B}*;1xSSC |
| C | DNA:RNA | >50 | 67°C; 1xSSC -or- 45°C; 1xSSC, 50% formamide | 67°C; 0.3xSSC |
| D | DNA:RNA | <50 | T_{D}*; 1xSSC | T_{D}*; 1xSSC |
| E | RNA:RNA | >50 | 70°C; 1xSSC -or- 50°C; 1xSSC, 50% formamide | 70°C; 0.3xSSC |
| F | RNA:RNA | <50 | T_{F}*; 1xSSC | T_{F}*; 1xSSC |
| G | DNA:DNA | >50 | 65°C; 4xSSC -or- 42°C; 4xSSC, 50% formamide | 65°C; 1xSSC |
| H | DNA:DNA | <50 | T_{H}*; 4xSSC | T_{H}*; 4xSSC |
| I | DNA:RNA | >50 | 67°C; 4xSSC -or- 45°C; 4xSSC, 50% formamide | 67°C; 1xSSC |
| J | DNA:RNA | <50 | T_{J}*; 4xSSC | T_{J}*; 4xSSC |
| K | RNA:RNA | >50 | 70°C; 4xSSC -or- 50°C; 4xSSC, 50% formamide | 67°C; 1xSSC |
| L | RNA:RNA | <50 | T_{L}*; 2xSSC | T_{L}*; 2xSSC |
| M | DNA:DNA | >50 | 50°C; 4xSSC -or- 40°C; 6xSSC, 50% formamide | 50°C;2xSSC |
| N | DNA:DNA | <50 | T_{N}*; 6xSSC | T_{N}*; 6xSSC |
| O | DNA:RNA | >50 | 55°C; 4xSSC -or- 42°C; 6xSSC, 50% formamide | 55°C; 2xSSC |
| P | DNA:RNA | <50 | T_{P}*; 6xSSC | T_{P}*; 6xSSC |
| Q | RNA:RNA | >50 | 60°C; 4xSSC -or- 45°C; 6xSSC, 50% formamide | 60°C; 2xSSC |
| R | RNA:RNA | <50 | T_{R}*; 4xSSC | T_{R}*; 4xSSC |

| | | | | |
|---|---|---|---|---|
| ¹: The hybrid length is that anticipated for the hybridized region(s) of the hybridizing polynucleotides. When hybridizing a polynucleotide to a target polynucleotide of unknown sequence, the hybrid length is assumed to be that of the hybridizing polynucleotide. When polynucleotides of known sequence are hybridized, the hybrid length can be determined by aligning the sequences of the polynucleotides and identifying the region or regions of optimal sequence complementarity. ^{H}: SSPE (1xSSPE is 0.15M NaCl, 10mM NaH₂PO₄, and 1.25mM EDTA, pH 7.4) can be substituted for SSC (1xSSC is 0.15M NaCl and 15mM sodium citrate) in the hybridization and wash buffers; washes are performed for 15 minutes after hybridization is complete. T_{B}* - T_{R}*: The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A+T bases) ⁺ 4(# of G+C bases). For hybrids between 18 and 49 base pairs in length, Tₘ(°C) = 81.5+16.6(log₁₀Na⁺) + 0.41(%G⁺C) - (600/N), where N is the number of bases in the hybrid, and Na⁺ is the molar concentration of sodium ions in the hybridization buffer (Na⁺ for 1xSSC = 0.165M). | | | | |

The probes based on the nucleotide sequence of a CCG or of a CCPN can be used to detect transcripts or genomic sequences of the CCG or CCPP. In certain embodiments, the probe comprises a label group attached thereto, e.g., the label group can be a radioisotope, a fluorescent compound, an enzyme, or an enzyme co-factor. Such probes can be used as part of a diagnostic test kit for identifying cells or tissue which over-express or under-express a CCG polynucleotide or polypeptide of the invention, or which have greater or fewer copies of a CCG. For example, the level of a CCG product in a sample of cells from a subject may be detected, the amount of polypeptide or mRNA transcript of a CCG may be determined, or the presence of mutations or deletions of a CCG of the invention may be assessed.

The invention further encompasses polynucleotide molecules that differ from the polynucleotide sequences of CCGs due to degeneracy of the genetic code but encode the same proteins encoded by the CCGs.

The invention also encompasses homologs of CCGs of other species. Gene homologs are well understood in the art and are available using databases or search engines such as the Pubmed-Entrez database.

In addition, the invention encompasses polynucleotide molecules which are structurally different from the molecules described above, but which have substantially the same properties as the molecules above. Such molecules include allelic variants.

DNA sequence polymorphisms leading to changes in the amino acid sequences of the proteins can exist within a population (*e.g.,* a human population). These polymorphic DNA sequences can be used by the present invention. Such genetic polymorphism may exist among individuals within a population due to natural allelic variation. In addition, it will be appreciated that DNA polymorphisms that affect RNA expression levels can also exist and may affect the overall expression level of that gene (e.g., by affecting regulation or degradation).

Polynucleotide molecules corresponding to natural allelic variants or homologs of CCGs can be isolated based on their homology to the CCGs using standard hybridization techniques under stringent or highly stringent hybridization conditions. Polynucleotide molecules corresponding to natural allelic variants or homologs of CCGs can further be isolated by mapping to the same chromosome or locus as the original CCG.

In another embodiment, a polynucleotide molecule used in the invention is at least 15, 20, 25, 30, 50, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000 or more nucleotides in length and can hybridize under reduced stringent, stringent, or highly stringent conditions to a sequence encoded by a CCG. In one example, the isolated polynucleotide molecule can hybridize under reduced stringent, stringent, or highly stringent conditions to a sequence selected from SEQ ID NOS:1-63.

In addition to naturally-occurring CCG allelic variants, the skilled artisan will further appreciate that changes can be introduced by mutation into the nucleotide sequences of the CCGs, thereby leading to changes in the amino acid sequences of the encoded proteins, without significantly altering the biological activities of these proteins. For example, nucleotide substitutions leading to amino acid substitutions at "non-essential" amino acid residues can be made. A "non-essential" amino acid residue is a residue that can be altered from the wild-type sequence of a protein without significantly altering the biological activity.

Accordingly, another aspect of the invention pertains to CCPP variants that contain changes in amino acid residues that are not essential for activity. Such variants differ in amino acid sequence from the original CCPP, yet retain biological activity of the corresponding CCPP. In one embodiment, a variant comprises an amino acid sequence with at least about 60%, 65%, 70%, 75%, 80%, 85%, 90%, 95%, 98% or more sequence identity or similarity to a CCPP.

A polynucleotide may be further modified to increase stability *in vivo.* Possible modifications include, but are not limited to, the addition of flanking sequences at the 5' or 3' ends; the use of phosphorothioate or 2-o-methyl rather than phosphodiester linkages in the backbone; and the inclusion of nontraditional bases such as inosine, queosine and wybutosine, as well as acetyl- methyl-, thio- and other modified forms of adenine, cytidine, guanine, thymine and uridine.

Another aspect of the invention pertains to isolated polynucleotide molecules that are antisense to the CCGs of the invention. An "antisense" polynucleotide comprises a nucleotide sequence which is complementary to a "sense" polynucleotide encoding a protein, *e.g.,* complementary to the coding strand of a double-stranded cDNA molecule or complementary to an mRNA sequence. Accordingly, an antisense polynucleotide can form hydrogen bonds to a sense polynucleotide. The antisense polynucleotide can be complementary to an entire coding strand of a CCG of the invention or to only a portion thereof. In one embodiment, an antisense polynucleotide molecule is antisense to a "coding region" of the coding strand of a nucleotide sequence of the invention. The term "coding region" includes the region of the nucleotide sequence comprising codons which are translated into amino acid. In another embodiment, the antisense polynucleotide molecule is antisense to a "noncoding region" of the coding strand of a nucleotide sequence of the invention.

Antisense polynucleotides of the invention can be designed according to the rules of Watson and Crick base pairing. The antisense polynucleotide molecule can be complementary to the entire coding region of an mRNA corresponding to a gene of the invention. In one embodiment, the antisense polynucleotide molecule is an oligonucleotide which is antisense to only a portion of the coding or noncoding region. An antisense oligonucleotide can be, for example, about 5, 10, 15, 20, 25, 30, 35, 40, 45 or 50 nucleotides in length. An antisense polynucleotide of the invention can be constructed using chemical synthesis and enzymatic ligation reactions known in the art. For example, an antisense polynucleotide can be chemically synthesized using naturally occurring nucleotides or variously modified nucleotides designed to increase the biological stability of the molecules or to increase the physical stability of the duplex formed between the antisense and sense polynucleotides, e.g., phosphorothioate derivatives and acridine substituted nucleotides can be used. Examples of modified nucleotides which can be used to generate the antisense polynucleotide include 5-fluorouracil, 5-bromouracil, 5-chlorouracil, 5-iodouracil, hypoxanthine, xanthine, 4-acetylcytosine, 5-(carboxyhydroxymethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladen4exine, unacil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, 3-(3-amino-3-N-2-carboxypropyl) uracil, (acp3)w, and 2,6-diaminopurine. Alternatively, the antisense polynucleotide can be produced biologically using an expression vector into which a polynucleotide has been subcloned in an antisense orientation (*e.g.*, RNA transcribed from the inserted polynucleotide will be of an antisense orientation to a target polynucleotide of interest, described further in the following subsection).

The antisense polynucleotide molecules of the invention are typically administered to a subject or generated *in situ* such that they hybridize with or bind to cellular mRNA or genomic DNA encoding a CCPP of the invention to thereby inhibit expression of the protein, e.g., by inhibiting transcription or translation. The hybridization may occur based on conventional nucleotide complementarity to form a stable duplex or, in the cases of an antisense polynucleotide molecule which binds to DNA duplexes, through specific interactions in the major groove of the DNA double helix. An example of a route of administration of antisense polynucleotide molecules of the invention is direct injection at a tissue site (e.g., intestine). Alternatively, antisense polynucleotide molecules can be modified to target selected cells and then administered systemically. For systemic administration, antisense molecules can be modified such that they specifically bind to receptors or antigens expressed on a selected cell surface, *e.g.,* by linking the antisense polynucleotide molecules to peptides or antibodies which bind to cell surface receptors or antigens. The antisense polynucleotide molecules can also be delivered to cells using the vectors described herein. To achieve sufficient intracellular concentrations of the antisense molecules, vector constructs in which the antisense polynucleotide molecule is placed under the control of a strong promoter, such as pol II or pol III promoter, may be employed.

In yet another embodiment, the antisense polynucleotide molecule of the invention is an α-anomeric polynucleotide molecule. An α-anomeric polynucleotide molecule forms specific double-stranded hybrids with complementary RNA in which, contrary to the usual β-units, the strands run parallel to each other (Gaultier et al., Polynucleotides. Res., 15:6625-6641, 1987). The antisense polynucleotide molecule can also comprise a 2'-o-methylribonucleotide or a chimeric RNA-DNA analogue.

In still another embodiment, an antisense polynucleotide of the invention is a ribozyme. Ribozymes are catalytic RNA molecules with ribonuclease activity which are capable of cleaving a single-stranded polynucleotide, such as an mRNA, to which they have a complementary region. Thus, ribozymes (*e.g.,* hammerhead ribozymes) can be used to catalytically cleave mRNA transcripts of the CCGs to thereby inhibit translation of said mRNA. A ribozyme having specificity for a CCPN can be designed based upon the nucleotide sequence of a gene of the invention, disclosed herein. For example, a derivative of a *Tetrahymena* L-19 IVS RNA can be constructed in which the nucleotide sequence of the active site is complementary to the nucleotide sequence to be cleaved in a CCG protein-encoding mRNA. Alternatively, mRNA transcribed from a gene of the invention can be used to select a catalytic RNA having a specific ribonuclease activity from a pool of RNA molecules. Alternatively, expression of a CCG of the invention can be inhibited by targeting the regulatory region of these genes (e.g., the promoter or enhancers) with complementary nucleotide sequences that will form triple helical structures with the target sequence to prevent transcription of the gene in target cells.

Expression of CCGs can also be inhibited using RNA interference ("RNAi"). RNAi is a phenomenon in which the introduction of double-stranded RNA (dsRNA) into certain organisms or cell types causes degradation of the homologous mRNA. First discovered in the nematode Caenorhabditis elegans, RNAi has since been found to operate in a wide range of organisms. For example, in mammalian cells, introduction of long dsRNA can initiate a potent antiviral response, exemplified by nonspecific inhibition of protein synthesis and RNA degradation. RNA interference provides a mechanism of gene silencing at the mRNA level. In recent years, RNAi has become an endogenous and potent gene-specific silencing technique that uses double-stranded RNAs (dsRNA) to mark a particular transcript for degradation in vivo. It also offers an efficient and broadly applicable approach for gene knock-out. In addition, RNAi technology can be used for therapeutic purposes. For example, RNAi targeting Fas-mediated apoptosis has been shown to protect mice from fulminant hepatitis.

Sequences capable of inhibiting gene expression by RNA interference can have any desired length. For instance, the sequence can have at least 10, 15, 20, 25, or more consecutive nucleotides. The sequence can be dsRNA or any other type of polynucleotide, provided that the sequence can form a functional silencing complex to degrade the target mRNA transcript.

In one embodiment, the sequence comprises or consists of a short interfering RNA (siRNA). The siRNA can be, for example, dsRNA having 19-25 nucleotides siRNAs can be produced endogenously by degradation of longer dsRNA molecules by an RNase III-related nuclease called Dicer siRNAs can also be introduced into a cell exogenously or by transcription of an expression construct. Once formed, the siRNAs assemble with protein components into endoribonuclease-containing complexes known as RNA-induced silencing complexes (RISCs). An ATP-generated unwinding of the siRNA activates the RISCs, which in turn target the complementary mRNA transcript by Watson-Crick base-pairing, thereby cleaving and destroying the mRNA. Cleavage of the mRNA takes place near the middle of the region bound by the siRNA strand. This sequence-specific mRNA degradation results in gene silencing.

At least two ways can be employed to achieve siRNA-mediated gene silencing. First, siRNAs can be synthesized in vitro and introduced into cells to transiently suppress gene expression. Synthetic siRNA provides an easy and efficient way to achieve RNAi. siRNA are duplexes of short mixed oligonucleotides which can include, for example, 19 nucleotides with symmetric dinucleotide 3' overhangs. Using synthetic 21 bp siRNA duplexes (e.g., 19 RNA bases followed by a UU or dTdT 3' overhang), sequence-specific gene silencing can be achieved in mammalian cells. These siRNAs can specifically suppress targeted gene translation in mammalian cells without activation of DNA-dependent protein kinase (PKR) by longer dsRNA, which may result in non-specific repression of translation of many proteins.

Second, siRNAs can be expressed in vivo from vectors. This approach can be used to stably express siRNAs in cells or transgenic animals. In one embodiment, siRNA expression vectors are engineered to drive siRNA transcription from polymerase III (pol III) transcription units. Pol III transcription units are suitable for hairpin siRNA expression, since they deploy a short AT rich transcription termination site that leads to the addition of 2 bp overhangs (e.g., UU) to hairpin siRNAs - a feature that is helpful for siRNA function. The Pol III expression vectors can also be used to create transgenic mice that express siRNA.

In another embodiment, siRNAs can be expressed in a tissue-specific manner. Under this approach, long double-stranded RNAs (dsRNAs) are first expressed from a promoter (such as CMV (pol II)) in the nuclei of selected cell lines or transgenic mice. The long dsRNAs are processed into siRNAs in the nuclei (e.g., by Dicer). The siRNAs exit from the nuclei and mediate gene-specific silencing. A similar approach can be used in conjunction with tissue-specific promoters to create tissue-specific knockdown mice.

Any 3' dinucleotide overhang, such as UU, can be used for siRNA design. In some cases, G residues in the overhang are avoided because of the potential for the siRNA to be cleaved by RNase at single-stranded G residues.

With regard to the siRNA sequence itself, it has been found that siRNAs with 30-50% GC content can be more active than those with a higher G/C content in certain cases. Moreover, since a 4-6 nucleotide poly(T) tract may act as a termination signal for RNA pol III, stretches of > 4 Ts or As in the target sequence may be avoided in certain cases when designing sequences to be expressed from an RNA pol III promoter. In addition, some regions of mRNA may be either highly structured or bound by regulatory proteins. Thus, it may be helpful to select siRNA target sites at different positions along the length of the gene sequence. Finally, the potential target sites can be compared to the appropriate genome database (human, mouse, rat, etc.). Any target sequences with more than 16-17 contiguous base pairs of homology to other coding sequences may be eliminated from consideration in certain cases.

In one embodiment, siRNA is designed to have two inverted repeats separated by a short spacer sequence and end with a string of Ts that serve as a transcription termination site. This design produces an RNA transcript that is predicted to fold into a short hairpin siRNA. The selection of siRNA target sequence, the length of the inverted repeats that encode the stem of a putative hairpin, the order of the inverted repeats, the length and composition of the spacer sequence that encodes the loop of the hairpin, and the presence or absence of 5'-overhangs, can vary to achieve desirable results.

The siRNA targets can be selected by scanning an mRNA sequence for AA dinucleotides and recording the 19 nucleotides immediately downstream of the AA. Other methods can also been used to select the siRNA targets. In one example, the selection of the siRNA target sequence is purely empirically determined (see e.g., Sui et al., Proc. Natl. Acad. Sci. USA 99: 5515-5520, 2002), as long as the target sequence starts with GG and does not share significant sequence homology with other genes as analyzed by BLAST search. In another example, a more elaborate method is employed to select the siRNA target sequences. This procedure exploits an observation that any accessible site in endogenous mRNA can be targeted for degradation by synthetic oligodeoxyribonucleotide /RNase H method (Lee et al., Nature Biotechnology 20:500-505, 2002).

In another embodiment, the hairpin siRNA expression cassette is constructed to contain the sense strand of the target, followed by a short spacer, the antisense strand of the target, and 5-6 Ts as transcription terminator. The order of the sense and antisense strands within the siRNA expression constructs can be altered without affecting the gene silencing activities of the hairpin siRNA. In certain instances, the reversal of the order may cause partial reduction in gene silencing activities.

The length of nucleotide sequence being used as the stem of siRNA expression cassette can range, for instance, from 19 to 29. The loop size can range from 3 to 23 nucleotides. Other lengths and/or loop sizes can also be used.

In yet another embodiment, a 5' overhang in the hairpin siRNA construct can be used, provided that the hairpin siRNA is functional in gene silencing. In one example, the 5' overhang includes about 6 nucleotide residues.

In still yet another embodiment, the target sequences for RNAi are 21-mer sequence fragments selected from CCG coding sequences, such as SEQ ID NOS:1-63. The target sequences can be selected from either ORF regions or non-ORF regions. The 5' end of each target sequence has dinucleotide "NA," where "N" can be any base and "A" represents adenine. The remaining 19-mer sequence has a GC content of between 30% and 65%. In many examples, the remaining 19-mer sequence does not include any four consecutive A or T (i.e., AAAA or TTTT), three consecutive G or C (i.e., GGG or CCC), or seven "GC" in a row. Examples of the target sequences prepared using the above-described criteria ("Relaxed Criteria") are illustrated in Table 7. Each target sequence in Table 7 has SEQ ID NO:3n+1, and the corresponding siRNA sense and antisense strands have SEQ ID NO:3n+2 and SEQ ID NO:3(n+1), respectively, where n is an integer. Antisense strand seqeunces (SEQ ID NO:3(n+1)) are presented in the 3' to 5' direction. For each CCG coding sequence (SEQ ID NOS:1-63), multiple target sequences can be selected.

Additional criteria can be used for RNAi target sequence design. In one example, the GC content of the 19-mer sequence is limited to between 35% and 55%, and any 19-mer sequence having three consecutive A or T (i.e., AAA or TTT) or a palindrome sequence with 5 or more bases is excluded. In addition, the 19-mer sequence can be selected to have low sequence homology to other human genes. In one embodiment, potential target sequences are searched by BLASTN against NCBI's human UniGene cluster sequence database. The human UniGene database contains non-redundant sets of gene-oriented clusters. Each UniGene cluster includes sequences that represent a unique gene. 19-mer sequences producing no hit to other human genes under the BLASTN search can be selected. During the search, the e-value may be set at a stringent value (such as "1"). Furthermore, the target sequence can be selected from the ORF region, and is at least 75-bp from the start and stop codons. Examples of the target sequences prepared using these criteria ("Stringent Criteria") are demonstrated in Table 7. siRNA sense and antisense sequences (SEQ ID NO:3n+2 and SEQ ID NO:3(n+1), respectively) for each target sequence (SEQ ID NO:3n) are also provided. Antisense strand seqeunces (SEQ ID NO:3(n+1)) are presented in the 3' to 5' direction.

**Table 7. RNAi Target Sequences and siRNA Sequences for CCGs**

| SEQ ID NO (CCG coding seq.) | Relaxed Criteria (target seq.: SEQ ID NO:3n+1; siRNA sense seq.: SEQ ID NO:3n+2; siRNA antisense seq.: SEQ ID NO:3(n+1)) | Stringent Criteria (target seq.: SEQ ID NO:3n+1; siRNA sense seq.: SEQ ID NO:3n+2; siRNA antisense seq.: SEQ ID NO:3(n+1)) |
|---|---|---|
| 1 | SEQ ID NOS: 127-1554 | SEQ ID NOS: 1555-1788 |
| 2 | SEQ ID NOS: 1789-2625 | SEQ ID NOS: 2626-2697 |
| 3 | SEQ ID NOS: 2698-3195 | |
| 4 | SEQ ID NOS: 3196-3609 | SEQ ID NOS: 3610-3669 |
| 5 | SEQ ID NOS: 3670-4101 | SEQ ID NOS: 4102-4113 |
| 6 | SEQ ID NOS: 4114-5715 | SEQ ID NOS: 5716-6015 |
| 7 | SEQ ID NOS: 6016-6231 | |
| 8 | SEQ ID NOS: 6232-8097 | SEQ ID NOS: 8098-8571 |
| 9 | SEQ ID NOS: 8572-9321 | SEQ ID NOS: 9322-9462 |
| 10 | SEQ ID NOS: 9463-10176 | SEQ ID NOS: 10177-10356 |
| 11 | SEQ ID NOS: 10357-10695 | SEQ ID NOS: 10696-10752 |
| 12 | SEQ ID NOS: 10753-11379 | SEQ ID NOS: 11380-11484 |
| 13 | SEQ ID NOS: 11485-11811 | SEQ ID NOS: 11812-11826 |
| 14 | SEQ ID NOS: 11827-13056 | SEQ ID NOS: 13057-13272 |
| 15 | SEQ ID NOS: 13273-13479 | SEQ ID NOS: 13480-13488 |
| 16 | SEQ ID NOS: 13489-14265 | SEQ ID NOS: 14266-14364 |
| 17 | SEQ ID NOS: 14365-15303 | SEQ ID NOS: 15304-15432 |
| 18 | SEQ ID NOS: 15433-15732 | SEQ ID NOS: 15733-15741 |
| 19 | SEQ ID NOS: 15742-16488 | SEQ ID NOS: 16489-16530 |
| 20 | SEQ ID NOS: 16531-17247 | SEQ ID NOS: 17248-17337 |
| 21 | SEQ ID NOS: 17338-18255 | SEQ ID NOS: 18256-18456 |
| 22 | SEQ ID NOS: 18457-19605 | SEQ ID NOS: 19606-19800 |
| 23 | SEQ ID NOS: 19801-20016 | |
| 24 | SEQ ID NOS: 20017-21621 | SEQ ID NOS: 21622-22014 |
| 25 | SEQ ID NOS: 22015-22428 | SEQ ID NOS: 22429-22461 |
| 26 | SEQ ID NOS: 22462-23148 | SEQ ID NOS: 23149-23151 |
| 27 | SEQ ID NOS: 23152-23187 | |
| 28 | SEQ ID NOS: 23188-24174 | SEQ ID NOS: 24175-24180 |
| 29 | SEQ ID NOS: 24181-24339 | SEQ ID NOS: 24340-24366 |
| 30 | SEQ ID NOS: 24367-24567 | SEQ ID NOS: 24568-24582 |
| 31 | SEQ ID NOS: 24583-25188 | SEQ ID NOS: 25189-25206 |
| 32 | SEQ ID NOS: 25207-25515 | SEQ ID NOS: 25516-25518 |
| 33 | SEQ ID NOS: 25519-25851 | SEQ ID NOS: 25852-25857 |
| 34 | SEQ ID NOS: 25858-26070 | SEQ ID NOS: 26071-26148 |
| 35 | SEQ ID NOS: 26149-26550 | |
| 36 | SEQ ID NOS: 26551-31761 | SEQ ID NOS: 31762-32658 |
| 37 | SEQ ID NOS: 32659-34032 | SEQ ID NOS: 34033-34323 |
| 38 | SEQ ID NOS: 34324-35706 | SEQ ID NOS: 35707-35892 |
| 39 | SEQ ID NOS: 35893-36381 | SEQ ID NOS: 36382-36429 |
| 40 | SEQ ID NOS: 36430-37146 | SEQ ID NOS: 37147-37239 |
| 41 | SEQ ID NOS: 37240-37713 | SEQ ID NOS: 37714-37752 |
| 42 | SEQ ID NOS: 37753-38154 | SEQ ID NOS: 38155-38208 |
| 43 | SEQ ID NOS: 38209-38844 | SEQ ID NOS: 38845-38883 |
| 44 | SEQ ID NOS: 38884-39837 | SEQ ID NOS: 39838-39924 |
| 45 | SEQ ID NOS: 39925-40125 | |
| 46 | SEQ ID NOS: 40126-40863 | SEQ ID NOS: 40864-40902 |
| 47 | SEQ ID NOS: 40903-42051 | SEQ ID NOS: 42052-42267 |
| 48 | SEQ ID NOS: 42268-42921 | SEQ ID NOS: 42922-42996 |
| 49 | SEQ ID NOS: 42997-43470 | SEQ ID NOS: 43471-43527 |
| 50 | SEQ ID NOS: 43528-43926 | |
| 51 | SEQ ID NOS: 43927-45804 | SEQ ID NOS: 45805-46167 |
| 52 | SEQ ID NOS: 46168-46731 | SEQ ID NOS: 46732-46833 |
| 53 | SEQ ID NOS: 46834-47874 | SEQ ID NOS: 47875-48030 |
| 54 | SEQ ID NOS: 48031-4.8297 | SEQ ID NOS: 48298-48303 |
| 55 | SEQ ID NOS: 48304-48807 | |
| 56 | SEQ ID NOS: 48808-49608 | SEQ ID NOS: 49609-49683 |
| 57 | SEQ ID NOS: 49684-49956 | SEQ ID NOS: 49957-49971 |
| 58 | SEQ ID NOS: 49972-51438 | SEQ ID NOS: 51439-51699 |
| 59 | SEQ ID NOS: 51700-52878 | SEQ ID NOS: 52879-52926 |
| 60 | SEQ ID NOS: 52927-53196 | SEQ ID NOS: 53197-53205 |
| 61 | SEQ ID NOS: 53206-54168 | SEQ ID NOS: 54169-54306 |
| 62 | SEQ ID NOS: 54307-54708 | SEQ ID NOS: 54709-54747 |
| 63 | SEQ ID NOS: 54748-54873 | |

The effectiveness of the siRNA sequences can be evaluated using various methods known in the art. For instance, a siRNA sequence of the present invention can be introduced into a cell that expresses a CCG. The polypeptide or mRNA level of the CCG in the cell can be detected. A substantial change in the expression level of the CCG before and after the introduction of the siRNA sequence is indicative of the effectiveness of the siRNA sequence in suppressing the expression of the CCG. In one example, the expression levels of other genes are also monitored before and after the introduction of the siRNA sequence. A siRNA sequence which has inhibitory effect on the CCG expression but does not significantly affect the expression of other genes can be selected. In another example, multiple siRNA or other RNAi sequences can be introduced into the same target cell. These siRNA or RNAi sequences specifically inhibit the CCG gene expression but not the expression of other genes. In yet another example, siRNA or other RNAi sequences that inhibit the expression of both the CCG gene and other gene or genes can be used.

In yet another embodiment, the polynucleotide molecules employed in the present invention can be modified at the base moiety, sugar moiety or phosphate backbone to improve, e.g., the stability, hybridization, or solubility of the molecule. For example, the deoxyribose phosphate backbone of the polynucleotide molecules can be modified to generate peptide polynucleotides. As used herein, the terms "peptide polynucleotides" or "PNAs" refer to polynucleotide mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols.

PNAs can be used in therapeutic and diagnostic applications. For example, PNAs can be used as antisense or antigene agents for sequence-specific modulation of CCG expression by inducing transcription or translation arrest or inhibiting replication. PNAs of the polynucleotide molecules of the invention (e.g., SEQ ID NOS:1-63, or homologs or fragments thereof) can also be used in the analysis of single base pair mutations in a gene, (e.g., by PNA-directed PCR clamping), as artificial restriction enzymes when used in combination with other enzymes (e.g., S1 nucleases) or as probes or primers for DNA sequencing or hybridization.

In another embodiment, PNAs can be modified to enhance their stability or cellular uptake by attaching lipophilic or other helper groups to PNA, by the formation of PNA-DNA chimeras, or by the use of liposomes or other techniques of drug delivery known in the art. For example, PNA-DNA chimeras of the polynucleotide molecules of the invention can be generated. Such chimeras allow DNA recognition enzymes, (e.g., RNase H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation. The synthesis of PNA-DNA chimeras can be performed. For example, a DNA chain can be synthesized on a substrate support using standard phosphoramidite coupling chemistry and modified nucleoside analogs, *e.g.,* 5'-(4-methoxytrityl)amino-5'-deoxy-thymidine phosphoramidite, can be used as a spacer between the PNA and the 5' end of DNA. PNA monomers are then coupled in a stepwise manner to produce a chimeric molecule with a 5' PNA segment and a 3' DNA segment. Alternatively, chimeric molecules can be synthesized with a 5' DNA segment and a 3' PNA segment.

In other embodiments, the polynucleotide may include other appended groups such as peptides (*e.g.,* for targeting host cell receptors *in vivo),* or agents facilitating transport across the cell membrane or the blood-kidney barrier (see, e.g., PCT Publication No. W089/10134). In addition, polynucleotides can be modified with hybridization-triggered cleavage agents or intercalating agents. To this end, the polynucleotide may be conjugated to another molecule (e.g., a peptide, hybridization triggered cross-linking agent, transport agent, or hybridization-triggered cleavage agent). Finally, the polynucleotide may be detectably labeled, either such that the label is detected by the addition of another reagent (e.g., a substrate for an enzymatic label), or is detectable immediately upon hybridization of the nucleotide (e.g., a radioactive label or a fluorescent label).

### Isolated Polypeptides

Several aspects of the invention pertain to isolated CCPPs and biologically active portions thereof, as well as polypeptide fragments suitable for use as immunogens to raise anti-CCPP antibodies. In one embodiment, native CCPPs can be isolated from cells or tissue sources by an appropriate purification scheme using standard protein purification techniques. The degree of purification will vary depending on the use of the CCPP. In some instances, no purification will be necessary.

In one embodiment, mutated CCPPs capable of inhibiting normal CCPP activity (dominant-negative mutants) are produced by recombinant DNA techniques. Alternative to recombinant expression, mutated CCPPs can be synthesized chemically using standard peptide synthesis techniques.

In another embodiment, the invention provides CCPPs encoded by CCGs set forth in Tables 1-5, or the homologs thereof. In another embodiment, the CCPPs are substantially homologous to a CCPP selected from Tables 1-5, and retain the functional activity of the CCPP, yet differ in amino acid sequence due to allelic variation or mutagenesis. In still another embodiment, the CCPPs are variants which have at least about 60%, 65%, 70%, 75%, 80%, 85%, 90%, 95%, 98%, or more sequence identity or similarity to the original CCPPS (*e.g.,* SEQ ID NOS:64-126, or fragments thereof).

To determine the percent identity or similarity of two amino acid sequences or two nucleotide sequences, the sequences are aligned for optimal comparison purposes (e.g., gaps can be introduced in one or both of a first and a second amino acid or polynucleotide sequence for optimal alignment and non-homologous sequences can be disregarded for comparison purposes). The percent identity or similarity between the two sequences is a function of the number of identical or similar positions shared by the sequences; taking into account the number of gaps, and the length of each gap, which need to be introduced for optimal alignment of the two sequences.

The comparison of sequences and determination of percent identity between two sequences can be accomplished using a mathematical algorithm. In one embodiment, the percent identity or similarity between two amino acid sequences is determined using the Needleman and Wunsch (J. Mol. Biol., 48:444-453, 1970) algorithm which has been incorporated into the GAP program in the GCG software package, using either a Blossom 62 matrix or a PAM250 matrix, and a gap weight of 16, 14, 12, 10, 8, 6, or 4 and a length weight of 1, 2, 3, 4, 5, or 6. In another embodiment, the percent identity or similarity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6.

The polynucleotides and protein sequences of the present invention can further be used as "query sequences" to perform searches against public databases to, for example, identify other family members or related sequences. Such searches can be performed using BLAST programs available at the BLAST website maintained by the National Center of Biotechnology Information (NCBI), National Library of Medicine, Washington, DC, USA.

The invention also provides chimeric or fusion CCPPs. A fusion CCPP may contain all or a portion of a CCPP and a fusion partner. In one embodiment, a fusion CCPP comprises at least one biologically active portion of a CCPP. The fusion partner can be fused to the N-terminus or C-terminus of the CCPP.

A peptide linker sequence may be employed to separate the CCPP from its fusion partner by a distance sufficient to ensure that each polypeptide folds into its secondary and tertiary structures. Such a peptide linker sequence is incorporated into the fusion protein using standard techniques well-known in the art. Suitable peptide linker sequences may be chosen based on the following factors: (1) their ability to adopt a flexible extended conformation; (2) their inability to adopt a secondary structure that could interact with functional epitopes on the CCPP or its fusion partner; and (3) the lack of hydrophobic or charged residues that might react with the polypeptide functional epitopes. Exemplary peptide linker sequences contain Gly, Asn and Ser residues. Other near neutral amino acids, such as Thr and Ala can also be used in the linker sequence. Amino acid sequences which may be usefully employed as linkers include those disclosed in Maratea et al., Gene, 40:39-46, 1985; Murphy et al., Proc. Natl. Acad. Sci., USA, 83:8258-8262, 1986; U.S. Pat. No. 4,935,233 and U.S. Pat. No. 4,751,180. The linker sequence may generally be from 1 to about 50 amino acids in length. Linker sequences are not required when the CCPP-related polypeptide and non-CCPP-related polypeptide have non-essential N-terminal amino acid regions that can be used to separate the functional domains and prevent steric interference.

In one embodiment, the fusion protein is a glutathione s-transferase (GST)-CCPP fusion protein in which the CCPP sequence is fused to the C-terminus of the GST sequences. Such fusion proteins can facilitate the purification of recombinant CCPPs.

In another embodiment, the fusion protein is a CCPP containing a heterologous signal sequence at its N-terminus. In certain host cells (e.g., mammalian host cells), expression or secretion of CCPPs can be increased through use of a heterologous signal sequence. Such signal sequences are well-known in the art.

The CCPP fusion proteins of the invention can be incorporated into pharmaceutical compositions and administered to a subject *in vivo,* as described herein. The CCPP fusion proteins can be used to affect the bioavailability of a CCPP substrate. Use of CCPP fusion proteins may be useful therapeutically for the treatment of or prevention of damage caused by, for example, (i) aberrant modification or mutation of a CCG; (ii) mis-regulation of a CCG; and (iii) aberrant post-translational modification of a CCPP.

Moreover, the CCPP fusion proteins of the invention can be used as immunogens to produce anti-CCPP antibodies in a subject, to purify CCPP ligands and in screening assays to identify molecules which inhibit the interaction of a CCPP with a CCPP substrate.

CCPP fusion proteins used as immunogens may comprise a non-CCPP immunogenic polypeptide. In one example, the immunogenic protein is capable of eliciting a recall response. In another example, a CCPP chimeric or fusion protein of the invention is produced by standard recombinant DNA techniques. For instance, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation.

In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence. Moreover, many expression vectors are commercially available that already encode a fusion moiety (e.g., a GST polypeptide). A CCPP-encoding polynucleotide can be cloned into such an expression vector such that the fusion moiety is linked in-frame to the CCPP.

A signal sequence can be used to facilitate secretion and isolation of the secreted protein or other proteins of interest. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, the invention pertains to the described polypeptides having a signal sequence, as well as to polypeptides from which the signal sequence has been proteolytically cleaved (*i.e.,* the cleavage products). In one embodiment, a polynucleotide sequence encoding a signal sequence can be operably linked in an expression vector to a protein of interest, such as a protein which is ordinarily not secreted or is otherwise difficult to isolate. The signal sequence directs secretion of the protein, such as from a eukaryotic host into which the expression vector is transformed, and the signal sequence is subsequently or concurrently cleaved. The protein can then be readily purified from the extracellular medium by art recognized methods.

Alternatively, the signal sequence can be linked to the protein of interest using a sequence which facilitates purification, such as with a GST domain.

The present invention also pertains to variants of the CCPPs of the invention which function as either agonists or as antagonists to the CCPPs. In one embodiment, antagonists or agonists of CCPPs are used as therapeutic agents. For example, antagonists of an up-regulated CCG that can decrease the activity or expression of such a gene may ameliorate colon cancer in a subject wherein the CCG is abnormally increased in level or activity. In this embodiment, treatment of such a subject may comprise administering the antagonists to decrease activity or expression of the targeted CCG. Variants of the CCPPs can be generated by mutagenesis, e.g., discrete point mutation or truncation of a CCPP.

In certain embodiments, an agonist of the CCPPs can retain substantially the same, or a subset, of the biological activities of the naturally occurring form of a CCPP or may enhance an activity of a CCPP. In certain embodiments, an antagonist of a CCPP can inhibit one or more of the activities of the naturally occurring form of the CCPP by, for example, competitively modulating an activity of a CCPP. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the CCPP.

Mutants of a CCPP which function as either CCPP agonists or as CCPP antagonists can be identified by screening combinatorial libraries of mutants, e.g., truncation mutants, of a CCPP for CCPP agonist or antagonist activity. A variegated library of CCPP variants can be produced by, for example, enzymatically ligating a mixture of synthetic oligonucleotides into gene sequences such that a degenerate set of potential CCPP sequences is expressible as individual polypeptides, or alternatively, as a set of larger fusion proteins (e.g., for phage display) containing the set of CCPP sequences therein. There are a variety of methods which can be used to produce libraries of potential CCPP variants from a degenerate oligonucleotide sequence. Chemical synthesis of a degenerate gene sequence can be performed in an automatic DNA synthesizer, and the synthetic gene is then ligated into an appropriate expression vector. Use of a degenerate set of genes allows for the provision, in one mixture, of all of the sequences encoding the desired set of potential CCPP sequences. Methods for synthesizing degenerate oligonucleotides are known in the art.

In addition, libraries of fragments of a protein coding sequence corresponding to a CCPP of the invention can be used to generate a variegated population of CCPP fragments for screening and subsequent selection of variants of a CCPP. In one embodiment, a library of coding sequence fragments can be generated by treating a double-stranded PCR fragment of a CCPP coding sequence with a nuclease under conditions wherein nicking occurs only about once per molecule, denaturing the double-stranded DNA, renaturing the DNA to form double-stranded DNA which can include sense/antisense pairs from different nicked products, removing single-stranded portions from reformed duplexes by treatment with S1 nuclease, and ligating the resulting fragment library into an expression vector. By this method, an expression library can be derived which encodes N-terminal, C-terminal and internal fragments of various sizes of the CCPP.

Several techniques are known in the art for screening gene products of combinatorial libraries made by point mutations or truncation, and for screening cDNA libraries for gene products having a selected property. Some widely used techniques, which are amenable to high-throughput analysis, for screening large gene libraries include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify CCPP variants (Delgrave et al. Protein Engineering, 6:327-331, 1993).

Portions of a CCPP or variants of a CCPP may also be generated by synthetic means, using techniques well-known to those of ordinary skill in the art. For example, such polypeptides may be synthesized using any of the commercially available solid-phase techniques, such as the Merrifield solid-phase synthesis method, where amino acids are sequentially added to a growing amino acid chain. Equipment for automated synthesis of polypeptides is commercially available from suppliers such as Perkin Elmer/Applied BioSystems Division (Foster City, CA), and may be operated according to the manufacturer's instructions.

Methods and compositions for screening for protein inhibitors or activators are known in the art (see U.S. patent 4,980,281, 5,266,464, 5,688,635, and 5,877,007).

In yet another embodiment, the present invention provides variants of CCPPs that have substantially the same immunogenicity as the original CCPPs. In certain examples, these variants have at least 60%, 65%, 50%, 75%, 80%, 85%, 90%, 95%, 98%, or more sequence identity or similarity to the original CCPPs. In some other examples, the variants include the same immunogenic epitopes as the original CCPPs. These variants can be used, for example, for making antibodies, vaccines, or biochips.

### Antibodies

In another aspect, the invention provides antibodies that are specific to CCPPs or their variants. In one embodiment, the antibodies are monoclonal antibodies. In another embodiment, the antibodies are humanized antibodies.

In yet another aspect, the invention provides methods of making an isolated hybridoma which produces an antibody useful for diagnosing a patient or animal with colon cancer. In this method, a CCPP or its variant is isolated (*e.g.,* by purification from a cell in which it is expressed or by transcription and translation of a polynucleotide encoding the protein *in vivo* or *in vitro* using known methods). A vertebrate, such as a mouse, rabbit, sheep, or another mammal, is immunized using the isolated polypeptide or polypeptide fragment. The vertebrate may optionally be immunized at least one additional time with the isolated polypeptide or polypeptide fragment, so that the vertebrate exhibits a robust immune response to the polypeptide or polypeptide fragment. Splenocytes are isolated from the immunized vertebrate and fused with an immortalized cell line to form hybridomas, using any of a variety of methods well-known in the art. Hybridomas formed in this manner are then screened using standard methods to identify one or more hybridomas which produce an antibody which specifically binds with the polypeptide or polypeptide fragment. The invention also includes hybridomas made by this method and antibodies made using such hybridomas.

An isolated CCPP, or a portion or fragment thereof, can be used as an immunogen to generate antibodies that bind the CCPP using standard techniques for polyclonal and monoclonal antibody preparation. A full-length CCPP can be used or, alternatively, the invention provides antigenic peptide fragments of the CCPP for use as immunogens. In one example, the antigenic peptide of a CCPP comprises at least 8 amino acid residues of an amino acid sequence encoded by a CCG set forth in Tables 1-5, and encompasses an epitope of a CCPP such that an antibody raised against the peptide forms a specific immune complex with the CCPP.

Immunogenic portions (or epitopes) may generally be identified using well-known techniques. Such techniques include screening polypeptides for the ability to react with antigen-specific antibodies, antisera or T cell lines or clones. As used herein, antisera and antibodies are "specific" for an antigen if they bind to the antigen with a substantially high binding affinity. In some examples, binding affinity between the antisera or antibodies and the antigen can be at least 10⁵ M⁻¹, 10⁶ M⁻¹, 10⁷ M⁻¹, 10⁸ M⁻¹, or more. Such antisera and antibodies may be prepared as described herein using well-known techniques.

Exemplary epitopes encompassed by the antigenic peptide are regions of the CCPP that are located on the surface of the protein, *e.g.,* hydrophilic regions, as well as regions with high antigenicity.

A CCPP immunogen typically is used to prepare antibodies by immunizing a suitable subject (*e.g.,* rabbit, goat, mouse or other mammal) with the immunogen. An appropriate immunogenic preparation can contain, for example, recombinantly expressed CCPP or a chemically synthesized CCPP. The preparation can further include an adjuvant, such as Freund's complete or incomplete adjuvant, or similar immunostimulatory agent. Immunization of a suitable subject with an immunogenic CCPP preparation induces a polyclonal anti-CCPP antibody response. Techniques for preparing, isolating and using antibodies are well-known in the art.

Accordingly, another aspect of the invention pertains to monoclonal or polyclonal anti-CCPP antibodies. Examples of immunologically active portions of immunoglobulin molecules include F(ab) and F(ab')₂ fragments which can be generated by treating the antibody with an enzyme such as pepsin. The invention provides polyclonal and monoclonal antibodies that bind to CCPP.

Polyclonal anti-CCPP antibodies can be prepared as described above by immunizing a suitable subject with a CCPP. The anti-CCPP antibody titer in the immunized subject can be monitored over time by standard techniques, such as with an enzyme linked immunosorbent assay (ELISA) using immobilized CCPP. If desired, the antibody molecules directed against CCPPs can be isolated from the mammal (*e.g.,* from the blood) and further purified by well-known techniques, such as protein A chromatography, to obtain the IgG fraction. At an appropriate time after immunization, e.g., when the anti-CCPP antibody titers are highest, antibody-producing cells can be obtained from the subject and used to prepare monoclonal antibodies by standard techniques, such as the hybridoma technique, human B cell hybridoma technique, the EBV-hybridoma technique, or trioma techniques. The technology for producing monoclonal antibody hybridomas is well-known. Briefly, an immortal cell line (typically a myeloma) is fused to lymphocytes (typically splenocytes) from a mammal immunized with a CCPP immunogen as described above, and the culture supernatants of the resulting hybridoma cells are screened to identify a hybridoma producing a monoclonal antibody that binds to a CCPP of the invention.

Numerous protocols are available for fusing lymphocytes and immortalized cell lines to generate anti-CCPP monoclonal antibodies. Moreover, the ordinarily skilled worker will appreciate that there are many variations of such methods which also would be useful. Typically, the immortal cell line (*e.g.,* a myeloma cell line) is derived from the same mammalian species as the lymphocytes. For example, murine hybridomas can be made by fusing lymphocytes from a mouse immunized with an immunogenic preparation of the present invention with an immortalized mouse cell line. Exemplary immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g.,* the P3-NS1/1-Ag4-1, P3-x63-Ag8.653 or Sp210-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind to a CCPP, e.g., using a standard ELISA assay.

Alternative to preparing monoclonal antibody-secreting hybridomas, a monoclonal anti-CCPP antibody can be identified and isolated by screening a recombinant combinatorial immunoglobulin library (e.g., an antibody phase display library) with CCPP to thereby isolate immunoglobulin library members that bind to a CCPP. Kits for generating and screening phage display libraries are commercially available (e.g., the Pharmacia Recombinant Phage Antibody System, Catalog No. 27-9400-01; and the Stratagene SurfZAP^{™} Phage Display Kit, Catalog No. 240612).

The anti-CCPP antibodies also include "Single-chain Fv" or "scFv" antibody fragments. The scFv fragments comprise the V_{H} and V_{L} domains of an antibody, wherein these domains are present in a single polypeptide chain. Generally, the Fv polypeptide further comprises a polypeptide linker between the V_{H} and V_{L} domains which enables the scFv to form the desired structure for antigen binding.

Additionally, recombinant anti-CCPP antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art.

Humanized antibodies are particularly desirable for therapeutic treatment of human subjects. Humanized forms of non-human (e.g., murine) antibodies are chimeric molecules of immunoglobulins, immunoglobulin chains or fragments thereof (such as Fv, Fab, Fab', F(ab')₂ or other antigen-binding subsequences of antibodies), which contain minimal sequence derived from non-human immunoglobulin. Humanized antibodies include human immunoglobulins (recipient antibody) in which residues forming a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In one embodiment, the humanized antibody comprises substantially all of at least one, and typically two, variable domains, in which all or substantially all of the CDR regions correspond to those of a non-human immunoglobulin and all or substantially all of the constant regions being those of a human immunoglobulin consensus sequence. In another embodiment, the humanized antibody can also comprise at least a portion of an immunoglobulin constant region (Fc), typically that of a human immunoglobulin.

Such humanized antibodies can be produced using transgenic mice which are incapable of expressing endogenous immunoglobulin heavy and light chain genes, but which can express human heavy and light chain genes. The transgenic mice are immunized in the normal fashion with a selected antigen, e.g., all or a portion of a polypeptide corresponding to a CCPP of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies.

Humanized antibodies which recognize a selected epitope can be generated using a technique referred to as "guided selection." In this approach a selected non-human monoclonal antibody, e.g., a murine antibody, is used to guide the selection of a humanized antibody recognizing the same epitope.

In one embodiment, the antibodies to CCPP are capable of reducing or eliminating the biological function of CCPP, as is described below. That is, the addition of anti-CCPP antibodies (either polyclonal or monoclonal) to CCPP (or cells containing CCPP) can reduce or eliminate the CCPP activity. In one example, the CCPP activity is reduced by at least 25%. In another example, the CCPP activity is reduced by at least about 50%, such as 95-100%.

An anti-CCPP antibody can be used to isolate a CCPP of the invention by standard techniques, such as affinity chromatography or immunoprecipitation. An anti-CCPP antibody can facilitate the purification of natural CCPPs from cells and of recombinantly produced CCPPs expressed in host cells. Moreover, an anti-CCPP antibody can be used to detect a CCPP (e.g., in a cellular lysate or cell supernatant on the cell surface) in order to evaluate the abundance and pattern of expression of the CCPP. Anti-CCPP antibodies can be used diagnostically to monitor protein levels in tissue as part of a clinical testing procedure, for example, to determine the efficacy of a given treatment regimen. Detection can be facilitated by coupling (e.g., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Anti-CCPP antibodies of the invention are also useful for targeting a therapeutic to a cell or tissue comprising the antigen of the anti-CCPP antibody. For example, a therapeutic such as a small molecule can be linked to the anti-CCPP antibody in order to target the therapeutic to the cell or tissue comprising the CCPP antigen. The method is particularly useful in connection with CCPPs which are surface markers.

A therapeutic agent may be coupled (e.g., covalently bonded) to a suitable monoclonal antibody either directly or indirectly (*e.g.,* via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional, may be employed as the linker group. Coupling may be effected, for example, through amino groups, carboxyl groups, sulfhydryl groups or oxidized carbohydrate residues. There are numerous references describing such methodology, e.g., U.S. Pat. No. 4,671,958, to Rodwell et al*.*

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (e.g., U.S. Pat. No. 4,489,710, to Spitler), by irradiation of a photolabile bond (e.g., U.S. Pat. No. 4,626,014, to Senter et al.)*,* by hydrolysis of derivatized amino acid side chains *(e.g.,* U.S. Pat. No. 4,638,045, to Kohn et al*.*)*,* by serum complement-mediated hydrolysis *(e.g.,* U.S. Pat. No. 4,671,958, to Rodwell et al*.*)*,* and acid-catalyzed hydrolysis *(e.g.,* U.S. Pat. No. 4,569,789, to Blattler et al*.*).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers that provide multiple sites for attachment can be used.

In a specific embodiment, antibodies to a CCPP may be used to eliminate the CCPP-containing cell population *in* vivo by activating the complement system, by mediating antibody-dependent cellular cytotoxicity (ADCC), or by causing uptake of the antibody coated cells by the receptor-mediated endocytosis (RE) system.

### CCPP-specific cytotoxic lymphocytes (T cells)

Another aspect of the invention pertains to immunotherapeutic compositions comprising T cells specific for a CCPP. Such cells may generally be prepared *in vitro* or ex vivo, using standard procedures. T cells may be isolated from bone marrow, peripheral blood, or a fraction of bone marrow or peripheral blood of a patient, using a commercially available cell separation system, such as the Isolex^{™} System, available from Nexell Therapeutics, Inc. (Irvine, CA). Alternatively, T cells may be derived from related or unrelated humans, non-human mammals, cell lines or cultures.

T cells may be stimulated with a CCPP or polynucleotide encoding a CCPP or an antigen presenting cell (APC) that expresses a CCPP. Such stimulation is performed under conditions and for a time sufficient to permit the generation of T cells that are specific for the polypeptide. In one example, a CCPP or polynucleotide encoding a CCPP is present within a delivery vehicle, such as a microsphere, to facilitate the generation of specific T cells.

T cells are considered to be specific for a CCPP if the T cells specifically proliferate, secrete cytokines or kill target cells coated with the polypeptide or expressing a gene encoding the polypeptide. T cell specificity may be evaluated using any of a variety of standard techniques. For example, within a chromium release assay or proliferation assay, a stimulation index of more than two-fold increase in lysis or proliferation, compared to negative controls, indicates T cell specificity. Alternatively, detection of the proliferation of T cells may be accomplished by a variety of known techniques. For example, T cell proliferation can be detected by measuring an increased rate of DNA synthesis *(e.g.,* by pulse-labeling cultures of T cells with tritiated thymidine and measuring the amount of tritiated thymidine incorporated into DNA). Contact with a colon tumor polypeptide (e.g., 100 ng/ml - 100 µg/ml, such as 200 ng/ml - 25 µg/ml) for 3-7 days should result in at least a two-fold increase in proliferation of the T cells. Contact as described above for 2-3 hours should result in activation of the T cells, as measured using standard cytokine assays in which a two-fold increase in the level of cytokine release (*e.g.,* TNF or IFNγ) is indicative of T cell activation. T cells that have been activated in response to a CCPP, a polynucleotide encoding a CCPP, or a CCPP-expressing APC, may be CD4⁺ or CD8⁺. Colon tumor protein-specific T cells may be expanded using standard techniques. Within certain embodiments, the T cells are derived from a patient, a related donor or an unrelated donor, and are administered to the patient following stimulation and expansion.

For therapeutic purposes, CD4⁺ or CD8⁺ T cells that proliferate in response to a CCPP, a polynucleotide encoding a CCPP, or an APC can be expanded in number either in *vitro* or *in vivo.* Proliferation of such T cells *in vitro* may be accomplished in a variety of ways. For example, the T cells can be re-exposed to a CCPP, or a short peptide corresponding to an immunogenic portion of such a polypeptide, with or without the addition of T cell growth factors, such as interleukin-2, or stimulator cells that synthesize a CCPP. Alternatively, one or more T cells that proliferate in the presence of a CCPP can be expanded in number by cloning. Methods for cloning cells are well-known in the art, and include limiting dilution.

### Vaccines

Within certain aspects, CCPP, CCPN, CCPP-specific T cell, CCPP-presenting APC, and CCG-containing vectors including, but not limited to, expression vectors and gene delivery vectors, may be utilized as vaccines for colon cancer. Vaccines may comprise one or more such compounds/cells and an immunostimulant. An immunostimulant may be any substance that enhances or potentiates an immune response (antibody or cell-mediated) to an exogenous antigen. Examples of immunostimulants include adjuvants, biodegradable microspheres (*e.g.,* polylactic galactide) and liposomes (into which the compound is incorporated). Vaccines within the scope of the present invention may also contain other compounds, which may be biologically active or inactive. For example, one or more immunogenic portions of other tumor antigens may be present, either incorporated into a fusion polypeptide or as a separate compound, within the composition of vaccine.

A vaccine may contain DNA encoding one or more CCPP or portion of CCPP, such that the polypeptide is generated *in situ.* As noted above, the DNA may be present within any of a variety of delivery systems known to those of ordinary skill in the art, including nucleic acid expression vectors, gene delivery vectors, and bacteria expression systems. Numerous gene delivery techniques are well-known in the art. Appropriate nucleic acid expression systems contain the necessary DNA sequences for expression in the patient (such as a suitable promoter and terminating signal). Bacterial delivery systems involve the administration of a bacterium (such as Bacillus-Calmette-Guerrin) that expresses an immunogenic portion of the polypeptide on its cell surface or secretes such an epitope. In one embodiment, the DNA may be introduced using a viral expression system (e.g., vaccinia or other pox virus, retrovirus, or adenovirus), which may involve the use of a non-pathogenic (defective), replication competent virus. Techniques for incorporating DNA into such expression systems are well-known to those of ordinary skill in the art. The DNA may also be "naked," as described, for example, in Ulmer et al., (Science, 259:1745-1749, 1993). The uptake of naked DNA may be increased by coating the DNA onto biodegradable beads, which are efficiently transported into the cells. A vaccine may comprise both a polynucleotide and a polypeptide component. Such vaccines may provide for an enhanced immune response.

A vaccine may contain pharmaceutically acceptable salts of the polynucleotides and polypeptides provided herein. Such salts may be prepared from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

Any of a variety of immunostimulants may be employed in the vaccines of this invention. For example, an adjuvant may be included. Many adjuvants contain a substance designed to protect the antigen from rapid catabolism, such as aluminum hydroxide or mineral oil, and a stimulator of immune responses, such as lipid A, Bortadellci pertussis or Mycobacterium tuberculosis derived proteins. Suitable adjuvants are commercially available as, for example, Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable micro spheres; monophosphoryl lipid A and quil A. Cytokines, such as GM-CSF or IL-2, IL-7, or IL-12, may also be used as adjuvants.

Within the vaccines provided herein, the adjuvant composition can be, for example, designed to induce an immune response predominantly of the Th1 type or Th2-type. High levels of Th1-type cytokines (e.g., IFNγ, TNFα, IL-2 and IL-12) tend to favor the induction of cell mediated immune responses to an administered antigen. High levels of Th2-type cytokines (e.g., IL-4, IL-5, IL-6 and IL-10) tend to favor the induction of humoral immune responses. Following application of a vaccine as provided herein, a patient will support an immune response that includes Th1- or Th2-type responses. In one embodiment, a response is predominantly Thl-type, and the level of Th1-type cytokines increase to a greater extent than the level of Th2-type cytokines. The levels of these cytokines can be readily assessed using standard assays.

Adjuvants for use in eliciting a predominantly Th1-type response include, but are not limited to, a combination of monophosphoryl lipid A (e.g., 3-de-O-acylated monophosphoryl lipid A (3D-MPL)) together with an aluminum salt. MPL adjuvants are available from Corixa Corporation (Seattle, WA). CpG-containing oligonucleotides (in which the CpG dinucleotide is unmethylated) also induce a predominantly Th1 response. Such oligonucleotides are well-known. Immunostimulatory DNA sequences are also described, for example, by Sato et al., Science, 273:352, 1996. Another suitable adjuvant is a saponin, such as QS21 (Aquila Biopharmaceuticals Inc., Framingham, Mass.), which may be used alone or in combination with other adjuvants. For example, an enhanced system involves the combination of a monophosphoryl lipid A and saponin derivative, such as the combination of QS21 and 3D-MPL as described in WO94/00153, or a less reactogenic composition where the QS21 is quenched with cholesterol, as described in WO96/33739. In another example, the formulation of the present invention includes an oil-in-water emulsion and tocopherol. A particularly potent adjuvant formulation involving QS21, 3D-MPL and tocopherol in an oil-in-water emulsion is described in WO 95/17210.

Other suitable adjuvants include, but are not limited to, Montanide ISA 720 (Seppic, France), SAF (Chiron, CA), ISCOMS (CSL), MF-59 (Chiron, CA), the SBAS series of adjuvants (*e.g.,* SBAS-2 or SBAS-4, available from SmithKline Beecham, Rixensart, Belgium), Detox (Ribi ImmunoChem Research Inc., Hamilton, MT), RC-529 (Ribi ImmunoChem Research Inc., Hamilton, Mont.) and Aminoalkyl glucosaminide 4-phosphates (AGPs).

Any vaccine provided herein may be prepared using well-known methods that result in a combination of antigen, immune response enhancer and a suitable carrier or excipient. The compositions described herein may be administered as part of a sustained release formulation (*e.g.,* a formulation such as a capsule, sponge or gel (composed of polysaccharides, for example) that effects a slow release of compound following administration). Such formulations may generally be prepared using well-known technology and administered by, for example, oral, rectal or subcutaneous implantation, or by implantation at the desired target site. Sustained-release formulations may contain a polypeptide, polynucleotide or antibody dispersed in a carrier matrix or contained within a reservoir surrounded by a rate controlling membrane.

Carriers for use within such formulations are biocompatible, and may also be biodegradable. In one example, the formulation provides a relatively constant level of active component release. Such carriers include microparticles of poly (lactide-co-glycolide), as well as polyacrylate, latex, starch, cellulose and dextran. Other delayed-release carriers include supramolecular biovectors, which comprise a non-liquid hydrophilic core (*e.g.,* a cross-linked polysaccharide or oligosaccharide) and, optionally, an external layer comprising an amphiphilic compound, such as a phospholipid (see *e.g.,* U.S. Pat. No. 5,151,254). The amount of active compound contained within a sustained release formulation depends upon the site of implantation, the rate and expected duration of release and the nature of the condition to be treated or prevented.

Any of a variety of delivery vehicles may be employed within vaccines to facilitate production of an antigen-specific immune response that targets cancer cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation or maintenance of the T cell response, to have anti-tumor effects per se or to be immunologically compatible with the receiver (*e.g.,* matched HLA haplotype). APCs may generally be isolated from any of a variety of biological fluids and organs, including tumor and peritumoral tissues, and may be autologous, allogeneic, syngeneic or xenogeneic cells.

. Certain embodiments of the present invention use dendritic cells or progenitors thereof as APCs. Dendritic cells are highly potent APCs and have been shown to be effective as a physiological adjuvant for eliciting prophylactic or therapeutic antitumor immunity. Dendritic cells can be identified based on their typical shape (stellate *in situ,* with marked cytoplasmic processes (dendrites) visible *in vitro*), their ability to take up, process and present antigens with high efficiency and their ability to activate naive T cell responses. Dendritic cells may, of course, be engineered to express specific cell-surface receptors or ligands that are not commonly found on dendritic cells *in vivo,* and such modified dendritic cells are contemplated by the present invention. As an alternative to dendritic cells, secreted vesicles antigen-loaded dendritic cells (called exosomes) may be used within a vaccine (see Zitvogel et al., Nature Med., 4:594-600,1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand or other compound(s) that induce differentiation, maturation and proliferation of dendritic cells.

Dendritic cells are conveniently categorized as "immature" and "mature" cells. This categorization provides a simple way to discriminate between two well-characterized phenotypes. However, this nomenclature should not be construed to exclude all possible intermediate stages of differentiation. Immature dendritic cells are characterized as APC with a high capacity for antigen uptake and processing, which correlates with the high expression of Fcy receptor and mannose receptor. The mature phenotype is typically characterized by a lower expression of these markers, but a high expression of cell surface molecules responsible for T cell activation such as class and class II MHC, adhesion molecules (*e.g.,* CD54 and CD11) and costimulatory molecules (*e.g.*, CD40, CD80, CD86 and 4-1BB).

APCs can be transfected with a polynucleotide encoding a CCPP (or portion or other variant thereof) such that the CCPP, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, can be performed using any suitable methods known in the art, such as those described in WO97/24447, or the gene gun approach described by Mahvi et al., Immunology and Cell Biology, 75:456-460, 1997. Antigen loading of dendritic cells may be achieved by incubating dendritic cells or progenitor cells with the CCPPs, DNA or RNA; or with antigen-expressing recombinant bacterium or viruses (*e.g.,* vaccinia, fowlpox, adenovirus or lentivirus vectors). Prior to loading, the polypeptide may be covalently conjugated to an immunological partner that provides T cell help (e.g., a carrier molecule). Alternatively, a dendritic cell may be pulsed with a nonconjugated immunological partner, separately or in the presence of the polypeptide.

Vaccines may be presented in unit-dose or multi-dose containers, such as sealed ampoules or vials. Such containers can be, for example, hermetically sealed to preserve sterility of the formulation until use. Formulations can be stored as suspensions, solutions, or emulsions in oily or aqueous vehicles. Alternatively, a vaccine may be stored in a freeze-dried condition requiring only the addition of a sterile liquid carrier immediately prior to use.

### Vectors

Another aspect of the invention pertains to vectors containing polynucleotides encoding CCPPs or portions thereof. Vectors can be plasmids or viral vectors.

The expression vectors of the invention can be designed for expression of CCPPs in prokaryotic or eukaryotic cells. For example, CCPPs can be expressed in bacterial cells such as E. *coli,* insect cells (using baculovirus expression vectors), yeast cells, or mammalian cells. In certain embodiments, such protein may be used, for example, as a therapeutic protein of the invention. Alternatively, the expression vector can be transcribed and translated *in vitro,* for example using T7 promoter regulatory sequences and T7 polymerase.

In another embodiment, mammalian expression vector including tissue-specific regulatory elements are used to express the polynucleotides of interest. Tissue-specific regulatory elements are known in the art and may include epithelial cell-specific promoters. Other non-limiting examples of suitable tissue-specific promoters include the liver-specific albumin promoter, lymphoid-specific promoters, promoters of T cell receptors and immunoglobulins, neuron-specific promoters (e.g., the neurofilament promoter), pancreas-specific promoters, and mammary gland-specific promoters (e.g., milk whey promoter). Developmentally-regulated promoters are also encompassed, for example the α-fetoprotein promoter.

The CCGs identified in the present invention can be used for therapeutical purposes. For example, antisense constructs of the CCGs can be delivered therapeutically to cancer cells. The goal of such therapy is to retard the growth rate of the cancer cells. Expression of the sense molecules and their translation products or expression of the antisense mRNA molecules has the effect of inhibiting the growth rate of cancer cells or inducing apoptosis (a radical reduction in the growth rate of a cell).

The invention also provides a recombinant expression vector comprising a polynucleotide encoding a CCPP cloned into the expression vector in an antisense orientation. That is, the DNA molecule is operatively linked to a regulatory sequence in a manner which allows for expression (by transcription of the DNA molecule) of an RNA molecule which is antisense to mRNA corresponding to a CCG of the invention. Regulatory sequences operatively linked to a polynucleotide cloned in the antisense orientation can be chosen to direct the continuous expression of the antisense RNA molecule in a variety of cell types. For instance viral promoters or enhancers, or regulatory sequences can be chosen to direct constitutive, tissue specific or cell type specific expression of antisense RNA. The antisense expression vector can be in the form of a recombinant plasmid, phagemid or attenuated virus in which antisense polynucleotides are produced under the control of a high efficiency regulatory region. The activity of the promoter/enhancer can be determined by the cell type into which the vector is introduced.

The invention further provides gene delivery vehicles for delivery of polynucleotides to cells, tissues, or a mammal for expression. For example, a polynucleotide sequence of the invention can be administered either locally or systemically in a gene delivery vehicle. These constructs can utilize viral or non-viral vector approaches in *in vivo* or ex *vivo* modality. Expression of such coding sequence can be induced using endogenous mammalian or heterologous promoters. Expression of the coding sequence *in vivo* can be either constituted or regulated. The invention includes gene delivery vehicles capable of expressing the contemplated polynucleotides. The gene delivery vehicle can be, for example, a viral vector, such as a retroviral, lentiviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector.

Delivery of the gene therapy constructs of this invention into cells is not limited to the above mentioned viral vectors. Other delivery methods and media may be employed such as, for example, nucleic acid expression vectors, polycationic condensed DNA linked or unlinked to killed adenovirus alone, ligand linked DNA, liposome-DNA complex, eukaryotic cell delivery vehicles cells, deposition of photopolymerized hydrogel materials, handheld gene transfer particle gun, ionizing radiation, nucleic charge neutralization or fusion with cell membranes. Particle mediated gene transfer may be employed. Briefly, the sequence can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose or transferrin. Naked DNA may also be employed. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Another aspect of the invention pertains to the expression of CCGs using a regulatable expression system. These systems include, but are not limited to, the Tet-on/off system, the Ecdysone system, the Progesterone-system, and the Rapamycin-system.

Another aspect of the invention pertains to the use of host cells which are transformed, transfected, or transduced with vectors encoding or comprising CCGs or portions thereof. The host cells can be prokaryotic or eukaryotic cells. These host cells can be employed to express any desired CCPP.

### Transgenic and Knockout Animals

The host cells of the invention can also be used to produce non-human transgenic animals. For example, in one embodiment, a host cell of the invention is a fertilized oocyte or an embryonic stem cell into which CCPP-coding sequences have been introduced. Such host cells can then be used to create non-human transgenic animals in which exogenous sequences encoding a CCPP of the invention have been introduced into their genome or homologous recombinant animals in which endogenous sequences encoding the CCPP of the invention have been altered. Such animals are useful for studying the function or activity of a CCPP and for identifying or evaluating modulators of CCPP activity. A "transgenic animal" is a non-human animal, such as a rodent (*e.g.,* a rat or mouse), in which one or more of the cells of the animal includes a transgene. Other examples of transgenic animals include non-human primates, sheep, dogs, cows, goats, chickens, amphibians, and the like. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, a "homologous recombinant animal" is a non-human animal, such a mammal (*e.g.,* a mouse), in which an endogenous CCG of the invention (e.g., listed in Tables 1-5) has been altered by homologous recombination between the endogenous gene and an exogenous DNA molecule introduced into a cell of the animal, e.g., an embryonic cell of the animal, prior to development of the animal.

A transgenic animal of the invention can be created by introducing a CCPP-encoding polynucleotide into the mate pronuclei of a fertilized oocyte, *e.g.,* by microinjection or retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to a transgene to direct expression of a CCPP to particular cells. Methods for generating transgenic animals via embryo manipulation and microinjection, particularly animals such as mice, have become conventional in the art. Similar methods are used for production of other transgenic animals. A transgenic founder animal can be identified based upon the presence of a transgene of the invention in its genome or expression of mRNA corresponding to a gene of the invention in tissues or cells of the animals. A transgenic founder animal can then be used to breed additional animals carrying the transgene. Moreover, transgenic animals carrying a transgene encoding a CCPP can further be bred to other transgenic animals carrying other transgenes.

To create a homologous recombinant animal (knockout animal), a vector is prepared which contains at least a portion of a gene of the invention into which a deletion, addition or substitution has been introduced to thereby alter, e.g., functionally disrupt, the gene. The gene can be a human gene, or a non-human homolog thereof (e.g., a homolog of a CCG listed in Tables 1-5). For example, a mouse gene can be used to construct a homologous recombination polynucleotide molecule, e.g., a vector, suitable for altering an endogenous gene of the invention in the mouse genome. In one embodiment, the homologous recombination polynucleotide molecule is designed such that, upon homologous recombination, the endogenous gene of the invention is functionally disrupted (*e.g.,* no longer encodes a functional protein; also referred to as a "knockout" vector). Alternatively, the homologous recombination polynucleotide molecule can be designed such that, upon homologous recombination, the endogenous gene is mutated or otherwise altered but still encodes functional protein (e.g., the upstream regulatory region can be altered to thereby alter the expression of the endogenous CCPP). In the homologous recombination polynucleotide molecule, the altered portion of the gene of the invention is flanked at its 5' and 3' ends by additional polynucleotide sequence of the gene of the invention to allow for homologous recombination to occur between the exogenous gene carried by the homologous recombination polynucleotide molecule and an endogenous gene in a cell, *e.g.,* an embryonic stem cell. The additional flanking polynucleotide sequence is of sufficient length for successful homologous recombination with the endogenous gene.

Typically, several kilobases of flanking DNA (both at the 5' and 3' ends) are included in the homologous recombination polynucleotide molecule. The homologous recombination polynucleotide molecule is introduced into a cell, e.g., an embryonic stem cell line (e.g., by electroporation) and cells in which the introduced gene has homologously recombined with the endogenous gene are selected. The selected cells can then be injected into a blastocyst of an animal (*e.g*., a mouse) to form aggregation chimeras. A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination polynucleotide molecules are well-known in the art.

In another embodiment, transgenic non-human animals can be produced which contain selected systems which allow for regulated expression of the trans gene. One example of such a system is the *cre*/*loxP* recombinase system of bacteriophage P1. For a description of the *cre*/*loxP* recombinase system, see, *e.g.,* Laksa et al., Proc. Natl. Acad. Sci., USA, 89:6232-6236, 1992. Another example of a recombinase system is the FLP recombinase system of *Saccharomyces cerevisiae* (O'Gorman et al., Science, 251:1351-1355, 1991). If a *cre*/*loxP* recombinase system is used to regulate expression of the transgene, animals containing transgenes encoding both the *Cre* recombinase and a selected protein are required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut, I. et al., Nature, 385:810-813, 1997, and PCT International Publication Nos. WO97/07668 and WO97/07669. In brief, a cell, e.g., a somatic cell, from the transgenic animal can be isolated and induced to exit the growth cycle and enter Go phase. The quiescent cell can then be fused, *e.g.,* through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell, *e.g.,* the somatic cell, is isolated.

In certain embodiments of the invention, the non-human transgenic animals comprise a CCG, such as, for example, STK15. In some other embodiments, the non-human "knock-out" transgenic animal is a STK15 knock-out.

### Detection Methods

As discussed earlier, expression level of CCGs may be used as a marker for colon cancer. Detection and measurement of the relative amount of a CCG product (polynucleotide or polypeptide) of the invention can be carried out by any method known in the art.

Typical methodologies for detection of a transcribed polynucleotide include RNA extraction from a cell or tissue sample, followed by hybridization of a labeled probe (*e.g.*, a complementary polynucleotide molecule) specific for the target RNA to the extracted RNA and detection of the probe (*e.g.*, Northern blotting).

Typical methodologies for peptide detection include protein extraction from a cell or tissue sample, followed by binding of an antibody specific for the target protein to the protein sample, and detection of the antibody. For example, detection of STK15 may be accomplished using polyclonal anti-STK15 antibody. Antibodies can be detected by the use of a labeled secondary antibody. The label can be a radioisotope, a fluorescent compound, an enzyme, an enzyme co-factor, or ligand. Such methods are well understood in the art.

In certain embodiments, the CCGs themselves may serve as markers for colon cancer. For example, an increase of genomic copies of a CCG, such as by duplication of the gene, may also be correlated with colon cancer.

Detection of specific polynucleotide molecules may also be assessed by gel electrophoresis, column chromatography, or direct sequencing, quantitative PCR (in the case of polynucleotide molecules), RT-PCR, or nested-PCR among many other techniques well-known to those skilled in the art.

Detection of the presence or number of copies of all or a part of a CCG of the invention may be performed using any method known in the art. Typically, it is convenient to assess the presence or quantity of a DNA or cDNA by Southern analysis, in which total DNA from a cell or tissue sample is extracted, is hybridized with a labeled probe (*e.g.,* a complementary DNA molecules), and the probe is detected. The label group can be a radioisotope, a fluorescent compound, an enzyme, or an enzyme co-factor. Other useful methods of DNA detection or quantification include direct sequencing, gel electrophoresis, column chromatography, and quantitative PCR, as is known by one skilled in the art.

In certain embodiments, the CCPPs may serve as markers for colon cancer. Detection of specific polypeptide molecules may be assessed by gel electrophoresis, Western blot, column chromatography, or direct sequencing, among many other techniques well-known to those skilled in the art.

### Panels of CCGs

Expression level of each CCG may be considered individually, although it is within the scope of the invention to provide combinations of two or more CCGs for use in the methods and compositions of the invention to increase the confidence of the analysis. In another aspect, the invention provides panels of the CCGs of the invention. A panel of CCGs comprises two or more CCGs. A panel may also comprise 2-5, 5-15, 15-35, 35-50, or more than 50 CCG_{S}. In one embodiment, these panels of CCGs are selected such that the CCGs within any one panel share certain features. For example, the CCGs of a first panel may be protein kinases that exhibit at least a two-fold increase in quantity or activity in a colon cancer sample, as compared to a sample which is substantially free of colon cancer from the same subject or a sample which is substantially free of colon cancer from a different subject without colon cancer. Alternatively, CCGs of a second panel may each exhibit differential regulation as compared to a first panel. Similarly, different panels of CCGs may be composed of CCGs representing different stages of colon cancer. Panels of the CCGs of the invention may be made by independently selecting CCGs from Tables 1-5, and may further be provided on biochips, as discussed below.

### Screening Methods

The invention also provides methods (also referred to herein as "screening assays") for identifying modulators, *e.g.,* candidate or test compounds or agents comprising therapeutic moieties (e.g., peptides, peptidomimetics, peptoids, polynucleotides, small molecules or other drugs) which (a) bind to a CCPP, or (b) have a modulatory (*e.g.*, stimulatory or inhibitory) effect on the activity of a CCPP or, more specifically, (c) have a modulatory effect on the interactions of the CCPP with one or more of its natural substrates, or (d) have a modulatory effect on the expression of the CCPPs. Such assays typically comprise a reaction between the CCPP and one or more assay components. The other components may be either the test compound itself, or a combination of the test compound and a binding partner of the CCPP.

The test compounds of the present invention can be, for instance, small molecules or biomolecules. Small molecules include, but are not limited to, inorganic molecules and small organic molecules. Biomolecules include, but are not limited to, naturally-occurring and synthetic compounds that have a bioactivity in mammals, such as polypeptides, polysaccharides, and polynucleotides. In one embodiment the test compound is a small molecule. In another embodiment, the test compound is a biomolecule. One skilled in the art will appreciate that the nature of the test compound may vary depending on the nature of the protein encoded by the CCG of the invention. For example, if the CCG encodes an orphan receptor having an unknown ligand, the test compound may be any of a number of biomolecules which may act as cognate ligand, including but not limited to, cytokines, lipid-derived mediators, small biogenic amines, hormones, neuropeptides, or proteases.

The test compounds of the present invention may be obtained from any available source, including systematic libraries of natural or synthetic compounds. Test compounds may also be obtained by any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, *e.g.,* Zuckermann et al., J. Med. Chem., 37:2678-85, 1994); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, Anticancer Drug Des., 12:145, 1997).

### Screening for Inhibitors of CCPP

The invention provides methods of screening test compounds for inhibitors of CCPP, and to the pharmaceutical compositions comprising the test compounds. The method of screening comprises obtaining samples from subjects diagnosed with or suspected of having colon cancer, contacting each separate aliquot of the samples with one of a plurality of test compounds, and comparing expression of one or more CCGs in each of the aliquots to determine whether any of the test compounds provides a substantially decreased level of expression or activity of a CCG relative to samples with other test compounds or relative to an untreated sample or control sample. In addition, methods of screening may be devised by combining a test compound with a protein and thereby determining the effect of the test compound on the protein.

In addition, the invention is further directed to a method of screening for test compounds capable of modulating with the binding of a CCPP and a binding partner, by combining the test compound, CCPP, and binding partner together and determining whether binding of the binding partner and CCPP occurs. The test compound may be either small molecules or a biomolecule. As discussed below, test compounds may be provided from a variety of libraries well-known in the art.

Modulators of a CCG expression, activity or binding ability are useful as therapeutic compositions of the invention. Such modulators (e.g., antagonists or agonists) may be formulated as pharmaceutical compositions, as described herein below. Such modulators may also be used in the methods of the invention, for example, to diagnose, treat, or prognose colon cancer.

### High-Throughput Screening Assays

The invention provides methods of conducting high-throughput screening for test compounds capable of inhibiting activity or expression of a CCPP of the present invention. In one embodiment, the method of high-throughput screening involves combining test compounds and the CCPP and detecting the effect of the test compound on the CCPP.

A variety of high-throughput functional assays well-known in the art may be used in combination to screen or study the reactivity of different types of activating test compounds. Since the coupling system is often difficult to predict, a number of assays may need to be configured to detect a wide range of coupling mechanisms. A variety of fluorescence-based techniques are well-known in the art and are capable of high-throughput and ultra high throughput screening for activity, including but not limited to BRET^{®} or FRET^{®} (both by Packard Instrument Co., Meriden, CT). The ability to screen a large volume and a variety of test compounds with great sensitivity permits for analysis of the therapeutic targets of the invention to further provide potential inhibitors of colon cancer. For example, where the CCG encodes an orphan receptor with an unidentified ligand, high-throughput assays may be utilized to identify the ligand, and to further identify test compounds which prevent binding of the receptor to the ligand. The BIACORE^{®} system may also be manipulated to detect binding of test compounds with individual components of the therapeutic target, to detect binding to either the encoded protein or to the ligand.

By combining test compounds with CCPPs of the invention and determining the binding activity between such, diagnostic analysis can be performed to elucidate the coupling systems. Generic assays using cytosensor microphysiometer may also be used to measure metabolic activation, while changes in calcium mobilization can be detected by using the fluorescence-based techniques such as FLIPR^{®} (Molecular Devices Corp, Sunnyvale, CA). In addition, the presence of apoptotic cells may be determined by TUNEL assay, which utilizes flow cytometry to detect free 3-OH termini resulting from cleavage of genomic DNA during apoptosis. As mentioned above, a variety of functional assays well-known in the art may be used in combination to screen or study the reactivity of different types of activating test compounds. In one example, the high-throughput screening assay of the present invention utilizes label-free plasmon resonance technology as provided by BIACORE^{®} systems (Biacore International AB, Uppsala, Sweden). Plasmon free resonance occurs when surface plasmon waves are excited at a metal/liquid interface. By reflecting directed light from the surface as a result of contact with a sample, the surface plasmon resonance causes a change in the refractive index at the surface layer. The refractive index change for a given change of mass concentration at the surface layer is similar for many bioactive agents (including proteins, peptides, lipids and polynucleotides), and since the BIACORE^{®} sensor surface can be functionalized to bind a variety of these bioactive agents, detection of a wide selection of test compounds can thus be accomplished.

Therefore, the invention provides for high-throughput screening of test compounds for the ability to inhibit activity of a protein encoded by CCGs, such as those listed in Tables 1-5, by combining the test compounds and the protein in high-throughput assays such as BIACORE^{®}, or in fluorescence-based assays such as BRET^{®}. In addition, high-throughput assays may be utilized to identify specific factors which bind to the encoded proteins, or alternatively, to identify test compounds which prevent binding of the receptor to the binding partner. In the case of orphan receptors, the binding partner may be the natural ligand for the receptor. Moreover, the high-throughput screening assays may be modified to determine whether test compounds can bind to either the encoded protein or to the binding partner (e.g., substrate or ligand) which binds to the protein.

In a specific embodiment, the high-throughput screening assay detects the ability of a plurality of test compounds to bind to a Group I gene product. In another specific embodiment, the high-throughput screening assay detects the ability of a plurality of a test compound to inhibit a binding partner (such as a ligand) to bind to a Group I gene product. In yet another specific embodiment, the high-throughput screening assay detects the ability of a plurality of a test compounds to modulate signaling through a Group I gene product.

### Predictive Medicine

The present invention pertains to the field of predictive medicine in which diagnostic assays, prognostic assays, pharmacogenetics and monitoring clinical trials are used for prognostic (predictive) purpose to thereby treat an individual prophylactically. Accordingly, one aspect of the present invention relates to diagnostic assays for determining CCG polynucleotide or polypeptide expression or activity, in the context of a biological sample (e.g., blood, serum, cells, tissue) to thereby determine whether an individual is at risk for developing colon cancer associated with modulated CCG expression or activity. The invention also provides for prognostic (or predictive) assays for determining whether an individual is at risk of developing colon cancer associated with aberrant CCG protein or polynucleotide expression or activity.

For example, the number of copies of a CCG can be assayed in a biological sample. Such assays can be used for prognostic or predictive purposes to thereby prophylactically treat an individual prior to the onset of colon cancer associated with aberrant CCG protein, polynucleotide expression or activity.

Another aspect of the invention pertains to monitoring the influence of agents (e.g., drugs, compounds) on the expression or activity of CCGs in clinical trials.

### Diagnostic Assays

An exemplary method for detecting the presence or absence of a CCPP or polynucleotide encoding a CCPP in a biological sample involves contacting a biological sample with a compound or an agent capable of detecting the CCPP or polynucleotide (e.g., mRNA, genomic DNA) that encodes the CCPP such that the presence of the CCPP or polynucleotide is detected in the biological sample. An example agent for detecting mRNA or genomic DNA corresponding to a CCG or CCPP of the invention is a labeled polynucleotide probe capable of hybridizing to an mRNA or genomic DNA of the invention. In one embodiment, the polynucleotides to be screened are arranged on a GeneChip®. Suitable probes for use in the diagnostic assays of the invention are described herein. An example agent for detecting a CCPP of the invention is an antibody which specifically recognizes the CCPP.

The diagnostic assays may also be used to quantify the amount of expression or activity of a CCG in a biological sample. Such quantification is useful, for example, to determine the progression or severity of colon cancer. Such quantification is also useful, for example, to determine the severity of colon cancer following treatment.

### Determining severity of colon cancer

In the field of diagnostic assays, the invention also provides methods for determining the severity of colon cancer by isolating a sample from a subject (*e.g.,* a colon biopsy), detecting the presence, quantity or activity of one or more CCGs of the invention in the sample relative to a second sample from a normal sample or control sample. In one embodiment, the expression levels of CCGs in the two samples are compared, and a modulation in one or more CCGs in the test sample indicates colon cancer. In other embodiments, modulations of 2, 3, 4 or more CCGs indicate a severe case of colon cancer.

In another aspect, the invention provides CCGs whose quantity or activity is correlated with the severity of colon cancer. The subsequent level of expression may further be compared to different expression profiles of various stages of the cancer to confirm whether the subject has a matching profile. In yet another aspect, the invention provides CCGs whose quantity or activity is correlated with a risk in a subject for developing colon cancer.

An example agent for detecting CCPP is an antibody capable of binding to CCPP. In one example, the antibody is conjugated with a detectable label. Antibodies can be polyclonal or monoclonal. An intact antibody, or a fragment thereof (*e.g.,* Fab or F(ab')₂) can be used. The term "labeled," with regard to the probe or antibody, is intended to encompass direct labeling of the probe or antibody by coupling (*e.g.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present-within a subject. That is, the detection method of the invention can be used to detect CCG mRNA, protein or genomic DNA in a biological sample *in vitro* as well as *in vivo.* For example, *in vitro* techniques for detection of CCG mRNA include Northern hybridizations and *in situ* hybridizations. *In vitro* techniques for detection of CCPP include enzyme linked immunosorbent assays (ELISAs), Western blots, immunoprecipitations and immunofluorescence. *In vitro* techniques for detection of CCG genomic DNA include Southern hybridizations. Furthermore, *in vivo* techniques for detection of CCPP include introducing into a subject a labeled anti-CCPP antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. In one example, the biological sample is a serum sample isolated by conventional means from a subject, e.g., a biopsy or blood draw.

In another embodiment, the methods further involve obtaining a control biological sample from a subject, contacting the control sample with a compound or agent capable of detecting CCG protein, mRNA, or genomic DNA, such that the presence of CCG protein, mRNA or genomic DNA is detected in the biological sample, and comparing the presence of CCG protein, mRNA or genomic DNA in the control sample with the presence of CCG protein, mRNA or genomic DNA in the test sample.

### Detection of CCPP Specific T cells

Colon cancer may also be detected based on the presence of T cells that specifically react with a CCPP in a biological sample. Within certain methods, a biological sample comprising CD4⁺ or CD8⁺ T cells isolated from a patient is incubated with a CCPP, a polynucleotide encoding such a polypeptide or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with polypeptide (*e.g.,* 5-25 µg/ml). It may be desirable to incubate another aliquot of a T cell sample in the absence of colon tumor polypeptide to serve as a control. For CD4⁺ T cells, activation can be detected, for example, by evaluating proliferation of the T cells. For CD8⁺ T cells, activation can be detected, for example, by evaluating cytolytic activity. A level of proliferation that is at least two-fold greater or a level of cytolytic activity that is at least 20% greater than in disease-free patients indicates the presence of colon cancer in the patient.

### Prognostic Assays

The diagnostic method described herein can furthermore be utilized to identify subjects having or at risk of developing colon cancer associated with aberrant CCG expression or activity.

The assays described herein, such as the preceding or following assays, can be utilized to identify a subject having colon cancer associated with an aberrant level of CCG activity or expression. Alternatively, the prognostic assays can be utilized to identify a subject at risk for developing colon cancer associated with aberrant levels of CCG protein activity or polynucleotide expression. Thus, the present invention provides a method for identifying colon cancer associated with aberrant CCG expression or activity in which a test sample is obtained from a subject and CCG protein or polynucleotide (*e.g.,* mRNA or genomic DNA) is detected, wherein the presence of CCG protein or polynucleotide is diagnostic or prognostic for a subject having or at risk of developing colon cancer with aberrant CCG expression or activity.

Furthermore, the prognostic assays described herein can be used to determine whether a subject can be administered an agent (e.g., an agonist, antagonist, peptidomimetic, protein, peptide, polynucleotide, small molecule, or other drug candidate) to treat or prevent colon cancer associated with aberrant CCG expression or activity, such as, for example, a cytokine. For example, such methods can be used to determine whether a subject can be effectively treated with an agent to inhibit colon cancer. Thus, the present invention provides methods for determining whether a subject can be effectively treated with an agent for colon cancer associated with increased CCG expression or activity in which a test sample is obtained and CCG protein or polynucleotide expression or activity is detected (e.g., wherein the abundance of CCG protein or polynucleotide expression or activity is diagnostic for a subject that can be administered the agent to treat injury associated with aberrant CCG expression or activity).

Prognostic assays can be devised to determine whether a subject undergoing treatment for colon cancer has a poor outlook for long term survival or disease progression. In one embodiment, prognosis can be determined shortly after diagnosis, e.g., within a few days. By establishing expression profiles of different stages of CCGs, from onset to later stages, an expression pattern may emerge to correlate a particular expression profile to increased likelihood of a poor prognosis. The prognosis may then be used to devise a more aggressive treatment program and enhance the likelihood of long-term survival and well-being.

The methods of the invention can also be used to detect genetic alterations in a CCG, thereby determining if a subject with the altered gene is at risk for damage characterized by aberrant regulation in CCG protein activity or polynucleotide expression. In certain embodiments, the methods include detecting, in a sample of cells from the subject, the presence or absence of a genetic alteration characterized by at least one alteration affecting the integrity of a CCG, or the aberrant expression of the CCG. For example, such genetic alterations can be detected by ascertaining the existence of at least one of the following: 1) deletion of one or more nucleotides from a CCG; 2) addition of one or more nucleotides to a CCG; 3) substitution of one or more nucleotides of a CCG; 4) a chromosomal rearrangement of a CCG; 5) alteration in the level of a messenger RNA transcript of a CCG; 6) aberrant modification of a CCG, such as of the methylation pattern of the genomic DNA; 7) the presence of a non-wild-type splicing pattern of a messenger RNA transcript of a CCG; 8) non-wild-type level of a CCG protein; 9) allelic loss of a CCG; and 10) inappropriate post-translational modification of a CCG protein. As described herein, there are a large number of assays known in the art which can be used for detecting alterations in a CCG. A biological sample can be, without limitation, a blood sample isolated by conventional means from a subject.

In some other embodiments, detection of the alteration involves the use of a probe/primer in a polymerase chain reaction (PCR), such as anchor PCR or RACE PCR, or, alternatively, in a ligation chain reaction (LCR), the latter of which can be particularly useful for detecting point mutations in the CCG. This method can include the steps of collecting a cell sample of from a subject, isolating a polynucleotide sample (e.g., genomic, mRNA or both) from the cell sample, contacting the polynucleotide sample with one or more primers which specifically hybridize to a CCG under conditions such that hybridization and amplification of the CCG (if present) occur, and detecting the presence or absence of an amplification product, or detecting the size of the amplification product and comparing the length to a control sample. It is understood that PCR or LCR may be desirable to use as a preliminary amplification step in conjunction with any of the techniques used for detecting mutations described herein.

Alternative amplification methods include: self sustained sequence replication, transcriptional amplification system, Q-Beta Replicase, or any other polynucleotide amplification method, followed by the detection of the amplified molecules using techniques well-known to those of skill in the art. These detection schemes are especially useful for the detection of polynucleotide molecules if such molecules are present in very low numbers.

In an alternative embodiment, mutations in a CCG from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicate mutations in the sample DNA. Moreover, sequence specific ribozymes (see, for example, U.S. Patent No. 5,498,531) can be used to score for the presence of specific mutations by development or loss of a ribozyme cleavage site.

In other embodiments, genetic mutations in a CCG can be identified by hybridizing sample and control polynucleotides, e.g., DNA or RNA, to high density arrays containing hundreds or thousands of oligonucleotide probes. For example, genetic mutations in a CCG can be identified in two dimensional arrays containing light generated DNA probes. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the CCG and detect mutations by comparing the sequence of the sample CCG with the corresponding wild-type (control) sequence. It is also contemplated that any of a variety of automated sequencing procedures can be utilized when performing the diagnostic assays, including sequencing by mass spectrometry.

Other methods for detecting mutations in a CCG include methods in which protection from cleavage agents is used to detect mismatched bases in RNA/RNA or RNA/DNA heteroduplexes. The art technique of "mismatch cleavage" typically starts by providing heteroduplexes by hybridizing (labeled) RNA or DNA containing the wild-type CCG sequence with potentially mutant RNA or DNA obtained from a tissue sample. The double-stranded duplexes are treated with an agent which cleaves single-stranded regions of the duplex, which will exist due to base pair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNADNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. In one embodiment, the control DNA or RNA can be labeled for detection.

In still another embodiment, the mismatch cleavage reaction employs one or more proteins that recognize mismatched base, pairs in double-stranded DNA (so called "DNA mismatch repair" enzymes) in defined systems for detecting and mapping point ' mutations in CCG cDNAs obtained from samples of cells. For example, the mutY enzyme of *E. coli* cleaves A at G/A mismatches and the thymidine DNA glycosylase from HeLa cells cleaves T at G/T mismatches. According to an exemplary embodiment, a probe based on a CCG sequence (*e.g.,* a wild-type CCG sequence) is hybridized to cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in CCGs. For example, single strand conformation polymorphism (SSCP) may be used to detect differences in electrophoretic mobility between mutant and wild-type polynucleotides. Single-stranded DNA fragments of sample and control CCG polynucleotides will be denatured and allowed to renature. The secondary structure of single-stranded polynucleotides varies according to sequence. The resulting alteration in electrophoretic mobility enables the detection of even a single base change. The DNA fragments may be labeled or detected with labeled probes. The sensitivity of the assay may be enhanced by using RNA (rather than DNA) in which the secondary structure is more sensitive to a change in sequence. In one embodiment, the subject method utilizes heteroduplex analysis to separate double-stranded heteroduplex molecules on the basis of changes in electrophoretic mobility (Keen et al., Trends Genet., 7:5, 1991).

In yet another embodiment the movement of mutant or wild-type fragments in polyacrylamide gels containing a gradient of denaturant is assayed using denaturing gradient gel electrophoresis (DGGE). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control, and sample DNA (Rosenbaum and Reissner, Biophys. Chem., 265:12753, 1987).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al., Proc. Natl. Acad. Sci., USA, 86:6230, 1989). Such allele specific oligonucleotides are hybridized to PCR amplified target or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) or at the extreme 3' end of one primer where, under appropriate conditions, mismatch can prevent or reduce polymerase extension. In addition, it may be desirable to introduce a novel restriction site in the region of the mutation to create cleavage-based detection. It is anticipated that, in certain embodiments, amplification may also be performed using Taq ligase for amplification. In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe polynucleotide or antibody reagent described herein, which may be conveniently used, e.g., in clinical settings to diagnose subjects exhibiting symptoms or family history of a disease or illness involving a CCG.

Furthermore, any cell type or tissue in which a CCG is expressed may be utilized in the prognostic or diagnostic assays described herein.

### Monitoring Drug Effects During Clinical Trials

Monitoring the influence of agents (e.g., drugs, small molecules and biomolecule) on the expression or activity of a CCG protein can be applied not only in basic drug screening, but also in clinical trials. For example, the effectiveness of an agent determined by a screening assay, as described herein to decrease CCG expression, protein levels, or down-regulate CCG activity, can be monitored in clinical trials of subjects exhibiting increased CCG expression, protein levels; or up-regulated CCG activity. In such clinical trials, the expression or activity of a CCG can be used as a "read out" of the phenotype of a particular tissue.

For example, and not by way of limitation, genes, including CCGs, that are modulated in tissues by treatment with an agent that modulates CCPP activity (*e.g.,* identified in a screening assay as described herein) can be identified. Thus, to study the effect of agents on CCPP-associated damage, for example, in a clinical trial, cells can be isolated and RNA prepared and analyzed for the levels of expression of a CCG. The levels of gene expression or a gene expression pattern can be quantified by Northern blot analysis, RT-PCR or GeneChip® as described herein, or alternatively by measuring the amount of protein produced, by one of the methods as described herein, or by measuring the levels of activity of CCPP. In this way, the gene expression pattern can serve as a read-out, indicative of the physiological response of the cells to the agent. Accordingly, this response state may be determined before treatment and at various points during treatment of the individual with the agent.

In one embodiment, the present invention provides a method for monitoring the effectiveness of treatment of a subject with an agent (e.g., an agonist, antagonist, peptidomimetic, biomolecule, small molecule, or other drug candidate identified by the screening assays described herein) including the steps of (i) obtaining a pre-administration sample from a subject prior to administration of the agent; (ii) detecting the level of expression of a CCG protein or mRNA in the pre-administration sample; (iii) obtaining one or more post-administration samples from the subject; (iv) detecting the level of expression or activity of the CCG protein or mRNA in the post-administration samples; (v) comparing the level of expression or activity of the CCG protein or mRNA in the pre-administration sample with the CCG protein or mRNA the post administration sample or samples; and (vi) altering the administration of the agent to the subject accordingly. For example, decreased administration of the agent may be desirable to decrease expression or activity of CCG to lower levels than detected, e.g., to decrease the effectiveness of the agent. According to such an embodiment, CCG expression or activity may be used as an indicator of the effectiveness of an agent, even in the absence of an observable phenotypic response.

### Methods of Treatment

The present invention provides for both prophylactic and therapeutic methods of treating a subject at risk for, susceptible to or diagnosed with colon cancer. With regard to both prophylactic and therapeutic methods of treatment, such treatments may be specifically tailored or modified, based on knowledge obtained from the field of pharmacogenomics. "Pharmacogenomics," as used herein, includes the application of genomics technologies such as gene sequencing, statistical genetics, and gene expression analysis to drugs in clinical development and on the market. More specifically, the term refers the study of how a subject's genes determine his or her response to a drug (e.g., a subject's "drug response phenotype" or "drug response genotype"). Thus, another aspect of the invention provides methods for tailoring an individual's prophylactic or therapeutic treatment with either the CCPP molecules of the present invention or CCPP modulators (e.g., agonists or antagonists) according to that individual's drug response. Pharmacogenomics allows a clinician or physician to target prophylactic or therapeutic treatments to subjects who will benefit from the treatment and to avoid treatment of subjects who will experience toxic drug-related side effects.

### Prophylactic Methods

In one aspect, the invention provides a method for preventing in a subject colon cancer associated with aberrant CCG expression or activity, by administering to the subject a CCG protein or an agent which modulates CCG protein expression or activity.

Subjects at risk for colon cancer which is caused or contributed to by aberrant CCG expression or activity can be identified by, for example, any or a combination of diagnostic or prognostic assays as described herein.

Administration of a prophylactic agent can occur prior to the manifestation of symptoms characteristic of the differential CCG protein expression, such that colon cancer is prevented or, alternatively, delayed in its progression. Depending on the type of CCG aberrancy (*e.g.,* typically a modulation outside the normal standard deviation), a CCG protein, CCG agonist or antagonist agent can be used for treating the subject. The appropriate agent can be determined based on screening assays described herein.

### Therapeutic Methods

Another aspect of the invention pertains to methods of modulating CCG protein expression or activity for therapeutic purposes. Accordingly, in an exemplary embodiment, the modulatory method of the invention involves contacting a cell with an agent that modulates one or more of the activities of a CCG product activity associated with the cell. An agent that modulates CCG product activity can be an agent as described herein, such as a polynucleotide (e.g., an antisense molecule) or a polypeptide (e.g., a dominant-negative mutant of a CCPP), a naturally-occurring target molecule of a CCPP (e.g., a CCPP substrate), an anti-CCPP antibody, a CCPP modulator (*e.g.,* agonist or antagonist), a peptidomimetic of a CCG protein agonist or antagonist, or other small molecules.

The invention further provides methods of modulating a level of expression of a CCG of the invention, comprising administration to a subject having colon cancer, a variety of compositions which correspond to the CCGs of the invention (e.g., those listed in Tables 1-5), including proteins or antisense oligonucleotides. The protein may be provided by further providing a vector comprising a polynucleotide encoding the protein to the cells. Alternatively, the expression levels of the CCGs of the invention may be modulated by providing an antibody, a plurality of antibodies or an antibody conjugated to a therapeutic moiety. Treatment with the antibody may further be localized to the tissue comprising colon cancer. In another aspect, the invention provides methods for localizing a therapeutic moiety to colon cancer tissue or cells comprising exposing the tissue or cells to an antibody which is specific to a protein encoded by the CCGs of the invention. This method may therefore provide a means to inhibit expression of a specific gene corresponding to a CCG (such as one selected from Tables 1-5).

### Determining Efficacy of a Test Compound or Therapy

The invention also provides methods of assessing the efficacy of a test compound or therapy for inhibiting colon cancer in a subject. These methods involve isolating samples from a subject suffering from colon cancer, who is undergoing treatment or therapy, and detecting the presence, quantity, or activity of one or more CCGs of the invention in the first sample relative to a second sample. Where the efficacy of a test compound is determined, the first and second samples can be, for example, sub-portions of a single sample taken from the subject, wherein the first portion is exposed to the test compound and the second portion is not. In one aspect of this embodiment, the CCG is expressed at a substantially decreased level in the first sample, relative to the second. In one example, the level of expression in the first sample approximates (e.g., is less than the standard deviation for normal samples) the level of expression in a third control sample, taken from a control sample of normal tissue. This result suggests that the test compound inhibits the expression of the CCG in the sample. In another aspect of this embodiment, the CCG is expressed at a substantially increased level in the first sample, relative to the second. In one embodiment, the level of expression in the first sample approximates (e.g., is less than the standard deviation for normal samples) the level of expression in a third control sample, taken from a control sample of normal tissue. This result suggests that the test compound augments the expression of the CCG in the sample.

Where the efficacy of a therapy is being assessed, the first sample obtained from the subject can be obtained, for example, prior to provision of at least a portion of the therapy, whereas the second sample is obtained following provision of the portion of the therapy. The levels of CCGs in the samples can be compared, for instance, against a third control sample, and correlated with the presence, or risk of presence, of colon cancer. In one embodiment, the level of CCGs in the second sample approximates the level of expression of a third control sample. In the present invention, a substantially decreased level of expression of a CCG indicates that the therapy is efficacious for treating colon cancer.

### Pharmacogenomics

The CCG protein and polynucleotide molecules of the present invention, as well as agents, inhibitors or modulators which have a stimulatory or inhibitory effect on CCG or CCG protein as identified by a screening assay described herein, can be administered to individuals to treat (prophylactically or therapeutically) colon cancer associated with aberrant CCG activity.

In conjunction with such treatment, pharmacogenomics (*e.g.,* the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a CCG product (polynucleotide or polypeptide) or CCG modulator as well as tailoring the dosage or therapeutic regimen of treatment with a CCG product or CCG modulator.

Pharmacogenomics deals with clinically significant hereditary variations in the response to drugs due to altered drug disposition and abnormal action in affected persons. Two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as a "genome-wide association," relies primarily on a high-resolution map of the human genome consisting of already known gene-related sites (e.g., a "bi-allelic" gene marker map which consists of 60,000-100,000 polymorphic or variable sites on the human genome, each of which has two variants). Such a high-resolution genetic map can be compared to a map of the genome of each of a statistically substantial number of subjects taking part in a Phase II/III drug trial to identify genes associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000. bases of DNA. A SNP may be involved in a disease process. However, the vast majority of SNPs may not be disease associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals. Thus, mapping of the CCGs of the invention to SNP maps of colon cancer patients may allow easier identification of these genes according to the genetic methods described herein.

Alternatively, a method termed the "candidate gene approach," can be utilized to identify genes that predict drug response. According to this method, if a gene that encodes a drug target is known (e.g., a CCG of the present invention), all common variants of that gene can be fairly easily identified in the population and it can be determined if having one version of the gene versus another is associated with a particular drug response.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450. enzymes CYP2D6 and CYPZC19) has provided an explanation as to why some subjects do not obtain the expected drug effects or show exaggerated drug response and serious toxicity after taking the standard and safe dose of a drug. These polymorphisms are expressed in two phenotypes in the population, the extensive metabolizer and poor metabolizer. The prevalence of poor metabolizer phenotypes is different among different populations. For example, the gene coding for CYP2D6 is highly polymorphic and several mutations have been identified in poor metabolizers, which all lead to the absence of functional CYP2D6. Poor metabolizers of CYP2D6 and CYP2C19 quite frequently experience exaggerated drug response and side effects when they receive standard doses. If a metabolite is the active therapeutic moiety, poor metabolizers show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Alternatively, a method termed the "gene expression profiling" can be utilized to identify genes that predict drug response. For example, the gene expression of an animal dosed with a drug (*e.g.,* CCG expression in response to a CCG modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment an individual. This knowledge, when applied to dosing or drug selection, can avoid adverse reactions or therapeutic failure and thus enhance therapeutic or prophylactic efficiency when treating a subject with a CCG product or CCG modulator, such as a modulator identified by one of the exemplary screening assays described herein.

### Pharmaceutical Compositions

The invention is further directed to pharmaceutical compositions comprising the test compound, or bioactive agent, or a CCG modulator (e.g., agonist or antagonist), which may further include a CCG product, and can be formulated as described herein. Alternatively, these compositions may include an antibody which specifically binds to a CCG protein of the invention or an antisense polynucleotide molecule which is complementary to a CCG polynucleotide of the invention and can be formulated as described herein.

One or more of the CCGs of the invention, fragments of CCGs, CCG products, fragments of CCG products, CCG modulators, or anti-CCPP antibodies of the invention can be incorporated into pharmaceutical compositions suitable for administration.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The invention includes methods for preparing pharmaceutical compositions for modulating the expression or activity of a polypeptide or polynucleotide corresponding to a CCG of the invention. Such methods comprise formulating a pharmaceutically acceptable carrier with an agent which modulates expression or activity of a polypeptide or polynucleotide corresponding to a CCG of the invention. Such compositions can further include additional active agents. Thus, the invention further includes methods for preparing a pharmaceutical composition by formulating a pharmaceutically acceptable carrier with an agent which modulates expression or activity of a polypeptide or polynucleotide corresponding to a CCG of the invention and one or more additional bioactive agents.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, isotonic agents, such as sugars, polyalcohols (*e.g.,* manitol or sorbitol), or sodium chloride, can be included in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a fragment of a CCPP or an anti-CCPP antibody) in the required amount in an appropriate solvent with one or a combination of ingredients enumerated above, as required, followed by filtered sterilization. Dispersions can be prepared by incorporating the active compound into a sterile vehicle which contains a basic dispersion medium and the required other ingredients from those enumerated above. In the case of sterile powders for the preparation of sterile injectable solutions, the methods of preparation can be, for instance, vacuum drying and freeze-drying which yields a powder of the active ingredient plus any additional desired ingredient from a previously sterile-filtered solution thereof.

Oral compositions can include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose; a disintegrating agent such as alginic acid, Primogel, or com starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the bioactive compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the therapeutic moieties, which may contain a bioactive compound, are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers.

It is especially advantageous to formulate oral or parenteral compositions in dosage unit form for ease of administration and uniformity of dosage. Dosage unit form as used herein includes physically discrete units suited as unitary dosages for the subject to be treated; each unit contains a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices can be selected. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. In one embodiment, the dosage of such compounds lies within a range of circulating concentrations that includes the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

The CCGs of the invention can be inserted into gene delivery vectors and used as gene therapy vectors. Gene therapy vectors can be delivered to a subject by, for example, intravenous administration, intraportal administration, intrabiliary administration, intraarterial administration, direct injection into the liver parenchyma, by intramusclular injection, by inhalation, by perfusion, or by stereotactic injection. The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Kits

The invention also encompasses kits for detecting the presence of a CCG product in a biological sample, the kit comprising reagents for assessing expression of the CCGs of the invention. In one embodiment, the reagents include an antibody or fragment thereof, where the antibody or fragment thereof specifically binds with a protein corresponding to a CCG from Tables 1-5. For example, antibodies of interest may be prepared by methods known in the art. Optionally, the kits may comprise a polynucleotide probe wherein the probe specifically binds with a transcribed polynucleotide corresponding to a CCG. The kits may also include an array of CCGs arranged on a biochip, such as, for example, a GeneChip®. The kit may contain means for determining the amount of the CCG protein or mRNA in the sample and means for comparing the amount of the CCG protein or mRNA in the sample with a control or standard. The compound or agent can be packaged in a suitable container. The kit can further comprise instructions for using the kit to detect CCG protein or polynucleotide

The invention further provides kits for assessing the suitability of each of a plurality of compounds for inhibiting colon cancer in a subject. Such kits include a plurality of compounds to be tested, and a reagent (e.g., antibody specific to corresponding proteins, or a probe or primer specific to corresponding polynucleotides) for assessing expression of a CCG (such as one selected from Tables 1-5).

### Computer Readable Means and Arrays

Computer readable media comprising CCG information of the present invention is also provided. As used herein, "computer readable media" includes a medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. The skilled artisan will readily appreciate how any of the presently known computer readable mediums can be used to create a manufacture comprising computer readable medium having recorded thereon CCG information of the present invention.

As used herein, "recorded" includes a process for storing information on computer readable medium. Those skilled in the art can readily adopt any of the presently known methods for recording information on computer readable medium.

A variety of data processor programs and formats can be used to store the CCG information of the present invention on computer readable medium. For example, the polynucleotide sequence corresponding to the CCGs can be represented in a word processing text file, formatted in commercially-available software such as WordPerfect and Microsoft Word, or represented in the form of an ASCII file, stored in a database application, such as DB2, Sybase, Oracle, or the like. Any number of data processor structuring formats (e.g., text file or database) may be adapted in order to obtain computer readable medium having recorded thereon the CCG information of the present invention.

By providing the CCG information of the invention in computer readable form, one can routinely access the CCG sequence information for a variety of purposes. For example, one skilled in the art can use the nucleotide or amino acid sequences of the invention in computer readable form to compare a target sequence or target structural motif with the sequence information stored within the data storage means. Search means are used to identify fragments or regions of the sequences of the invention which match a particular target sequence or target motif.

### Arrays and Biochips

The invention also includes an array comprising a panel of CCGs of the present invention. The array can be used to assay expression of one or more genes in the array.

It will be appreciated by one skilled in the art that the panels of CCGs of the invention may conveniently be provided on substrates, as a biochip. For example, polynucleotides may be coupled to an array (e.g., a biochip using GeneChip® for hybridization analysis), to a resin (e.g., a resin which can be packed into a column for column chromatography), or a matrix (*e.g.,* a nitrocellulose matrix for northern blot analysis). The immobilization of molecules complementary to the CCG(s), either covalently or noncovalently, permits a discrete analysis of the presence or activity of each CCG in a sample. In an array, for example, polynucleotides complementary to each member of a panel of CCGs may individually be attached to different, known locations on the array. The array may be hybridized with, for example, polynucleotides extracted from a blood or colon sample from a subject. The hybridization of polynucleotides from the sample with the array at any location on the array can be detected, and thus the presence or quantity of the CCG and CCG transcripts in the sample can be ascertained. In one embodiment, an array based on a biochip is employed. Similarly, Western analyses may be performed on immobilized antibodies specific for CCPPs hybridized to a protein sample from a subject.

It will also be apparent to one skilled in the art that the entire CCG product (protein or polynucleotide) molecule need not be conjugated to the biochip support; a portion of the CCG product or sufficient length for detection purposes (e.g., for hybridization), for example a portion of the CCG product which is 7, 10, 15, 20, 25, 30, 35, 40, 45, 50, 55, 60, 65, 70, 75, 100 or more nucleotides or amino acids in length, may be sufficient for detection purposes.

In one embodiment, the array can be used to assay gene expression in a tissue to ascertain tissue specificity of genes in the array. In this manner, up to about 12,000 genes can be simultaneously assayed for expression. This allows an expression profile to be developed showing a battery of genes specifically expressed in one or more tissues at a given point in time.

In addition to such qualitative determination, the invention allows the quantitation of gene expression in the biochip. Thus, not only tissue specificity, but also the level of expression of a battery of CCGs in the tissue is ascertainable. Thus, CCGs can be grouped on the basis of their tissue expression per se and level of expression in that tissue. As used herein, a "normal level of expression" refers to the level of expression of a gene provided in a control sample, typically the control is taken from either a non-diseased animal or from a subject who has not suffered from colon cancer. The determination of normal levels of expression is useful, for example, in ascertaining the relationship of gene expression between or among tissues. Thus, one tissue or cell type can be perturbed and the effect on gene expression in a second tissue or cell type can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to coadminister a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the arrays can be used to monitor the time course of expression of one or more genes in the array. This can occur in various biological contexts, such as development and differentiation, disease progression and cellular transformation and activation.

The array is also useful for ascertaining the effect of the expression of a gene on the expression of other genes in the same cell or in different cells. This provides, for example, for a selection of alternate molecular targets for therapeutic intervention if the ultimate or downstream target cannot be regulated.

Importantly, the invention provides arrays useful for ascertaining differential expression patterns of one or more genes identified in diseased tissue versus non-diseased tissue. This provides a battery of genes that serve as a molecular target for diagnosis or therapeutic intervention. In particular; biochips can be made comprising arrays not only of the CCGs listed in Tables 1-5, but of CCGs specific to subjects suffering from specific manifestations or stages of the disease (*e.g.,* metastasized vs. non-metastasized colon cancer).

The probes can be attached to the biochip in a wide variety of ways, as will be appreciated by those in the art. As described herein, the nucleic acids can either be synthesized first, with subsequent attachment to the biochip, or can be directly synthesized on the biochip.

The biochip comprises a suitable substrate. By "substrate" or other grammatical equivalents herein is meant any material that can be modified to contain discrete individual sites appropriate for the attachment or association of the nucleic acid probes and is amenable to at least one detection method. As will be appreciated by those in the art, the number of possible substrates are very large, and include, but are not limited to, glass and modified or functionalized glass, plastics (including acrylics, polystyrene and copolymers of styrene and other materials, polypropylene, polyethylene, polybutylene, polyurethanes, Teflon, *etc*.), polysaccharides, nylon or nitrocellulose, resins, silica or silica-based materials including silicon and modified silicon, carbon, metals, etc. In general, the substrates allow optical detection and have low background fluorescence.

The substrate can be planar, although as will be appreciated by those in the art, other configurations of substrates may be used as well. For example, the probes may be placed on the inside surface of a tube, for flow-through sample analysis to minimize sample volume. Similarly, the substrate may be flexible, such as a flexible foam, including closed cell foams made of particular plastics.

In one embodiment, the surface of the biochip and the probe may be derivatized with chemical functional groups for subsequent attachment of the two. Thus, for example, the biochip is derivatized with a chemical functional group including, but are not limited to, amino groups, carboxy groups, oxo groups, or thiol groups. Using these functional groups, the probes can be attached using functional groups on the probes. For example, nucleic acids containing amino groups can be attached to surfaces comprising amino groups using homo-or hetero-bifunctional linkers. In addition, in some cases, additional linkers, such as alkyl groups (including substituted and heteroalkyl groups) may be used.

In an embodiment, the oligonucleotides are synthesized as is known in the art, and then attached to the surface of the substrate. As will be appreciated by those skilled in the art, either the 5' or 3' terminus may be attached to the substrate, or attachment may be via an internal nucleoside.

In an additional embodiment, the immobilization to the substrate may be very strong, yet non-covalent. For example, biotinylated oligonucleotides can be made, which bind to surfaces covalently coated with streptavidin, resulting in attachment.

Alternatively, the oligonucleotides may be synthesized on the surface. For example, photoactivation techniques utilizing photopolymerization compounds and techniques are used. In one embodiment, the nucleic acids can be synthesized *in situ,* using well-known photolithographic techniques.

Modifications to the above-described compositions and methods of the invention, according to standard techniques, will be readily apparent to one skilled in the art and are meant to be encompassed by the invention.

It should be understood that the above-described embodiments and the following examples are given by way of illustration, not limitation. Various changes and modifications within the scope of the present invention will become apparent to those skilled in the art from the present description.

### Examples

### Example 1: Identification of colon cancer genes

A query was performed to identify genes that are uniquely over-expressed in colon adenocarcinoma tissue relative to adjacent normal tissues using GeneExpress Oncology DataSuite^{™} and the fold change analysis function within GX2000 analytical program (Gene Logic Inc., Gaithersburg, MD). The GeneExpress Oncology DataSuite^{™} is an interactive information system that provides the global gene expression profiles of human cancer types. The GeneExpress system is based on the collection of cancer and normal tissue samples, the generation of global gene expression data using high-density microarrays, and the analysis of results using a variety of sophisticated software tools, such as GX2000. The normal tissues include human colon, cervix, kidney, left atrium, left ventricle, right atrium, right ventricle, lung, ovary, prostate, rectum, skin and stomach.

Initially, a total of 495 genes were found to have a two-fold differential expression between normal and cancer tissue. Contrast analysis was then used on these 495 genes to identify those genes having a p-value equal to or lower than 0.05. The contrast analysis generated 429 genes. Upon visual inspection of these 429 genes using e-northerns, 63 genes were identified as uniquely over-expressed in colon cancer tissue relative to the panel of normal tissues described above. These 63 genes were defined as colon cancer genes (CCGs) and were classified according to their functional categories into the five gene groups shown in Tables 1-5.

### Example 2: Hydrophobicity analysis

The hydrophobicity profiles of the polypeptides encoded by the CCGs were generated using the TopPred II program *(*Claros et al., TopPred II: An Improved Software For Membrane Protein Structure Predictions., CABIOS, 10, 685-686, 1994) at the Bioweb site maintained by the Pasteur Institute (Paris, France). The hydrophobicity profile is demonstrated in both KD (Kyte and Doolittle) scale and GES (Goldman, Engelman and Steitz) scale.

Briefly, KD scale is a hydropathy scale that associated a hydropathy value to each amino acid. A moving-window approach is implemented in which the hydrophobicity scale over a number of adjacent residues in the native sequence is summed in order to identify membrane regions. A threshold value *T* is defined to label a segment as 'membrane helix': if the sum over the hydrophobicity exceeded *T*, the segment was predicted to be a membrane helix.

The GES scale is also used to identify nonpolar transbilayer helices. The curve is the average of a residue-specific hydrophobicity scale over a window of 20 residues. When the line is in the upper half of the frame (positive), it indicates a hydrophobic region and when it is in the lower half (negative), a hydrophilic region.

In a typical hydrophobicity profile, the X-axis represents the length of the protein in amino acids (aa), while the Y-axis represents the KD or GES score. The curve line shows the KD or GES pattern of the entire protein, while the strait line shows putative cutoffs for potential membrane spanning domains.

The foregoing description of the present invention provides illustration and description, but is not intended to be exhaustive or to limit the invention to the precise one disclosed. Modifications and variations are possible consistent with the above teachings or may be acquired from practice of the invention. Thus, it is noted that the scope of the invention is defined by the claims and their equivalents.

## Claims

1. A method, comprising the steps of:
detecting a level of a polypeptide encoded by a colon cancer gene in a biological sample of a subject; and
comparing said level to a control level of said polypeptide, wherein the colon cancer gene is differentially expressed in colon cancer tissues as compared to disease-free colon tissues.

2. The method according to claim 1, wherein the biological sample is a colon tissue sample, a blood sample, or a bodily waste sample, and the control level is an average level of said polypeptide in control samples of disease-free subjects.

3. The method according to claim 2, wherein the colon cancer gene is selected from the group consisting of a kinase gene, a phosphatase gene, a protease gene, a metabolic enzyme gene, a G-protein coupled receptor gene, an ion channel gene, and a transcription factor gene.

4. The method according to claim 2, wherein the colon cancer gene is selected from Tables 1-5.

5. The method according to claim 4, wherein the subject has colon cancer.

6. The method according to claim 5, wherein the subject is subject to a therapeutic treatment of said cancer.

7. A method, comprising the steps of:
detecting an expression profile of one or more colon cancer genes in a biological sample of a subject; and
comparing the expression profile to a control expression profile of said one or more colon cancer genes, wherein each of said one or more colon cancer genes is differentially expressed in colon cancer tissues as compared to disease-free colon tissues.

8. The method according to claim 7, wherein said one or more colon cancer genes comprise at least one gene selected from Tables 1-5.

9. A method, comprising the steps of:
detecting in a biological sample the level of T cells that are activated by one or more polypeptides encoded by at least one colon cancer gene; and
comparing the level to a control level of said T cells, wherein said colon cancer gene is over-expressed in colon cancer tissues relative to disease-free colon tissues.

10. A pharmaceutical composition comprising a pharmaceutically acceptable carrier and at least one component selected from the group consisting of:
a polypeptide encoded by a colon cancer gene;
a variant of said polypeptide; and
a polynucleotide encoding said polypeptide or variant, wherein said colon cancer gene is over-expressed in colon cancer tissues relative to disease-free colon tissues.

11. The pharmaceutical composition according to claim 10, wherein the pharmaceutical composition is a vaccine formulation capable of eliciting an immune response against a colon cancer cell or a component thereof, and wherein said colon cancer gene is selected from Tables 1-5.

12. A method comprising administering an immunoeffective amount of the pharmaceutical composition of claim 11 to a subject.

13. A pharmaceutical composition comprising a pharmaceutically acceptable carrier and at least one component selected from the group consisting of:
an agent capable of modulating the expression of a colon cancer gene which is over-expressed in colon cancer tissues relative to disease-free colon tissues;
an agent capable of binding to, or modulating an activity of, a polypeptide encoded by said colon cancer gene; and
a T cell activated by said polypeptide.

14. The pharmaceutical composition according to claim 13, wherein said component is selected from the group consisting of:
a polynucleotide comprising or encoding an RNA that is capable of inhibiting or decreasing the expression of said colon cancer gene by RNA interference or an antisense mechanism;
an antibody specific for said polypeptide encoded by said colon cancer gene; and
an inhibitor of a biological activity of said polypeptide, wherein said colon cancer gene is selected from Tables 1-5.

15. A method comprising administering the pharmaceutical composition of claim 14 to a subject who has colon cancer.

16. The pharmaceutical composition according to claim 13, wherein said component is a polynucleotide comprising or encoding a siRNA sense or antisense sequence selected from Table 7.

17. A diagnostic kit comprising at least one of:
(a) a polynucleotide capable of hybridizing under reduced stringent, stringent, or highly stringent conditions to a sequence selected from SEQ ID NOS:1-63, or a complement thereof; and
(b) an antibody capable of specifically binding to a polypeptide selected from SEQ ID NOS:64-126.

18. A nucleic acid array comprising one or more substrate supports which are stably associated with polynucleotide probes, wherein a substantial portion of all polynucleotide probes that are stably associated with said one or more substrate supports are capable of hybridizing under reduced stringent, stringent, or highly stringent conditions to RNA transcripts of colon cancer genes, or the complements thereof, wherein said colon cancer genes are differentially expressed in colon cancer tissues as compared to disease-free colon tissues.

19. A polypeptide array comprising one or more substrate supports which are stably associated with a plurality of polypeptides, wherein a substantial portion of all polynucleotides that are stably associated with said one or more substrate supports are selected from the group consisting of:
polypeptides encoded by colon cancer genes;
variants of said encoded polypeptides;
antibodies specific for said encoded polypeptides or said variants;
polypeptides comprising said encoded polypeptides or said variants; and
any combination thereof,
wherein said colon cancer genes are differentially expressed in colon cancer tissues as compared to disease-free colon tissues.

20. A method for identifying an agent capable of modulating gene expression in a colon cancer cell, comprising the steps of:
contacting said agent with colon cancer cells; and
comparing gene expression profile or profiles of one or more colon cancer genes in said cells before and after said contacting to determine if said agent can modulate said gene expression profile or profiles, wherein each of said one or more colon cancer genes is differentially expressed in colon cancer tissues as compared to disease-free colon tissues.
